# EUROPÄISCHE PATENTSCHRIFT

(11) **EP 0 579 968 B1**
(45) Veröffentlichungstag und Bekanntmachung des Hinweises auf die Patenterteilung: **01.09.1999**
(21) Anmeldenummer: 93109965.9
(22) Anmeldetag: 22.06.1993
(51) Int. Cl.: C07D 215/42, C07D 401/06, A61K 31/47

(54) **4-Iminochinoline, Verfahren zu ihrer Herstellung und ihre Verwendung**
4-Iminoquinolines, processes for their preparation and their use
Dérivés de 4-Iminoquinoléine, procédé pour leur préparation et leur utilisation

(30) Priorität: 27.06.1992 DE 4221210
(43) Veröffentlichungstag der Anmeldung: 26.01.1994
(73) Patentinhaber: HOECHST AKTIENGESELLSCHAFT, 65926 Frankfurt am Main (DE)
(72) Erfinder: Billhardt-Troughton, Uta-Maria, Raleigh (US); Rösner, Manfred, D-6239 Eppstein/Ts (DE); Bender, Rudolf, D-6232 Bad Soden/Ts (DE); Meichsner, Christoph, D-6237 Liederbach (DE)

(56) Entgegenhaltungen:
- EP-A- 0 100 200
- EP-A- 0 476 544
- EP-A- 0 509 398
- WO-A-87/03200
- ANNALES PHARMACEUTIQUES FRANCAISES Bd. 35, Nr. 7-8, 1977, Seiten 239 - 247 'Recherche sur les aminoquinoléines'
- INDIAN JOURNAL OF CHEMISTRY Bd. 26B, 1987, Seiten 910 - 913 'A new synthesis of Dictamnine, Evolitrine and 6-methyldictamnine'
- J. CHEMICAL SOCIETY PERKIN TRANS 1 Bd. 22, 1975, Seiten 2271 - 2280 'Reactions of heterocycles with thiophosgene'
- EUR. J. MED. CHEM. Bd. 11, Nr. 6, 1976, Seiten 561 - 565 'RECHERCHE SUR LES AMINOQUINOL INES XVI'
- INDIAN J. MICROBIOLOGY Bd. 21, Nr. 2, 1981, Seite 159 'Antimicrobial activity of some substtiuted pyrimido[4,5b]quinolines and pyrimido[5,4c]quinolines'
- CANADIAN JOURNAL OF CHEMISTRY, vol.46, no.435, 1968, page 435 - 439, C.PODESVA 'SYNTHESIS AND CHEMISTRY OF 1-METHYL-3-HYDROXY-3-PHENYL-6-CHLORO-1,2,3,4-TE- TRAHYDROQUINOLINE-2,4-DIONE'
- INDIAN J. MICROBIOLOGY Bd. 21, Nr. 2, 1981, Seite 159 'Antimicrobial activity of some substtiuted pyrimido[4,5b]quinolines and pyrimido[5,4c]quinolines'

## Beschreibung

### Gegenstand der vorliegenden Erfindung sind 4-Iminochinoline, Verfahren zu ihrer Herstellung und ihre Verwendung

Mit den Iminochinolinen verwandte Verbindungen, jedoch mit weiteren anellierten Ringen, wurden in einer Publikation aus dem Jahre 1958 (M. Harfenist und E. Magnien, J. Amer. Chem. Soc. 1958, 80, 6080) als Zwischenverbindungen in einer Synthese erwähnt. Weitere Arbeiten beschreiben die Herstellung von Pyrrolo[3,2-c]chinolinonen (T. Tanaka, N. Taga, M. Miyazaki und I. Iijima, J. C.S. Perkin Trans. I 1974, 2110) und Isoxazolo[4,3-c]chinolinonen (P. Roschger und W. Stadlbauer, Liebigs Ann. Chem. 1990, 821; ibid 1991, 401). Eine pharmakologisch interessante Aktivität wurde für diese Verbindungen jedoch noch nicht nachgewiesen. Kürzlich wurden Pyrazolo[4,3-c]chinolinone (EP 0476544 A1) mit antiinflammatorischer Wirkung beschrieben.

Es wurde nun überraschenderweise gefunden, daß bestimmte 4-Iminochinoline eine hohe antivirale Wirksamkeit aufweisen.

Erfindungsgegenstand sind demzufolge Verbindungen der Formel I, 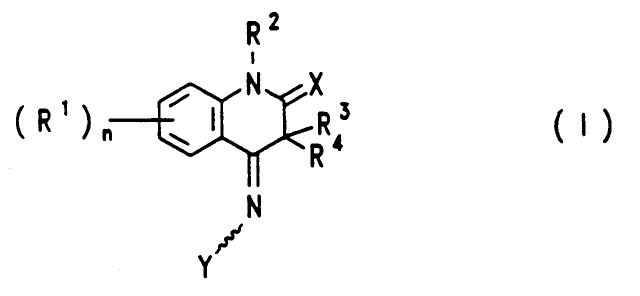 sowie deren tautomere Formen, der allgemeinen Formel Ia, 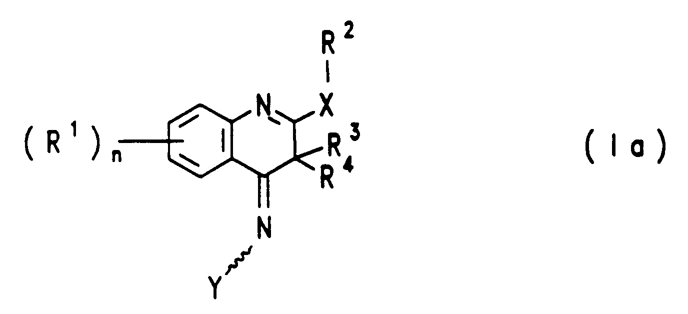 Ib und Ic 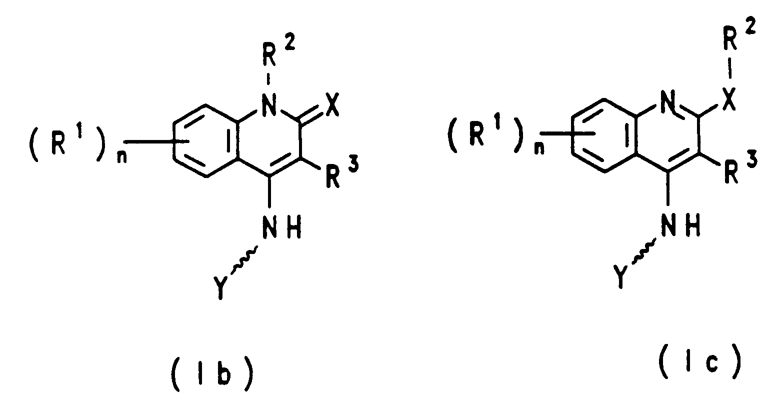 worin bedeuten:
1)
   n
   null,
   eins,
   zwei,
   drei,
   oder vier,
   die einzelnen Substituenten R1 unabhängig voneinander Fluor, Chlor, Brom, Jod, Trifluormethyl, Trifluormethoxy, Hydroxy, C1 - C8-Alkyl, C5 -C8- Cycloalkyl, C1 - C6-Alkoxy, (C1 - C6-Alkoxy)-(C1 - C4-alkoxy), C1- C6-Alkylthio, C1- C6-Alkylsulfinyl, C1- C6-Alkylsulfonyl, Nitro, Amino, Azido, C1 - C6-Alkylamino, Di(C1 - C6-alkyl)amino, Piperidino, Morpholino, 1-Pyrrolidinyl, C1 - C6-Acyl, C1 - C6-Acyloxy, C1 - C6-Acylamino, Cyano, Carbamoyl, Carboxy, (C1 - C6-Alkyl)-oxycarbonyl, Hydroxysulfonyl, Sulfamoyl
      oder
   einen gegebenenfalls mit bis zu fünf voneinander unabhängigen Resten R5 substituierten Phenyl-, Phenoxy-, Phenoxycarbonyl-, Phenylthio-, Phenylsulfinyl-, Phenylsulfonyl, Phenoxysulfonyl-, Phenylsulfonyloxy-, Phenylsulfonylamino, Benzoyl-, Heteroaroyl-, Heteroaryl- oder Heteroarylmethylrest,
      wobei R5
   Fluor, Chlor, Brom, Jod, Cyano, Trifluormethyl, Trifluormethoxy, Nitro, Amino, Azido, C1 - C6-Alkyl, C3 - C8-Cycloalkyl, C1 - C6-Alkoxy, C1 - C6-Alkylthio, C1 - C6-Alkylsulfinyl, C1 - C6-Alkylsulfonyl, C1 - C6-Alkylamino, Di(C1 - C6-alkyl)amino, (C1 - C6-Alkyl)-oxycarbonyl, Phenyl, Phenoxy oder Heteroaryl sein kann,
   X bedeutet Sauerstoff, Schwefel, Selen oder substituierten Stickstoff N-R2, N-O-R2, worin R2 die unten gegebenen Bedeutungen haben kann,
   Y bedeutet R6, O-R6, S-R6, N-R6R7, N=CR6R7 oder C-R6R7R8, wobei R6, R7 und R8 die unten gegebenen Bedeutungen haben können.
   R2, R6, R7 und R8 können gleich oder verschieden, unabhängig voneinander Wasserstoff,
   C1 - C8-Alkyl, gegebenenfalls substituiert mit Fluor, Chlor, Brom, Jod, Cyano, Amino, Mercapto, Hydroxy, C1 - C6-Acyloxy, Benzoyloxy, Benzyloxy, Phenoxy, C1 - C6-Alkoxy, C1 - C6-Alkylamino, Di(C1 - C6-alkyl)amino, C1 - C6-Alkylthio, C1 - C6-Alkylsulfonyl, Phenylsulfonyl, Oxo, Thioxo, Carboxy, Carbamoyl;
   C2 - C8-Alkenyl, gegebenenfalls substituiert mit Fluor, Chlor, Brom, Jod, Cyano, Amino, Mercapto, Hydroxy, C1 - C6-Acyloxy, Benzoyloxy, Benzyloxy, Phenoxy, C1 - C6-Alkoxy, C1 - C6-Alkylamino, Di(C1 - C6-alkyl)amino, C1 - C6-Alkylthio, C1 - C6-Alkylsulfonyl, Phenylsulfonyl, Oxo, Thioxo, Carboxy, Carbamoyl;
   C3 - C8-Alkinyl, gegebenenfalls substituiert mit Fluor, Chlor, Brom, Jod, Cyano, Amino, Mercapto, Hydroxy, C1 - C6-Acyloxy, Benzoyloxy, Benzyloxy, Phenoxy, C1 - C6-Alkoxy, C1 - C6-Alkylamino, Di(C1 - C6-alkyl)amino, C1 - C6-Alkylthio, C1 - C6-Alkylsulfonyl, Phenylsulfonyl, Oxo, Thioxo, Carboxy, Carbamoyl;
   C3 - C8-Cycloalkyl, gegebenenfalls substituiert mit Fluor, Chlor, Brom, Jod, Cyano, Amino, Mercapto, Hydroxy, C1 - C6-Acyloxy, Benzoyloxy, Benzyloxy, Phenoxy, C1 - C6-Alkoxy, C1 - C6-Alkylamino, Di(C1 - C6-alkyl)amino, C1 - C6-Alkylthio, C1 - C6-Alkylsulfonyl, Phenylsulfonyl, Oxo, Thioxo, Carboxy, Carbamoyl;
   C5 - C8-Cycloalkenyl, gegebenenfalls substituiert mit Fluor, Chlor, Brom, Jod, Cyano, Amino, Mercapto, Hydroxy, C1 - C6-Acyloxy, Benzoyloxy, Benzyloxy, Phenoxy, C1 - C6-Alkoxy, C1 - C6-Alkylamino, Di(C1 - C6- alkyl)amino, C1 - C6-Alkylthio, C1 - C6-Alkylsulfonyl, Phenylsulfonyl, Oxo, Thioxo, Carboxy, Carbamoyl;
   (C3 - C8-Cycloalkyl)-(C1 - C4-alkyl), gegebenenfalls substituiert mit Fluor, Chlor, Brom, Jod, Cyano, Amino, Mercapto, Hydroxy, C1 - C6-Acyloxy, Benzoyloxy, Benzyloxy, Phenoxy, C1 - C6-Alkoxy, C1 - C6-Alkylamino, Di(C1 - C6-alkyl)amino, C1 - C6-Alkylthio, C1 - C6-Alkylsulfonyl, Phenylsulfonyl, Oxo, Thioxo, Carboxy, Carbamoyl;
   (C5 - C8-Cycloalkenyl)-(C1 - C4-alkyl), gegebenenfalls substituiert mit Fluor, Chlor, Brom, Jod, Cyano, Amino, Mercapto, Hydroxy, C1 - C6-Acyloxy, Benzoyloxy, Benzyloxy, Phenoxy, C1 - C6-Alkoxy, C1 - C6-Alkylamino, Di(C1 - C6-alkyl)amino, C1 - C6-Alkylthio, C1 - C6-Alkylsulfonyl, Phenylsulfonyl, Oxo, Thioxo, Carboxy, Carbamoyl;
   C1 - C6-Alkylcarbonyl, gegebenenfalls substituiert mit Fluor, Chlor, Brom, Jod, Cyano, Amino, Mercapto, Hydroxy, C1 - C6-Acyloxy, Benzoyloxy, Benzyloxy, Phenoxy, C1 - C6-Alkoxy, C1 - C6-Alkylamino, Di(C1 - C6-alkyl)amino, C1 - C6-Alkylthio, C1 - C6-Alkylsulfonyl, Phenylsulfonyl, Oxo, Thioxo, Carboxy, Carbamoyl;
   C2 - C8-Alkenylcarbonyl, gegebenenfalls substituiert durch Fluor, Chlor oder Hydroxy, C1 - C4-Alkoxy, Oxo, Phenyl;
   (C3 - C8-Cycloalkyl)carbonyl, gegebenenfalls substituiert durch Fluor, Chlor oder Hydroxy, C1 - C4-Alkoxy, Oxo, Phenyl;
   (C5 - C8-Cycloalkenyl)carbonyl, gegebenenfalls substituiert durch Fluor, Chlor oder Hydroxy, C1 - C4-Alkoxy, Oxo, Phenyl;
   (C3 - C8-Cycloalkyl)-(C1 - C3-alkyl)carbonyl, gegebenenfalls substituiert durch Fluor, Chlor oder Hydroxy, C1 - C4-Alkoxy, Oxo, Phenyl;
   (C5 - C6-Cycloalkenyl)-(C1 - C3-alkyl)carbonyl, gegebenenfalls substituiert durch Fluor, Chlor oder Hydroxy, C1 - C4-Alkoxy, Oxo, Phenyl;
   C1 - C8-Alkyloxycarbonyl, gegebenenfalls substituiert durch Fluor, Chlor, Brom, Hydroxy, C1 - C4-Alkoxy, C1 - C4-Alkylamino, Di(C1 - C4-alkyl)amino, C1 - C4-Alkylthio;
   C2 - C8-Alkenyloxycarbonyl, gegebenenfalls substituiert durch Fluor, Chlor, Hydroxy, C1 - C4-Alkoxy, Oxo, Phenyl;
   C2 - C8-Alkinyloxycarbonyl, gegebenenfalls substituiert durch Fluor, Chlor, Hydroxy, C1 - C4-Alkoxy, Oxo, Phenyl;
   C1 - C8-Alkylthiocarbonyl,gegebenenfalls substituiert durch Fluor, Chlor, Hydroxy, C1 - C4-Alkoxy, Oxo, Phenyl;
   C2 - C8-Alkenylthiocarbonyl, gegebenenfalls substituiert durch Fluor, Chlor, Hydroxy, C1 - C4-Alkoxy, Oxo, Phenyl;
   C1 - C8-Alkylamino- und Di(C1 - C8-alkyl)aminocarbonyl, gegebenenfalls substituiert durch Fluor, Chlor, Hydroxy, C1 - C4-Alkoxy, Oxo, Phenyl;
   C2 - C8-Alkenylamino- und Di(C2 - C6-alkenyl)aminocarbonyl, gegebenenfalls substituiert durch Fluor, Chlor, Hydroxy, C1 - C4-Alkoxy, Oxo, Phenyl;
   C1 - C6-Alkylsulfonyl, gegebenenfalls substituiert durch Fluor, Chlor, Hydroxy, C1 - C4-Alkoxy, C1 - C4-Alkylthio, Oxo, Phenyl;
   C2 - C6-Alkenylsulfonyl, gegebenenfalls substituiert durch Fluor, Chlor, Hydroxy, C1 - C4-Alkoxy, Oxo, Phenyl;
   oder mit bis zu fünf voneinander unabhängigen Resten R5 substituiertes Aryl, Arylcarbonyl, Aryl(thiocarbonyl), (Arylthio)carbonyl, (Arylthio)thiocarbonyl, Aryloxycarbonyl, (Arylamino)thiocarbonyl, Arylsulfonyl, Arylalkyl, Arylalkenyl, Arylalkinyl, Arylalkylcarbonyl, Arylalkenylcarbonyl, Arylalkoxycarbonyl, wobei der Alkylrest jeweils 1 bis 5 C-Atome enthalten kann und R5 wie oben definiert ist
   oder mit bis zu drei voneinander unabhängigen Resten R5 substituiertes Heteroaryl, Heteroarylalkyl, Heteroarylalkenyl, Heteroarylalkylcarbonyl oder Heteroarylalkenylcarbonyl, wobei der Alkylrest jeweils 1 bis 3 C-Atome enthalten kann, und
   R3 und R4 gleich oder verschieden, unabhängig voneinander Wasserstoff, C1 - C8-Alkyl, gegebenenfalls substituiert mit Fluor, Chlor, Hydroxy, Amino, Mercapto, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, C1 - C4-Alkylsulfinyl, Carboxy, Carbamoyl;
   C2 - C8-Alkenyl, gegebenenfalls substituiert mit Fluor oder Chlor, Hydroxy, Amino, Mercapto, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, C1 - C4-Alkylsulfinyl, Carboxy, Carbamoyl;
   C3 - C8-Cycloalkyl, gegebenenfalls substituiert mit Fluor, Chlor, Hydroxy, Amino, Mercapto, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, C1 - C4-Alkylsulfinyl, Carboxy, Carbamoyl;
   C3 - C8-Cycloalkenyl, gegebenenfalls substituiert mit Fluor oder Chlor, Hydroxy, Amino, Mercapto, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, C1 - C4-Alkylsulfinyl, Carboxy, Carbamoyl;
   mit bis zu fünf voneinander unabhängigen Resten R5 substituiertes Aryl, Arylalkyl, Heteroaryl oder Heteroarylalkyl bedeuten, wobei der Alkylrest jeweils 1 bis 3 C-Atome enthalten kann und R5 wie oben definiert ist;
   R3 und R4 können zusammen auch einen über eine Doppelbindung verknüpften Rest = C-Z1Z2 bedeuten, wobei Z1 und Z2 die oben für R3 und R4 gegebene Bedeutung haben,
   R3 und R4 können ferner auch
      Teil eines gesättigten oder ungesättigten carbo- oder heterocyclischen Ringes sein, der gegebenenfalls mit Fluor Chlor, Hydroxy, Amino, C1 - C8-Alkyl, C2 - C8-Alkenyl, C2 - C8-Alkinyl, C1 - C8-Acyloxy, Benzoyloxy, C1 - C8-Alkoxy, C1 - C8-Alkylthio, Oxo, Thioxo, Carboxy, Carbamoyl oder Phenyl substituiert sein kann,
   deren optische Isomere, Diastereomere in reiner Form oder in Form ihrer Mischungen und deren Additionsalze,
      wobei in den vorstehenden Definitionen die heterocyclischen Ringe und Heteroarylgruppen 1-15 C-Atome und 1-6 Heteroatome ausgewählt aus der Gruppe O, S und N enthalten und wobei im Falle eines an dieser Stelle gesättigten N-haltigen Ringes N-Z vorliegt, worin Z H oder R2 mit den jeweiligen oben beschriebenen Definitionen bedeutet, mit Ausnahme der Verbindungen, in denen Gleichzeitig R3 und/oder R4 Wasserstoff und Y R6 oder CR6R7R8 bedeuten
   und mit Ausnahme der Verbindungen in denen gleichzeitig n = 0 oder 1, R1 = CH₃ oder OCH₃, R2 = CH₃, R3 = CH₂CH₂OCH₃, X = O und Y = H bedeuten und mit Ausnahme der Verbindungen in denen gleichzeitig n = 1, R1 = Cl, R2 = H oder CH₃, R3 = OH, R4 = Phenyl, X = O und Y = H oder OH bedeuten.

   In einer bevorzugten Gruppe von Verbindungen der Formel I, Ia, Ib bzw. Ic bedeuten:
2)
   n
   null,
   eins,
   oder zwei,
   die einzelnen Substituenten R1 unabhängig voneinander Fluor, Chlor, Brom, Jod, Trifluormethyl, Trifluormethoxy, Hydroxy, C1 - C6-Alkyl, C5 -C6-Cycloalkyl, C1 - C4-Alkoxy, (C1 - C4-Alkoxy)-(C1 - C2-alkoxy), C1- C4-Alkylthio, C1- C4-Alkylsulfinyl, C1- C4-Alkylsulfonyl, Nitro, Amino, C1 - C4-Alkylamino, Di(C1 - C4-alkyl)amino, C1 - C6-Acyl, C1 - C4-Acyloxy, C1 - C4-Acylamino, Cyano, Carbamoyl, Carboxy, (C1 - C4-Alkyl)-oxycarbonyl
      oder
   einen gegebenenfalls mit bis zu drei voneinander unabhängigen Resten R5 substituierten Phenyl-, Phenoxy-, Phenoxycarbonyl-, Phenylthio-, Phenylsulfinyl-, Phenylsulfonyl-, Phenoxysulfonyl-, Phenylsulfonyloxy-, Phenylsulfonylamino, Benzoyl-, Heteroaroyl-, Heteroaryl- oder Heteroarylmethylrest,
      wobei R5
   Fluor, Chlor, Trifluormethyl, Trifluormethoxy, Nitro, Amino, C1 - C4-Alkyl, C3 - C6-Cycloalkyl, C1 - C4-Alkoxy, C1 - C4-Alkylthio, C1 - C4-Alkylsulfinyl, C1 - C4-Alkylsulfonyl, C1 - C4-Alkylamino, Di(C1 - C4-alkyl)amino, Phenyl, Phenoxy oder Heteroaryl sein kann,
   X bedeutet Sauerstoff, Schwefel oder substituierten Stickstoff N-R2, N-O-R2, worin R2 die unten gegebenen Bedeutungen haben kann,
   Y bedeutet R6, O-R6, S-R6, N-R6R7, N=CR6R7 oder C-R6R7R8, wobei R6, R7 und R8 die unten gegebenen Bedeutungen haben können,
   R2, R6, R7 und R8 können gleich oder verschieden, unabhängig voneinander Wasserstoff,
   C1 - C6-Alkyl, gegebenenfalls substituiert mit Fluor, Chlor, Brom, Jod, Cyano, Amino, Mercapto, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, Phenylsulfonyl, Oxo, Thioxo, Carboxy, Carbamoyl;
   C2 - C6-Alkenyl, gegebenenfalls substituiert mit Fluor, Chlor, Cyano, Amino, Mercapto, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, Phenylsulfonyl, Carboxy, Carbamoyl;
   C3 - C6-Alkinyl, gegebenenfalls substituiert mit Fluor, Chlor, Cyano, Amino, Mercapto, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, Phenylsulfonyl, Carboxy, Carbamoyl;
   C3 - C6-Cycloalkyl, gegebenenfalls substituiert mit Fluor, Chlor, Cyano, Amino, Mercapto, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, Phenylsulfonyl, Carboxy, Carbamoyl;
   C5 - C6-Cycloalkenyl, gegebenenfalls substituiert mit Fluor, Chlor, Cyano, Amino, Mercapto, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, Phenylsulfonyl, Carboxy, Carbamoyl;
   (C3 - C6-Cycloalkyl)-(C1 - C2-alkyl), gegebenenfalls substituiert mit Fluor, Chlor, Cyano, Amino, Mercapto, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, Phenylsulfonyl, Carboxy, Carbamoyl;
   (C5 - C6-Cycloalkenyl)-(C1 - C2-alkyl), gegebenenfalls substituiert mit Fluor, Chlor, Cyano, Amino, Mercapto, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, Phenylsulfonyl, Carboxy, Carbamoyl;
   C1 - C6-Alkylcarbonyl, gegebenenfalls substituiert mit Fluor, Chlor, Cyano, Amino, Mercapto, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, Phenylsulfonyl, Carboxy, Carbamoyl;
   C2 - C6-Alkenylcarbonyl, gegebenenfalls substituiert durch Fluor, Chlor, Hydroxy, C1 - C4-Alkoxy, Oxo, Phenyl;
   (C3 - C6-Cycloalkyl)carbonyl, gegebenenfalls substituiert durch Fluor, Chlor, Hydroxy, C1 - C4-Alkoxy, Oxo, Phenyl;
   (C5 - C6-Cycloalkenyl)carbonyl, gegebenenfalls substituiert durch Fluor, Chlor, Hydroxy, C1 - C4-Alkoxy, Oxo, Phenyl;
   (C3 - C6-Cycloalkyl)-(C1 - C2-alkyl)carbonyl, gegebenenfalls substituiert durch Fluor, Chlor, Hydroxy, C1 - C4-Alkoxy, Oxo, Phenyl;
   (C5 - C6-Cycloalkenyl)-(C1 - C2-alkyl)carbonyl, gegebenenfalls substituiert durch Fluor, Chlor, Hydroxy, C1 - C4-Alkoxy, Oxo, Phenyl;
   C1 - C6-Alkyloxycarbonyl, gegebenenfalls substituiert durch Fluor, Chlor, C1 - C4-Alkoxy, C1 - C4-Alkylamino, Di(C1 - C4-alkyl)amino, C1 - C4-Alkylthio, C2 - C6-Alkenyloxycarbonyl, gegebenenfalls substituiert durch Fluor, Chlor, C1 - C4-Alkoxy, Phenyl;
   C2 - C6-Alkinyloxycarbonyl, gegebenenfalls substituiert durch Fluor, Chlor, C1 - C4-Alkoxy, Phenyl;
   C1 - C6-Alkylthiocarbonyl,gegebenenfalls substituiert durch Fluor, Chlor, C1 - C4-Alkoxy, Phenyl;
   C2 - C6-Alkenylthiocarbonyl, gegebenenfalls substituiert durch Fluor, Chlor, C1 - C4-Alkoxy, Phenyl;
   C1 - C6-Alkylamino- und Di(C1 - C6-alkyl)aminocarbonyl, gegebenenfalls substituiert durch Fluor, Chlor, C1 - C4-Alkoxy, Phenyl;
   C2 - C6-Alkenylamino- und Di(C2 - C6-alkenyl)aminocarbonyl, gegebenenfalls substituiert durch Fluor, Chlor, C1 - C4-Alkoxy, Phenyl;
   C1 - C6-Alkylsulfonyl, gegebenenfalls substituiert durch Fluor, Chlor, C1 - C4-Alkoxy, Phenyl;
   C2 - C6-Alkenylsulfonyl, oder mit bis zu drei voneinander unabhängigen Resten R5 substituiertes Aryl, Arylcarbonyl, Aryl(thiocarbonyl), (Arylthio)carbonyl, (Arylthio)thiocarbonyl, Aryloxycarbonyl, (Arylamino)thiocarbonyl, Arylsulfonyl, Arylalkyl, Arylalkenyl, Arylalkinyl, Arylalkylcarbonyl, Arylalkenylcarbonyl, Arylalkoxycarbonyl, wobei der Alkylrest jeweils 1 bis 4 C-Atome enthalten kann und R5 wie oben definiert ist oder mit bis zu drei voneinander unabhängigen Resten R5 substituiertes Heteroaryl, Heteroarylalkyl, Heteroarylalkenyl, Heteroarylalkylcarbonyl oder Heteroarylalkenylcarbonyl, wobei der Alkylrest jeweils 1 bis 3 C-Atome enthalten kann,
      und
   R3 und R4 gleich oder verschieden, unabhängig voneinander C1 - C6-Alkyl, gegebenenfalls substituiert mit Fluor, Chlor, Hydroxy, Amino, Mercapto, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, C1 - C4-Alkylsulfinyl, Carboxy, Carbamoyl;
   C2 - C6-Alkenyl, gegebenenfalls substituiert mit Fluor oder Chlor, Phenoxy, C1 - C4-Alkoxy, C1 - C4-Alkylthio, C1 - C4-Alkylsulfonyl, C1 - C4-Alkylsulfinyl;
   C3 - C6-Cycloalkyl, gegebenenfalls substituiert mit Fluor, Chlor, Hydroxy, Amino, Mercapto, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, C1 - C4-Alkylsulfinyl;
   C3 - C6-Cycloalkenyl, gegebenenfalls substituiert mit Fluor oder Chlor, Phenoxy, C1 - C4-Alkoxy, C1 - C4-Alkylthio, C1 - C4-Alkylsulfonyl, C1 - C4-Alkylsulfinyl;
   mit bis zu drei voneinander unabhängigen Resten R5 substituiertes Aryl, Arylalkyl, Heteroaryl oder Heteroarylalkyl bedeuten, wobei der Alkylrest jeweils 1 bis 3 C-Atome enthalten kann und R5 wie oben definiert ist, sein können und einer der Reste R3 oder R4 kann Wasserstoff sein,
   R3 und R4 können zusammen auch einen über eine Doppelbindung verknüpften Rest =C-Z1Z2 bedeuten, wobei Z1 und Z2 die oben für R3 und R4 gegebene Bedeutung haben.

   In einer nochmals bevorzugten Gruppe von Verbindungen der Formel I, Ia, Ib bzw. Ic bedeuten:
3)
   n
   null,
   eins,
   oder zwei,
   die einzelnen Substituenten R1 unabhängig voneinander Fluor, Chlor, Brom, Trifluormethyl, Trifluormethoxy, Hydroxy, C1 - C4-Alkyl, C1 - C4-Alkoxy, (C1 - C4-Alkoxy)-(C1 - C2-alkoxy), C1- C4-Alkylthio, C1- C4-Alkylsulfinyl, C1-C4-Alkylsulfonyl, Nitro, Amino, C1 - C4-Alkylamino, Di(C1 - C4-alkyl)amino, C1 - C4-Acyl, C1 - C4-Acyloxy, C1 - C4-Acylamino, Cyano, Carbamoyl, Carboxy, (C1 - C4-Alkyl)-oxycarbonyl
      oder
   einen gegebenenfalls mit bis zu drei voneinander unabhängigen Resten R5 substituierten Phenyl-, Phenoxy-, Phenoxycarbonyl, Phenylthio-, Phenylsulfinyl, Phenylsulfonyl-, Benzoyl-, Heteroaroyl-, Heteroaryl- oder Heteroarylmethylrest, wobei R5
   Fluor, Chlor, Trifluormethyl, Trifluormethoxy, Nitro, Amino, C1 - C4-Alkyl, C1 - C4-Alkoxy, C1 - C4-Alkylthio, C1 - C4-Alkylsulfinyl, C1 - C4-Alkylsulfonyl, C1 - C4-Alkylamino, Di(C1 - C4-alkyl)amino, Phenyl oder Phenoxy sein kann,
   X bedeutet Sauerstoff, Schwefel oder substituierten Stickstoff N-R2, N-O-R2, worin R2 die unten gegebenen Bedeutungen haben kann,
   Y bedeutet O-R6, S-R6, N-R6R7 oder N =CR6R7 ,wobei R6, R7 und R8 die unten gegebenen Bedeutungen haben können,
   R2, R6, R7 und R8 können gleich oder verschieden, unabhängig voneinander Wasserstoff,
   C1 - C6-Alkyl, gegebenenfalls substituiert mit Fluor, Chlor, Amino, Mercapto, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio;
   C2 - C6-Alkenyl, gegebenenfalls substituiert mit Fluor, Chlor, Amino, Mercapto, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio;
   C3 - C6-Alkinyl, gegebenenfalls substituiert mit Fluor, Chlor, Amino, Mercapto, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio;
   C3 - C6-Cycloalkyl, gegebenenfalls substituiert mit Fluor, Chlor, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio;
   C5 - C6-Cycloalkenyl,
   (C3 - C6-Cycloalkyl)-(C1 - C2-alkyl),
   (C5 - C6-Cycloalkenyl)-(C1 - C2-alkyl),
   C1 - C6-Alkylcarbonyl, gegebenenfalls substituiert mit Fluor, Chlor, Amino, Mercapto, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio;
   C2 - C4-Alkenylcarbonyl,
   (C3 - C6-Cycloalkyl)carbonyl,
   (C5 - C6-Cycloalkenyl)carbonyl,
   (C3 - C6-Cycloalkyl)-(C1 - C2-alkyl)carbonyl,
   (C5 - C6-Cycloalkenyl)-(C1 - C2-alkyl)carbonyl,
   C1 - C6-Alkyloxycarbonyl, gegebenenfalls substituiert durch Fluor, Chlor, C1 - C4-Alkoxy, C1 - C4-Alkylamino, Di(C1 - C4-alkyl)amino, C1 - C4-Alkylthio;
   C2 - C6-Alkenyloxycarbonyl,
   C2 - C6-Alkinyloxycarbonyl,
   C1 - C6-Alkylthiocarbonyl,
   C2 - C6-Alkenylthiocarbonyl,
   C1 - C6-Alkylamino- und Di(C1 - C6-alkyl)aminocarbonyl,
   C2 - C6-Alkenylamino- und Di(C2 - C4-alkenyl)aminocarbonyl,
   C1 - C6-Alkylsulfonyl,
   C2 - C6-Alkenylsulfonyl,
   oder mit bis zu zwei voneinander unabhängigen Resten R5 substituiertes Aryl, Arylcarbonyl, Aryl(thiocarbonyl), (Arylthio)carbonyl, (Arylthio)thiocarbonyl, Aryloxycarbonyl, (Arylamino)thiocarbonyl, Arylsulfonyl, Arylalkyl, Arylalkenyl, Arylalkinyl, Arylalkylcarbonyl, Arylalkenylcarbonyl, Arylalkoxycarbonyl, wobei der Alkylrest jeweils 1 bis 3 C-Atome enthalten kann und R5 wie oben definiert ist
   oder mit bis zu zwei voneinander unabhängigen Resten R5 substituiertes Heteroaryl, Heteroarylalkyl, Heteroarylalkenyl, Heteroarylalkylcarbonyl oder Heteroarylalkenylcarbonyl, wobei der Alkylrest jeweils 1 bis 2 C-Atome enthalten kann,
      und
   R3 und R4 gleich oder verschieden, unabhängig voneinander C1 - C6-Alkyl, gegebenenfalls substituiert mit Fluor, Chlor, Hydroxy, Amino, Mercapto, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, C1 - C4-Alkylsulfinyl, Carboxy;
   C2 - C6-Alkenyl, gegebenenfalls substituiert mit Fluor oder Chlor;
   C3 - C6-Cycloalkyl,
   C5 - C6-Cycloalkenyl, gegebenenfalls substituiert mit Fluor oder Chlor;
   mit bis zu drei voneinander unabhängigen Resten R5 substituiertes Aryl, Arylalkyl, Heteroaryl oder Heteroarylalkyl bedeuten, wobei der Alkylrest jeweils 1 bis 2 C-Atome enthalten kann und R5 wie oben definiert ist, sein kann,
   einer der Reste R3 oder R4 kann auch Wasserstoff sein,
   R3 und R4 können zusammen auch einen über eine Doppelbindung verknüpften Rest = C-Z1Z2 bedeuten, wobei Z1 und Z2 die oben für R3 und R4 gegebene Bedeutung haben.

Ein weiterer Gegenstand der vorliegenden Erfindung sind die oben unter 1) - 3) beschriebenen Verbindungen zur Anwendung als Arzneimittel.

Für die erfindungsgemäße Anwendung sind die oben unter 1) - 3) genannten Verbindungen bevorzugt.

Die in den vorangegangenen Definitionen genannten Alkylgruppen können geradkettig oder verzweigt sein. Sofern nicht anders definiert, enthalten sie vorzugsweise 1-8, besonders bevorzugt 1-6, insbesondere 1-4 C-Atome. Beispiele sind die Methyl-, Ethyl-, Propyl-, 1-Methylethyl-, Butyl-, 1-Methylpropyl-, 2-Methylpropyl-, 1,1-Dimethylethylgruppe und ähnliche.

Die in den vorangegangenen Definitionen genannten Alkenylgruppen können geradkettig oder verzweigt sein und enthalten 1 bis 3 Doppelbindungen. Sofern nicht anders definiert, enthalten diese Gruppen vorzugsweise 2-8, insbesondere 2-6 C-Atome. Beispiele sind die 2-Propenyl-, 1-Methylethenyl-, 2-Butenyl-, 3-Butenyl-, 2-Methyl-2-propenyl-, 3-Methyl-2-butenyl-, 2,3-Dimethyl-2-butenyl-, 3,3-Dichlor-2-propenyl- und Pentadienylgruppe und ähnliche.

Die in den vorangegangenen Definitionen genannten Alkinylgruppen können geradkettig oder verzweigt sein und enthalten 1 bis 3 Dreifachbindungen. Sofern nicht anders definiert, enthalten sie vorzugsweise 2-8, besonders bevorzugt 3-6 C-Atome. Beispiele sind die 2-Propinyl- und 3-Butinylgruppe und ähnliche.

Die in den vorangegangenen Definitionen genannten Cycloalkyl- und Cycloalkenylgruppen enthalten, sofern nicht anders definiert, vorzugsweise 3-8, besonders bevorzugt 4-6 C-Atome. Beispiele sind die Cyclopropyl-, Cyclobutyl-, Cyclopentyl-, Cyclopentenyl-, Cyclohexyl- oder Cyclohexenylgruppe.

Die in den vorangegangenen Definitionen genannten Acylgruppen können aliphatisch, cycloaliphatisch oder aromatisch sein. Sofern nicht anders definiert, enthalten sie vorzugsweise 1-8, besonders bevorzugt 2-7 C-Atome. Beispielhafte Acylgruppen sind die Formyl-, Acetyl-, Chloracetyl-, Trifluoracetyl-, Hydroxyacetyl-, Glycyl-, Propionyl-, Butyryl-, Isobutyryl-, Pivaloyl-, Cyclohexanoyl- oder Benzoylgruppe.

Für die in den vorangegangenen Definitionen genannten Arylgruppen sind vorzugsweise aromatische Gruppen mit 6-14 C-Atomen geeignet, insbesondere mit 6-10 C-Atomen wie zum Beispiel Phenyl und Naphthyl.

In den obengenannten heterocyclischen Ringen bzw. Heteroarylgruppen kommen als Heteroatome insbesondere zum Beispiel O, S, N in Betracht, wobei im Falle eines an dieser Stelle gesättigten N-haltigen Ringes N-Z vorliegt, worin Z, H oder R2 mit den jeweiligen oben beschriebenen Definitionen bedeutet.

Soweit nicht anders definiert, haben die heterocyclischen Ringe vorzugsweise 1-15 C-Atome und 1-6 Heteroatome, insbesondere 3-11 C-Atome und 1-4 Heteroatome.

Für die in den vorangegangenen Definitionen genannten heterocyclischen Ringe bzw. Heteroarylgruppen kommen beispielsweise Thiophen, Furan, Pyridin, Pyrimidin, Indol, Chinolin, Isochinolin, Oxazol, Isoxazol, Thiazol oder Isothiazol in Frage.

In gleicher Weise gelten diese Definitionen für Heteroaryl in dem Heteroarylmethylrest. Die in den vorausgegangenen Definitionen aufgeführten Aralkylgruppen sind beispielsweise Benzyl, Phenylethyl, Naphthylmethyl oder Styryl.

Die obengenannten Substituenten R1 bis R8 sind vorzugsweise 3-fach, besonders bevorzugt 2-fach, insbesondere einfach mit den jeweils angegebenen Substituenten substituiert.

Für die jeweiligen zusammengesetzten Substituentendefinitionen (wie z. B. Arylalkoxycarbonyl) sind die zuvor als bevorzugt beschriebenen Bereiche für die einzelnen Substituenten ebenfalls bevorzugt.

In Abhängigkeit von den verschiedenen Substituenten können Verbindungen der Formeln I, Ia, Ib und Ic mehrere asymmetrische Kohlenstoffatome besitzen.

Gegenstand der Erfindung sind deshalb sowohl die reinen Stereoisomeren als auch Mischungen derselben, wie z. B. das zugehörige Racemat.

Die reinen Stereoisomeren der Verbindungen der Formeln I, Ia, Ib und Ic lassen sich durch bekannte Methoden oder in Analogie zu bekannten Methoden direkt herstellen oder nachträglich trennen.

Zum Gegenstand der vorliegenden Erfindung gehört weiterhin ein Verfahren zur Herstellung von Verbindungen der Formeln I, Ia, Ib und Ic wie oben unter 1) - 3) erläutert, dadurch gekennzeichnet, daß
A) zur Herstellung von Verbindungen der Formeln I, Ib mit X gleich Sauerstoff und Ia, Ic mit X wie unter 1) - 3) definiert - mit der Ausnahme von N-R2 gleich N-H - Y gleich R6, O-R6, S-R6, N-R6R7, N = C-R6R7 oder C-R6R7R8 und den Resten R1, R2, R3, R4, R5, R6, R7 und R8 wie unter 1) - 3) definiert
   eine Verbindung der Formel II, IIa, IIb bzw. IIc, 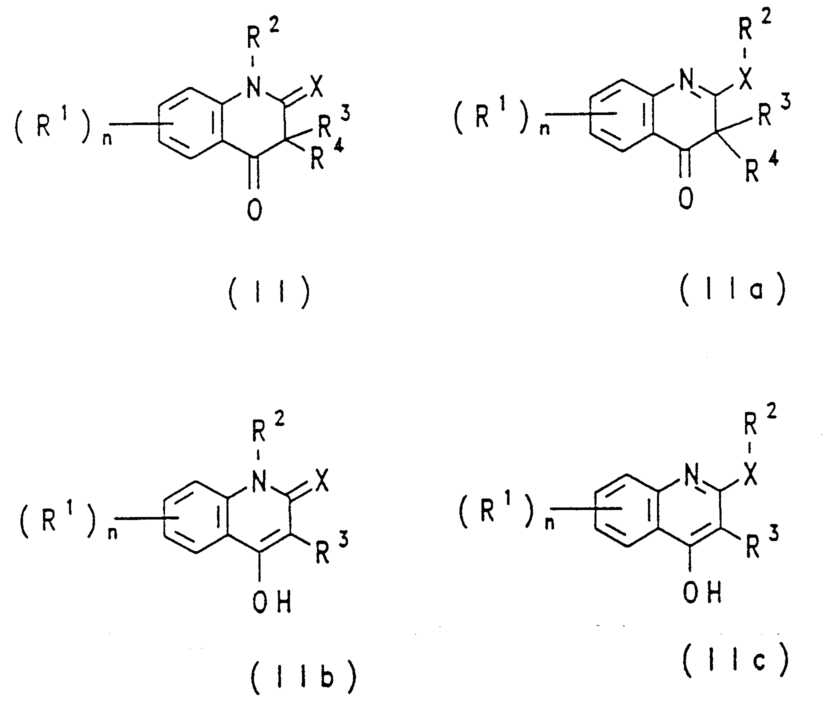 wobei für R1, R2, R3, R4 und R5 die unter 1) - 3) genannten Definitionen gelten,
   mit einer Verbindung der Formel III,

   Y-NH₂ (III)

   wobei Y R6, O-R6, S-R6, N-R6R7, N=C-R6R7 oder C-R6R7R8 bedeuten kann und für R6, R7 und R8 die unter 1) - 4) genanten Definitionen gelten, umgesetzt wird
   oder daß
B) Verbindungen der Formeln I, Ia, Ib und Ic mit X, Y und den Resten R1, R2, R3, R4, R5, R6, R7 und R8 wie unter 1) - 4) definiert, hergestellt werden durch Reaktion einer Verbindung der Formel I, Ia, Ib bzw. Ic, wobei für X und die Reste R1, R2, R3, R4, R5 und R6 die unter 1) - 4) genannten Definitionen gelten und Y gleich H, OH, SH, NH₂ oder NHR6 ist, mit einem Reagenz der Formel IV,

   R9-Z (IV)

   wobei R9 die oben unter 1) - 4) genannten Bedeutungen für R2, R6, R7 und R8 mit Ausnahme von Wasserstoff hat und Z eine Abgangsgruppe ist
   oder daß
C) Verbindungen der Formeln I und Ib, mit X gleich Schwefel und R1, R2, R3, R4, R5, R6, R7 und R8 wie unter 1) - 4) definiert hergestellt werden durch Reaktion einer Verbindung der Formel I bzw Ib, wobei X gleich Sauerstoff ist und für R1, R2, R3, R4, R5, R6, R7 und R8 die unter 1) - 4) genannten Definitionen gelten, mit einem Schwefelungsreagenz
   oder daß
D) zur Herstellung von Verbindungen der Formeln I, Ia, Ib und Ic, mit X und R1, R2, R3, R4 und R5 wie unter 1) - 4) definiert und Y gleich O-R6, S-R6 oder N-R6R7 Verbindungen der Formeln I, Ia, Ib bzw. Ic mit X und R1, R2, R3, R4 und R5 wie unter 1) - 4) definiert und Y gleich OH, SH, NH₂ oder NHR6
   mit einer Verbindung der Formel V,

   R9-OH (V)

   wobei R9 Alkyl, gegebenenfalls substituiert mit Fluor, Chlor, Brom, Jod, Cyano, Amino, Mercapto, Hydroxy, Acyloxy, Benzoyloxy, Benzyloxy, Phenoxy, Alkoxy, Alkylamino, Dialkylamino, Alkylthio, Alkylsulfonyl, Phenylsulfonyl, Oxo, Thioxo, Carboxy, Carbamoyl;
   Alkenyl, gegebenenfalls substituiert mit Fluor, Chlor, Brom, Jod, Cyano, Amino, Mercapto, Hydroxy, Acyloxy, Benzoyloxy, Benzyloxy, Phenoxy, Alkoxy, Alkylamino, Dialkylamino, Alkylthio, Alkylsulfonyl, Phenylsulfonyl, Oxo, Thioxo, Carboxy, Carbamoyl;
   Alkinyl, gegebenenfalls substituiert mit Fluor, Chlor, Brom, Jod, Cyano, Amino, Mercapto, Hydroxy, Acyloxy, Benzoyloxy, Benzyloxy, Phenoxy, Alkoxy, Alkylamino, Dialkylamino, Alkylthio, Alkylsulfonyl, Phenylsulfonyl, Oxo, Thioxo, Carboxy, Carbamoyl;
   Cycloalkyl, gegebenenfalls substituiert mit Fluor, Chlor, Brom, Jod, Cyano, Amino, Mercapto, Hydroxy, Acyloxy, Benzoyloxy, Benzyloxy, Phenoxy, Alkoxy, Alkylamino, Dialkylamino, Alkylthio, Alkylsulfonyl, Phenylsulfonyl, Oxo, Thioxo, Carboxy, Carbamoyl;
   Cycloalkenyl, gegebenenfalls substituiert mit Fluor, Chlor, Brom, Jod, Cyano, Amino, Mercapto, Hydroxy, Acyloxy, Benzoyloxy, Benzyloxy, Phenoxy, Alkoxy, Alkylamino, Dialkylamino, Alkylthio, Alkylsulfonyl, Phenylsulfonyl, Oxo, Thioxo, Carboxy, Carbamoyl;
   (Cycloalkyl)-(alkyl), gegebenenfalls substituiert mit Fluor,
      Chlor, Brom, Jod, Cyano, Amino, Mercapto, Hydroxy, Acyloxy, Benzoyloxy, Benzyloxy, Phenoxy, Alkoxy, Alkylamino, Dialkylamino, Alkylthio, Alkylsulfonyl, Phenylsulfonyl, Oxo, Thioxo, Carboxy, Carbamoyl;
   (Cycloalkenyl)-(alkyl), gegebenenfalls substituiert mit Fluor, Chlor, Brom, Jod, Cyano, Amino, Mercapto, Hydroxy, Acyloxy, Benzoyloxy, Benzyloxy, Phenoxy, Alkoxy, Alkylamino, Dialkylamino, Alkylthio, Alkylsulfonyl, Phenylsulfonyl, Oxo, Thioxo, Carboxy, Carbamoyl;
   Alkylcarbonyl, gegebenenfalls substituiert mit Fluor, Chlor, Brom, Jod, Cyano, Amino, Mercapto, Hydroxy, Acyloxy, Benzoyloxy, Benzyloxy, Phenoxy, Alkoxy, Alkylamino, Dialkylamino, Alkylthio, Alkylsulfonyl, Phenylsulfonyl, Oxo, Thioxo, Carboxy, Carbamoyl;
   Alkenylcarbonyl, gegebenenfalls substituiert durch Fluor, Chlor oder Hydroxy, Alkoxy, Oxo, Phenyl;
   (Cycloalkyl)carbonyl, gegebenenfalls substituiert durch Fluor, Chlor oder Hydroxy, Alkoxy, Oxo, Phenyl;
   (Cycloalkenyl)carbonyl, gegebenenfalls substituiert durch Fluor, Chlor oder Hydroxy, Alkoxy, Oxo, Phenyl;
   (Cycloalkyl)-(alkyl)carbonyl, gegebenenfalls substituiert durch Fluor, Chlor oder Hydroxy, Alkoxy, Oxo, Phenyl;
   (Cycloalkenyl)-(alkyl)carbonyl, gegebenenfalls substituiert durch Fluor, Chlor oder Hydroxy, Alkoxy, Oxo, Phenyl;
   oder mit bis zu fünf voneinander unabhängigen Resten R5 substituiertes Aryl, Arylcarbonyl, Arylalkyl, Arylalkenyl, Arylalkinyl, Arylalkylcarbonyl, Arylalkenylcarbonyl, wobei R5 wie oben definiert ist
   oder mit bis zu drei voneinander unabhängigen Resten R5 substituiertes Heteroaryl, Heteroarylalkyl, Heteroarylalkenyl, Heteroarylalkylcarbonyl oder Heteroarylalkenylcarbonyl sein kann in Gegenwart eines wasserentziehenden Mittels umgesetzt werden
   oder daß
E) Verbindungen der Formeln I und Ib, mit X gleich Sauerstoff und Y, R1, R2, R3, R4 und R5 wie unter 1) - 3), definiert sind, durch Cyclisierung einer Verbindung der Formel VI, 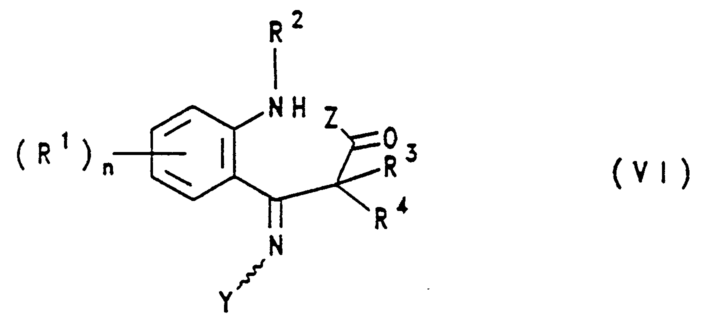 mit Y, R1, R2, R3, R4 und R5 wie unter 1) - 3) definiert und Z eine Abgangsgruppe ist, hergestellt werden.

Die obengenannte Methode A verläuft vorzugsweise unter folgenden Bedingungen ab:
Die Umsetzung wird zweckmäßigerweise in einem Lösungsmittel durchgeführt.

Geeignet sind z. B. aromatische Kohlenwasserstoffe wie Toluol oder Xylol, Wasser, niedere Alkohole wie Methanol, Ethanol, Methylglycol oder 1-Butanol, Ether wie Tetrahydrofuran oder Glycoldimethylether, basische Lösungsmittel wie Pyridin oder N-Methylimidazol, Carbonsäuren wie Essigsäure oder Gemische dieser Lösungsmittel.

Die Anwesenheit eines geeigneten sauren oder basischen Katalysators z. B. p-Toluolsulfonsäure, Essigsäure, Mineralsäuren oder Salze wie Natriumacetat, Natriumcarbonat, Kaliumcarbonat oder Pyridiniumhydrochlorid ist günstig.

Die Reaktionstemperatur kann zwischen 0 und 200 °C liegen, vorzugsweise bei der Siedetemperatur des Lösungsmittels.

Die obengenannte Methode B verläuft vorzugsweise unter folgenden Bedingungen:
Der Substituent Z in der Formel IV ist eine geeignete Abgangsgruppe, wie z. B. Chlor, Brom oder Jod, ein geeignetes Radikal der Schwefelsäure, ein aliphatischer oder aromatischer Sulfonsäureester oder ggf. halogeniertes Acyloxy.

Die Reaktion wird zweckmäßigerweise in einem Lösungsmittel in Gegenwart einer geeigneten Base zum Auffangen der bei der Reaktion freiwerdenden Säure durchgeführt.

Als Lösungsmittel können verwendet werden aromatische Kohlenwasserstoffe wie Toluol oder Xylol, niedere Alkohole wie Methanol, Ethanol, Methylglycol oder 1-Butanol, Ether wie Tetrahydrofuran oder Glycoldimethylether, dipolar aprotische Lösungsmittel wie N,N-Dimethyformamid, N-Methylpyrrolidon, Acetonitril, Nitrobenzol, Dimethylsulfoxid oder Gemische dieser Lösungsmittel.

Geeignete Basen sind z. B. Alkali- oder Erdalkalimetallcarbonate oder -hydrogencarbonate wie Natriumhydrogencarbonat, Natriumcarbonat oder Calciumcarbonat, Alkali- oder Erdalkalimetallhydroxide wie Kaliumhydroxid oder Bariumhydroxid, Alkoholate wie Natriumethanolat oder Kalium-tert.-butylat, lithiumorganische Verbindungen wie Butyllithium oder Lithiumdiisopropylamid, Alkali-oder Erdalkalimetallhydride wie Natriumhydrid oder Calciumhydrid, Alkalimetallfluoride wie Kaliumfluorid oder eine organische Base wie Triethylamin oder Pyridin.

Auch Zweiphasensysteme mit wäßrigen Lösungen von Basen in Gegenwart eines Phasentransferkatalysators wie z. B. Benzyltriethylammoniumchlorid, sind möglich.

In manchen Fällen ist der Zusatz eines Jodsalzes, z. B. Lithiumjodid, angebracht.

Die Reaktion wird gewöhnlich bei Temperaturen zwischen - 10 und 160 ° C durchgeführt vorzugsweise bei Raumtemperatur oder der Siedetemperatur des Lösungsmittels.

Für diese Umsetzung müssen etwaige nucleophile Substituenten wie z. B. Hydroxy-, Mercapto- oder Aminogruppen vor Durchführung der Reaktion in geeigneter Weise derivatisiert oder mit wieder abspaltbaren gebräuchlichen Schutzgruppen wie z. B. Acetyl oder Benzyl, versehen werden.

Für die Umsetzung wie zuvor unter C) beschrieben wird vorzugsweise als Schwefelungsreagenz 2,4-Bis(4-methoxyphenyl)-1,3-dithia-2,4-diphosphetan-2,4-disulfid (Lawessons Reagenz), Bis(tricyclohexylzinn)sulfid, Bis(tri-n-butylzinn)sulfid, Bis(triphenylzinn)sulfid, Bis(trimethylsilyl)sulfid oder Phosphorpentasulfid verwendet. Die Reaktion wird zweckmäßigerweise in einem organischen Lösungsmittel oder einem Lösungsmittelgemisch, bei Raumtemperatur oder höher, bevorzugt bei der Siedetemperatur des Reaktionsgemisches und möglichst unter wasserfreien Bedingungen durchgeführt. Geeignet sind z. B. Schwefelkohlenstoff, Toluol, Xylol, Pyridin oder 1,2-Dichlorethan. Bei Verwendung der erwähnten Zinn- oder Silylsulfide ist es angebracht, die Schwefelungsreaktion in Gegenwart einer Lewissäure wie Bortrichlorid durchzuführen.

In Gegenwart anderer Carbonylgruppen, sind diese gegebenenfalls vor der Schwefelungsreaktion nach bekannten Methoden durch eine geeignete Schutzgruppe, z. B. durch Acetalisierung, zu schützen.

Für die oben unter D) beschriebene Umsetzung werden wasserentziehende Systeme wie Dialkylazodicarbonsäureester und Trialkyl- oder Triarylphosphin benötigt.

Geeignete Lösungsmittel für diese Reaktion sind halogenierte Lösungsmittel wie Dichlormethan oder 1,2-Dichlorethan, Ether wie Tetrahydrofuran oder 1,2-Dimethoxyethan, aromatische Kohlenwasserstoffe wie Toluol oder Xylol oder Gemische dieser Lösungsmittel.

Es sollte unter wasserfreien Bedingungen gearbeitet werden.

Die Reaktion wird vorzugsweise bei Temperaturen zwischen 0 und 100 °C, besonders bevorzugt bei Raumtemperatur durchgeführt.

Die unter E) beschriebene Cyclisierung findet in einem geeigneten Lösungsmittel wie niederen Alkoholen, z. B. Methanol, Ethanol oder Methylglycol, Ethern, z. B. Tetrahydrofuran oder 1,2-Dimethoxyethan, dipolar aprotischen Lösungsmitteln, z. B. N,N-Dimethylformamid oder N-Methylpyrrolidon, in Gegenwart einer Base; geeignet sind Alkali- oder Erdalkalimetallcarbonate oder -hydrogencarbonate wie Natriumcarbonat, Calciumcarbonat oder Natriumbicarbonat, Alkali- oder Erdalkalimetallhydroxide wie Kaliumhydroxid oder Bariumhydroxid, Alkoholate wie Natriumethanolat oder Kalium-tert.-butylat, lithiumorganische Verbindungen wie Butyllithium oder Lithiumdiisopropylamid, Alkali- oder Erdalkalihydride wie Natriumhydrid oder Calciumhydrid oder eine organische Base wie Triethylamin oder Pyridin - letztere können auch als Lösungsmittel verwendet werden, oder organische oder anorganische Säuren wie Essigsäure, Trifluoressigsäure, Salzsäure oder Phosphorsäure.

Die Reaktion wird vorzugsweise bei Temperaturen zwischen - 10 und 120 °C, besonders bevorzugt bei Raumtemperatur durchgeführt.

Der Substituent Z in der Formel VI ist gleich Hydroxy, Alkoxy, Chlor, Brom oder Jod oder ggf. halogeniertes Acyloxy.

Die Ausgangmaterialien der allgemeinen Formeln II, IIa, IIb oder IIc sind literaturbekannt oder lassen sich nach literaturbeschriebenen Methoden herstellen (s. z. Bsp. A. B. Daruwala et al., J. Med. Chem. 1974, 17, 819 G. M. Coppola, Synthesis 1980, 505 und hier zitierte Literatur).

Ein weiteres Verfahren zur Herstellung von Verbindungen der Formeln II oder IIb, mit X gleich Sauerstoff und R1, R2, R3, R4 und R5 wie unter 1) - 4) definiert, ist die Cyclisierung einer Verbindung der Formel VII, 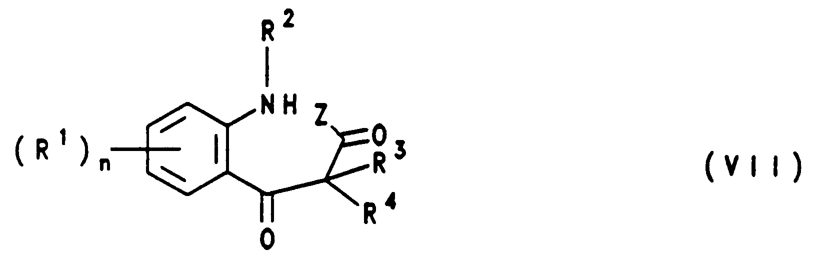 wobei R1, R2, R3, R4 und R5 wie unter 1) - 4) definiert sind und Z eine Abgangsgruppe ist. Die Reaktion wird durchgeführt wie unter E) beschrieben.

Verbindungen der Formel VI werden z. Bsp. durch Reaktion von Verbindungen der Formel VII mit Verbindungen der Formel III nach den unter A) beschriebenen Bedingungen erhalten. Für die Verbindungen der Formeln VI, VII und III gelten die unter A) genannten Definitionen für die Substituenten Y, R1, R2, R3, R4, R5, R6, R7 und R8.

Verbindungen der Formel VII sind literaturbekannt oder lassen sich nach literaturbeschriebenen Methoden herstellen (s. z. Bsp. Methoden der Organischen Chemie (Houben-Weyl), E. Müller (Herausgeber); G. Thieme Verlag, Stuttgart, 1957; Bd. VIII, S. 560 ff).

Die erfindungsgemäßen Arzneimittel können enteral (oral), parenteral (intravenös), rektal, subcutan, intramuskulär oder lokal (topisch) angewendet werden.

Sie können in Form von Lösungen, Pulvern (Tabletten, Kapseln einschließlich Microkapseln), Salben (Cremes oder Gele) oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage.

Als zweckmäßige Dosierung werden 0.1 - 10, vorzugsweise 0.2 - 8 mg/kg Körpergewicht ein- oder mehrmals täglich verabreicht. Die verwendeten Dosierungseinheiten richten sich zweckmäßigerweise nach der jeweiligen Pharmakokinetik der verwendeten Substanz bzw. der verwendeten galenischen Zubereitung.

Die verwendete Dosierungseinheit der erfindungsgemäßen Verbindungen beträgt z. B. 1 - 1500 mg, vorzugsweise 50 - 500 mg.

Die erfindungsgemäßen Verbindungen können auch in Kombination mit anderen antiviralen Mitteln, wie z. B. Nucleosidanaloga, Proteaseinhibitoren oder Adsorptionsinhibitoren und Immunstimulantien, Interferonen, Interleukinen und koloniestimulierenden Faktoren (z. B. GM-CSF, G-CSF, M-CSF) verabreicht werden.

### Wirksamkeitstests

### Prüfung von Präparaten gegen HIV in der Zellkultur

### Methodenbeschreibung

### Medium:

### RPMI pH 6.8

Komplettes Medium enthält zusätzlich 20 % foetales Kälberserum und 40 IU/ml rekombinantes Interleukin 2.

### Zellen:

Aus frischem Spenderblut mittels Ficol^{R}-Gradienten-Zentrifugation isolierte Lymphozyten werden unter Zusatz von 2 µg/ml Phytohämagglutinin (Wellcome) in komplettem Medium 36 h bei 37 °C unter 5 % CO2 kultiviert. Die Zellen werden nach Zusatz von 10 % DMSO bei einer Zelldichte von 5 x 10⁶ eingefroren und in flüssigem Stickstoff gelagert. Für den Versuch werden die Zellen aufgetaut, im RPMI-Medium gewaschen und im kompletten Medium 3 - 4 Tage kultiviert.

### Ansatz:

Die Prüfpräparate wurden in einer Konzentration von 16.7 mg/ml in DMSO gelöst und in komplettem Medium auf 1 mg/ml verdünnt.

In 24er Multiwell-Schalen wurden 0.4 ml Medium vorgelegt. Nach Zugabe von 0.1 ml des gelösten Präparates in die obere Reihe der Schale wurde durch Übertragung von jeweils 0.1 ml eine geometrische Verdünnungsreihe erzeugt. Präparatfreie Kontrollen enthielten stets 0.4 ml komplettes Medium mit 0.5% DMSO.

Lymphozytenkulturen mit einer Zellzahl von 5 x 10⁵ Zellen/ml wurden durch Zugabe 1/50 Volumen Überstand aus HIV-infizierten Lymphozytenkulturen infiziert. Der Titer dieser Kulturüberstände wurde durch Endpunktverdünnung mit 1 - 5 x 10⁶ infektiöse Einheiten/ml bestimmt. Nach 30 min Inkubation bei 37 °C wurden die infizierten Lymphozyten abzentrifugiert und im gleichen Volumen Medium wieder aufgenommen.

Von dieser Zellsuspension wurden jeweils 0.6 ml in alle Vertiefungen der Testplatte gegeben. Die Ansätze wurden 3 Tage bei 37 °C inkubiert.

### Auswertung:

Die infizierten Zellkulturen wurden unter dem Mikroskop auf Anwesenheit von Riesenzellen untersucht, die eine aktive Virusvermehrung in der Kultur anzeigen. Die geringste Präparatekonzentration, bei der keine Riesenzellen auftraten, wurde als Hemmkonzentration gegen HIV bestimmt. Zur Kontrolle wurden die Überstände aus den Kulturplatten mit Hilfe eines HIV-Antigentests entsprechend den Angaben des Herstellers (Organon) auf Anwesenheit von HIV-Antigen bestimmt.

### Ergebnisse:

Die Ergebnisse dieses Tests zeigt Tabelle 1.

**Tabelle 1**

| Verbindung des Beispiels Nr. | T-Zellkulturasssay MHK (µg/ml) |
|---|---|
| 2 | 0.2 |
| 3 | < 0.008 |
| 6 | 0.008 |
| 7 | 0.008 |
| 8 | 0.2 |
| 9 | 0.04 |
| 32 | 1.0 |
| 33 | 0.2 |
| 39 | 1.0 |
| 48 | 0.04 |
| 50 | 0.2 |
| 62 | 0.04 |
| 67 | 0.04 |
| 82 | < 0.08 |
| 87 | 0.04 |
| 103 | 0.04 |
| 104 | 0.04 |
| 115 | 0.04 |
| 119 | < 0.008 |
| 122 | < 0.008 |
| 124 | 0.4 |
| 131 | 1 |

Untersuchung der Substanzen auf Hemmung der HIV-"Reverse Transkriptase" Die Aktivität der Reversen Transkriptase (RT) wurde mit Hilfe eines "Scintillation Proximity Assay" (SPA) bestimmt.

Das Reagenzkit für den RT-SPA wurde von Amersham/Buchler (Braunschweig) bezogen. Das Enzym RT (aus HIV in E. coli cloniert) stammte von der Firma HT-Biotechnology LTD, Cambridge, UK.

### Ansatz:

Der Test wurde nach dem Methoden-Manual des Herstellers Amersham durchgeführt - mit folgenden Modifikationen:
- dem "Assay"-Puffer wurde Rinderserumalbumin zu der Endkonzentration 0.5 mg/ml zugesetzt.
- der Test wurde in Eppendorf-Reaktionsgefäßen mit 100 ml Ansatzvolumen durchgeführt.
- das RT-Konzentrat des Herstellers (5000 U/ml) wurde in Tris-HCl Puffer 20 mM; pH 7.2; 30 % Glycerin auf eine Aktivität von 15 U/ml verdünnt.
- die Inkubationszeit für die Ansätze betrug 60 min (37 °C).
- nach Abstoppen der Reaktion und "Entwicklung" mit der Perlen-Suspension wurden 130 ml Ansatz in 4.5 ml Tris-HCl Puffer, 10 mM; pH 7.4; 0.15 M NaCl transferiert
und die Tritium-Aktivität in einem β-Counter gemessen.

### Substanzprüfung:

Für eine Vorprüfung der Inhibitoraktivität wurden die Substanzen in DMSO gelöst (Stammlösung c = 1 mg/ml) und in Verdünnung in DMSO 10⁻¹, 10⁻², 10⁻³ usw. getestet.

Zur Bestimmung von IC₅₀-Werten wurden die Inhibitor-Stammlösungen in Tris-HCl Puffer, 50 mM, pH 8 weiterverdünnt und in geeigneten Konzentrationen getestet. Aus der graphischen Darstellung RT-Aktivität versus Log C_{lnh.} wurde die einer 50 %igen Enzymhemmung zugehörige Konzentration ermittelt.

Die Ergebnisse der Untersuchung zeigt Tabelle 2.

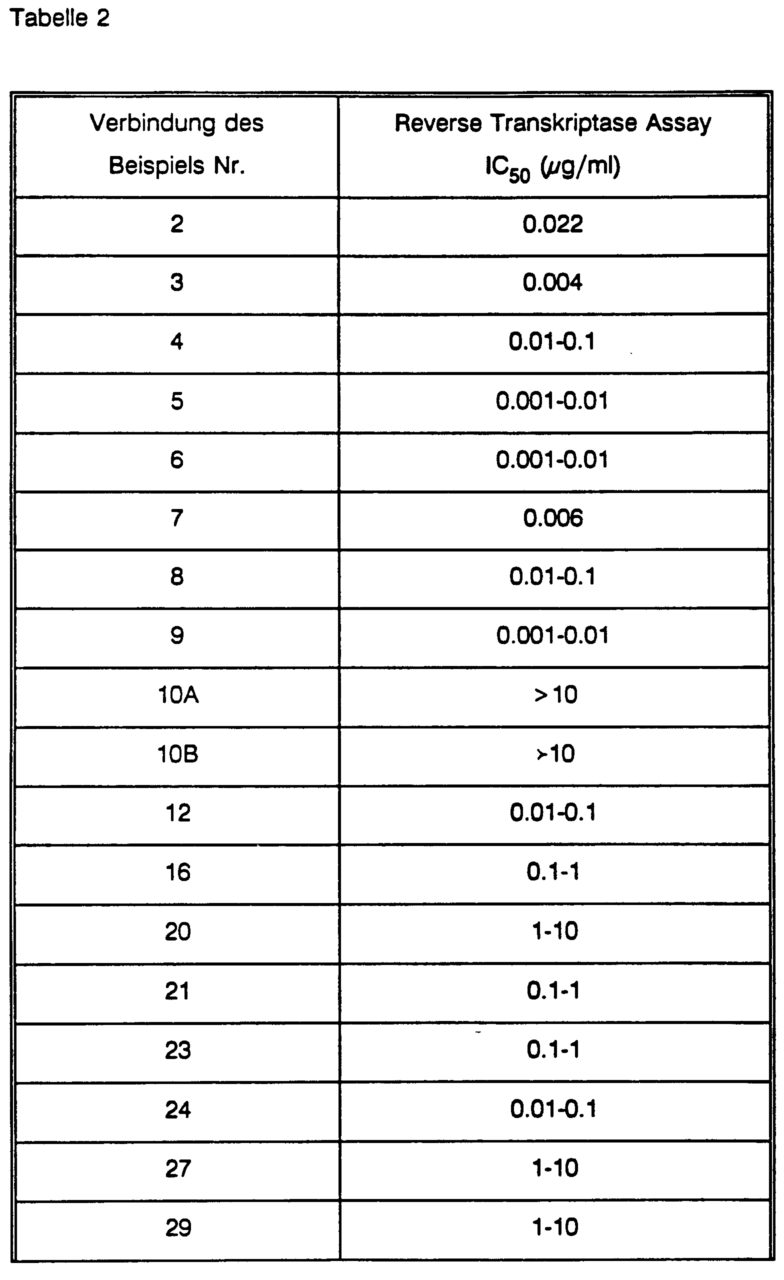

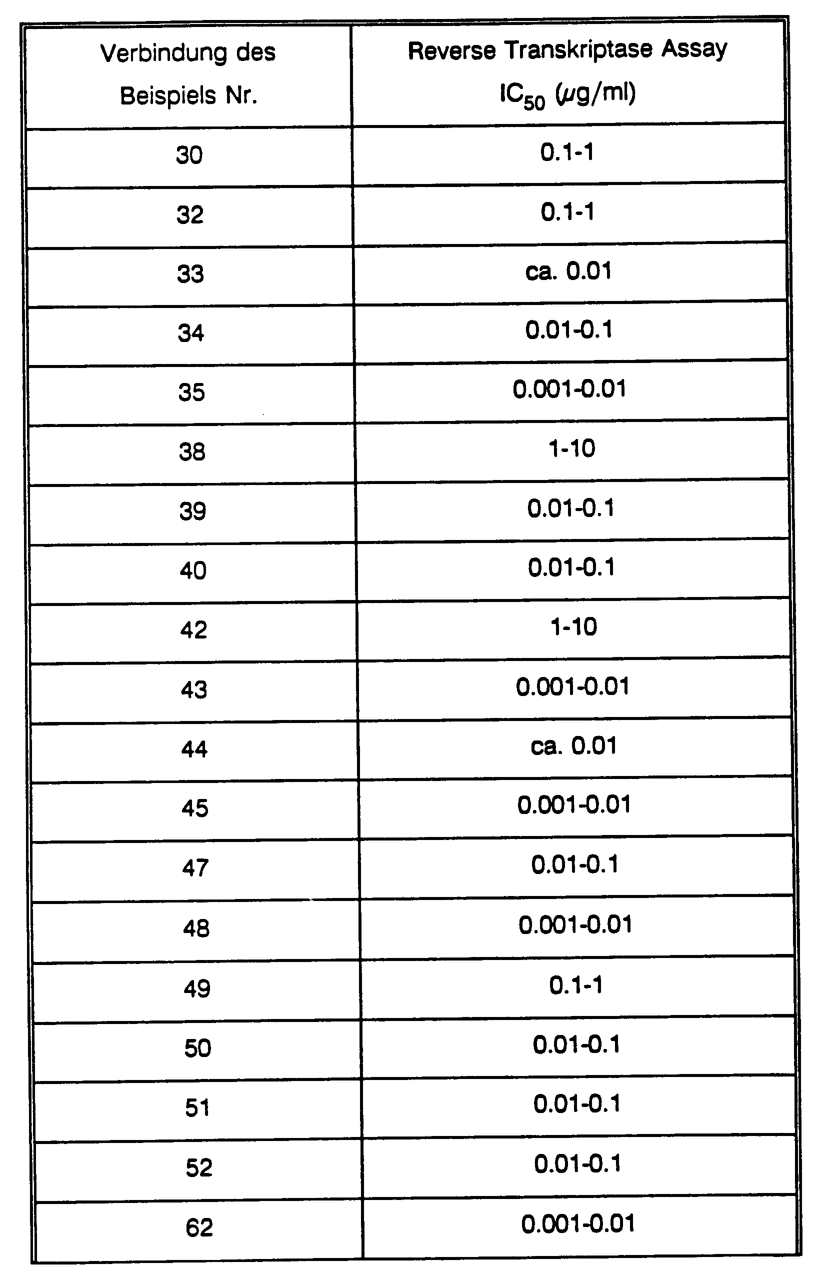

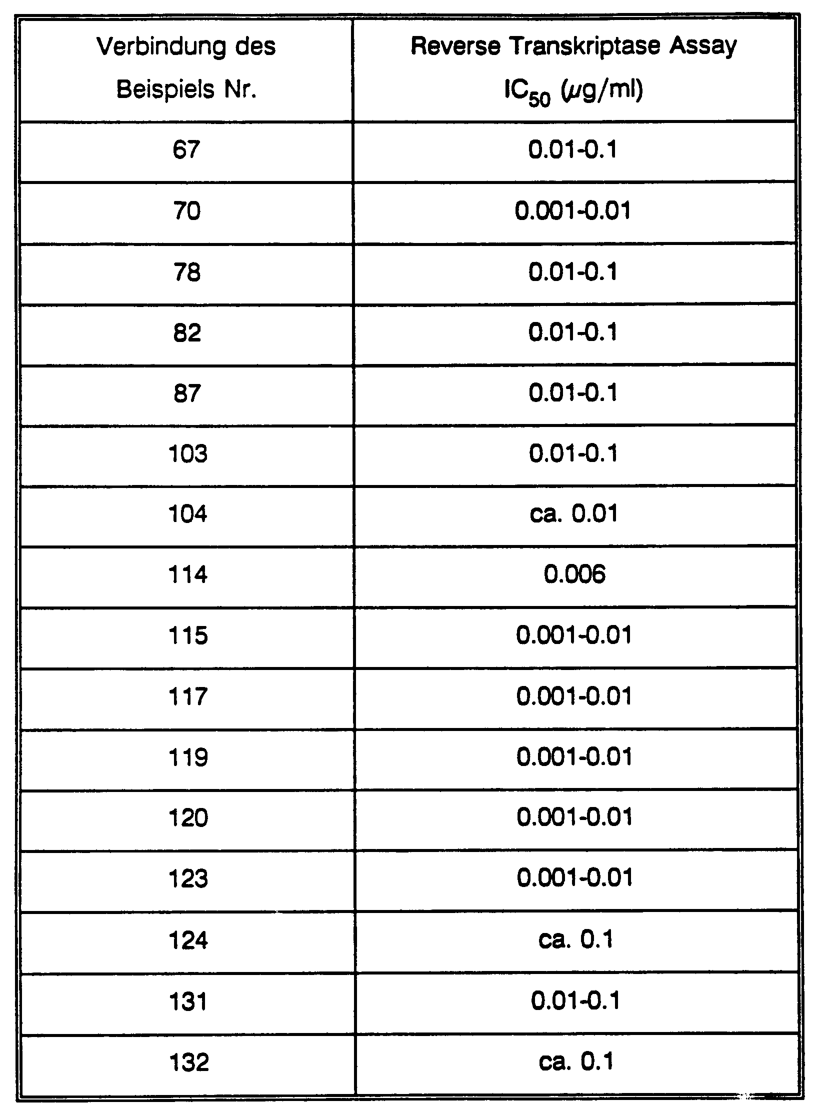

Durch die nachfolgenden Beispiele sowie durch den Inhalt der Patentansprüche wird die vorliegende Erfindung näher erläutert.

### Herstellung der Ausgangsmaterialien

### Beispiel I

### 6-Chlor-3,3-dimethyl-1,3-dihydrochinolin-2,4-dion

Bei -70 °C wurde aus 7.3 g (0.072 mol) Diisopropylamin in 100 ml wasserfreiem Tetrahydrofuran und 45 ml einer 1.6 M Lösung von n-Butyllithium in Hexan (0.072 mol) Lithiumdiisopropylamid hergestellt. Nach kurzem Erwärmen auf -20 °C wurde wieder bei -70 °C 3.48 g (0.03 mol) Isobuttersäureethylester zugegeben. Man ließ auf 0 °C erwärmen und 30 min bei 0 °C rühren. Die Lithiumverbindung wurde zu einer auf - 30 °C gekühlten Suspension von 5.9 g (0.03 mol) 5-Chlorisatinsäureanhydrid in 50 ml wasserfreiem Tetrahydrofuran zugetropft. Man ließ innerhalb einer Stunde auf 0 °C erwärmen und gab die gelbe Reaktionslösung auf 400 ml Eiswasser. Es wurde dreimal mit Essigsäureethylester extrahiert, die vereinigten organischen Phasen je einmal mit gesättigter wäßriger Natriumhydrogencarbonat- und gesättigter wäßriger Natriumchloridlösung gewaschen, über Natriumsulfat getrocknet und eingeengt. Nach Verrühren mit Ether/ Pentan erhielt man 5.1 g (76 %) der gewünschten Verbindung, Schmp. 211 - 212 °C nach Kristallisation aus Isopropanol.
¹H-NMR (200 MHz, d₆-DMSO): δ = 1.30 (s, 6 H), 7.11 (d, J = 7.5 Hz, 1 H), 7.6 - 7.7 (m, 2 H), 10.87 ppm (s, 1H).
MS: (M + H)⁺ = 224

### Beispiel II

### (3RS)-6-Chlor-3-ethyl-1,3-dihydrochinolin-2,4-dion

Eine Suspension von 12.0 g (0.05 mol) 3-Ethylmalonsäure-(4-chloranilid) in 110 g Polyphosphorsäure (ca. 84 % P₂O₅) wurde unter kräftigem Rühren auf 120 °C erwärmt und für 45 min auf dieser Temperatur gehalten. Dabei löste sich die Substanz unter Gelbfärbung und Schäumen. Nach beendeter Reaktion wurde die Reaktionslösung unter Rühren auf ca. 200 ml Eiswasser gegossen, wobei sich das Produkt als weißer Feststoff abschied. Es wurde abgesaugt, mit Wasser neutral gewaschen und unter Vakuum bei 50 °C getrocknet. Ausbeute: 8.3 g (75 %) vom Schmp. 228 °C (nach Umkristallisation aus Isopropanol/Heptan)
¹H-NMR (200 MHz, d₆-DMSO): δ = 1.0 (t, J = 7.5 Hz, 3 H), 2.56 (q, J = 7.5 Hz, 2 H), 7.25 (d, J = 9 Hz, 1 H), 7.48 (dd, J = 9, 2.5 Hz, 1 H), 7.85 (d, J = 2.5 Hz, 1 H), 10.23 (s, 1 H), 11.41 ppm (s, 1H).
MS: (M + H)⁺ = 224

### Beispiel III

### 3,3-Dimethyl-6-methoxy-1,3-dihydrochinolin-2,4-dion

Analog Beispiel II wurden unter Verwendung von 21.8 g (0.092 mol) 3,3-Dimethylmalonsäure-(4-methoxyanilid) 16.2 g (80 %) der gewünschten Verbindung erhalten. Schmp. 169 - 170 °C (nach Umkristallisation aus Isopropanol)
¹H-NMR (200 MHz, d₆-DMSO): δ = 1.31 (s, 6 H), 3.78 (s, 3 H), 7.02 - 7.28 (m, 3 H), 10.62 ppm (s, 1 H).
MS: (M + H)⁺ = 220

### Herstellung der Endprodukte

### Beispiel 1

### anti-6-Chlor-3,3-dimethyl-4-(hydroxyimino)-1,3-dihydrochinol in-2-on (A) und syn-6-Chlor-3,3-dimethyl-4-(hydroxyimino)-1,3-dihydrochinoli n-2-on (B)

Eine Lösung von 11.20 g (0.05 mol) 6-Chlor-3,3-dimethyl-1,3-dihydrochinolin-2,4-dion und 3.80 g (0.055 mol) Hydroxylamin Hydrochlorid wurden in 150 ml wasserfreiem Pyridin gelöst und für 24 h auf 100 °C erhitz. Anschließend wurde die Reaktionsmischung auf ca. 600 ml Eiswasser gegossen, das ausgefallene Produkt abgesaugt, mit Wasser neutral gewaschen und unter Vakuum bei 50 °C getrocknet.

Rohausbeute: 12.1 g vom Schmp. 242 °C (dec).

Durch Umkristallisation aus Isopropanol/Heptan und Essigsäureethylester/Heptan wurden 7.6 g (64 %) reines anti-Isomeres A isoliert. Aus der Mutterlauge ließen sich durch zweimalige Chromatographie an Kieselgel (Essigsäureethylester/Heptan = 2 : 1) 290 mg (2.4 %) des reinen syn-Isomeren B erhalten neben 2.6 g (22 %) des syn-/anti-Gemischs.

### anti-6-Chlor-3,3-dimethyl-4-(hydroxyimino)-1,3-dihydrochinol in-2-on (A)

¹H-NMR (200 MHz, d₆-DMSO): δ = 1.26 (s, 6 H), 7.02 (d, J = 9 Hz, 1 H), 7.43 (dd, J = 9, 2.5 Hz, 1 H), 8.25 (d, J = 2.5 Hz, 1 H), 10.53 (s, 1 H), 11.79 ppm (s, 1 H)
MS: (M + H)⁺ = 239
UV (MeOH): λₘₐₓ (ε) =224 (20 620), 250 (12 400), 320 nm (3 030)

### syn-6-Chlor-3,3-dimethyl-4-(hydroxyimino)-1,3-dihydrochinoli n-2-on (B)

¹H-NMR (200 MHz, d₆-DMSO): δ =1.60 (s, 6 H), 6.94 (d, J = 9 Hz, 1 H), 7.33 (dd, J = 9, 2.5 Hz, 1 H), 7.75 (d, J = 2.5 Hz, 1 H), 10.48 (s, 1 H), 11.68 ppm (s, 1 H)
MS: (M + H)⁺ = 239
UV (MeOH): λₘₐₓ (ε) =221 (15 280), 227 (17 370), 247 (22 200), 329 nm (3 920)

### Beispiel 2

### anti-6-Chlor-3,3-dimethyl-4-(ethyloxyimino)-1,3-dihydrochino lin-2-on

Eine Lösung von 560 mg (2.5 mmol) 6-Chlor-3,3-dimethyl-1,3-dihydrochinolin-2,4-dion (Beispiel I) und 370 mg (3.8 mmol) O-Ethylhydroxylamin Hydrochlorid in 5 ml Pyridin wurde für 20 h unter Rückfluß erhitzt. Anschließend wurde eingeengt, in Essigsäureethylester/Wasser aufgenommen, die Phasen getrennt und die organische einmal mit gesättigter wäßriger Natriumchloridlösung gewaschen. Nach dem Trocknen (Magnesiumsulfat) wurde eingeengt und an Kieselgel chromatographiert. Mit Essigsäureethylester/Heptan = 1 : 2 wurden 550 mg (82 %) des gewünschten Produkts als weißer Feststoff mit Schmp. 186 - 187 °C isoliert. Laut HPLC enthielt das Produkt 10 % des syn-Isomeren. Durch Umkristallisation aus Essigsäureethylester/Heptan erhielt man das reine anti-Isomere, Schmp. 190 - 192 °C.
¹H-NMR (270 MHz, DMSO-d₆): δ = 1.33 (t, J = 7 Hz, 3 H), 1.41 (s, 6 H), 4.23 (q, J = 7 Hz, 1 H), 6.76 (d, J = 9 Hz, 1 H), 7.29 (dd, J = 9, 2.5 Hz, 1 H), 7.88 (br. s, 1 H), 8.28 ppm (d, J = 2.5 Hz, 1 H).
MS: (M + H)⁺ = 267

### Beispiel 3

### anti-6-Chlor-3,3-dimethyl-4-(ethyloxyimino)-1,3-dihydrochino lin-2-thion

Eine Lösung von 200 mg (0.8 mmol) der Verbindung des Beispiels 2 in 10 ml wasserfreiem Toluol wurde mit 200 mg (0.5 mmol) Lawessons Reagenz versetzt und für 1 h auf 100 °C erhitzt. Nach beendeter Reaktion wurde das Lösungsmittel im Vakuum abgezogen und der Rückstand an Kieselgel chromatographiert. Mit Aceton/Heptan 1 : 3 als Elutionsmittel wurden 200 mg (88 %) des gewünschten Produkts als gelber Feststoff isoliert. Schmp. 175 °C (nach Umkristallisation aus Methyl-t.-butylether/Heptan)
¹H-NMR (270 MHz, d₆-DMSO): δ = 1.25 (t, J = 7 Hz, 3 H), 1.41 (s, 6 H), 4.19 (q, J = 7 Hz, 2 H), 7.28 (d, J = 9 Hz, 1 H), 7.56 (dd, J = 9, 2.5 Hz, 1 H), 8.13 (d, J = 2.5 Hz, 1 H), 12.55 ppm (br. s, 1 H).
MS: (M + H)⁺ = 283

### Beispiel 4

### syn-6-Chlor-3,3-dimethyl-4-(ethyloxyimino)-1,3-dihydrochinol in-2-on

Zu einer Lösung von 400 mg (1.68 mmol) der Verbindung des Beispiels 1B in 10 ml wasserfreiem Tetrahydrofuran und 2 ml absolutem Ethanol wurde unter Kühlung auf 0°C 1.32 g (5.03 mmol) Triphenylphosphin zugegeben. Anschließend tropfte man ebenfalls bei 0 °C eine Lösung von 1.1 ml (6.95 mmol) Azodicarbonsäurediethyester in 2 ml absolutem Ethanol langsam zu, ließ noch 30 min bei 0 °C dann 20 h bei Raumtemperatur rühren und engte ein. Nach Chromatographie an Kieselgel mit Essigsäureethylester/ Heptan = 1 : 3 als Elutionsmittel erhielt man 310 mg (69 %) der gewünschten Verbindung als weiß-kristallinen Feststoff vom Schmp. 206 - 207 °C.
¹H-NMR (200 MHz, d₆-DMSO): δ = 1.28 (t, J = 7 Hz, 3 H), 1.58 (s, 6 H), 4.22 (q, J = 7 Hz, 1 H), 6.96 (d, J = 9 Hz, 1 H), 7.37 (dd, J = 9, 2.5 Hz, 1 H), 7.80 (d, J = 2.5 Hz, 1 H), 10.55 ppm (br. s, 1 H).
MS: (M + H)⁺ = 267

### Beispiel 5

### syn-6-Chlor-3,3-dimethyl-4-(ethyloxyimino)-1,3-dihydrochinol in-2-thion

Wie unter Beispiel 3 beschrieben wurden 200 mg (0.8 mmol) der Verbindung des Beispiels 4 mit Lawessons Reagenz geschwefelt. Nach Chromatographie an Kieselgel mit Essigsäurethylester/Heptan = 1 : 5 als Elutionsmittel erhielt man 180 mg (85 %) der gewünschten Verbindung, jedoch als 1 : 2- Gemisch mit dem anti-Isomeren des Beispiels 3. (Thermisch tritt Isomerisierung ein.) Schmp. 163 - 164 °C
¹H-NMR (200 MHz, d₆-DMSO): δ = 1.30 (t, J = 7.5 Hz, 3 H), 1.38 (s, 6 H), 4.26 (q, J = 7.5 Hz, 1 H), 7.25 (d, J = 9 Hz, 1 H), 7.45 (dd, J = 9, 2.5 Hz, 1 H), 7.85 (d, J = 2.5 Hz, 1 H), 12.55 ppm (br. s, 1 H).
MS: (M + H)⁺ = 283

### Beispiel 6

### anti-6-Chlor-3,3-dimethyl-4-(isopropyloxyimino)-1,3-dihydroc hinolin-2-on

Die Verbindung des Beispiels 1A (954 mg, 4.0 mmol) in 30 ml wasserfreiem Tetrahydrofuran wurde mit 212 mg (4.4 mmol) einer 50 %-igen Natriumhydridsuspension in Mineralöl versetzt und 30 min unter Rückfluß erhitzt. Dann wurde auf Raumtemperatur abgekühlt, 0.86 ml (8.8 mmol) 2-Jodpropan zugegeben und für weitere 26 h unter Rückfluß erhitzt. Anschließend wurde die Reaktionslösung eingeengt und an Kieselgel chromatographiert (Essigsäureethyester/Heptan = 1 : 2). Es wurden 560 mg (50 %) des gewünschten Produkts vom Schmp. 207 - 208 °C isoliert.
¹H-NMR (200 MHz, d₆-DMSO): δ = 1.25 (d, J = 7 Hz, 6 H), 1.28 (s, 6 H), 4.38 (sept., J = 7 H, 1 H), 7.04 (d, J = 9 Hz, 1 H), 7.44 (dd, J = 9, 2.5 Hz, 1 H), 8.15 (d, J = 2.5 Hz, 1 H), 10.58 ppm (s, 1 H)
MS: (M + H)⁺ = 281

### Beispiel 7

### anti-6-Chlor-3,3-dimethyl-4-(isopropyloxyimino)-1,3-dihydroc hinolin-2-thion

Eine Suspension von 450 mg (1.6 mmol) der Verbindung des Beispiels 6 in 30 ml wasserfreiem Toluol wurde mit 357 mg (0.88 mmol) Lawessons Reagenz für 2 h unter Rückfluß erhitzt. Nach Entfernen des Lösungsmittels wurde an Kieselgel mit Essigsäureethylester/Heptan = 1 : 3 gereinigt. Die Ausbeute betrug 450 mg (94 %) kristalliner Feststoff vom Schmp. 194 °C.
¹H-NMR (200 MHz, d₆-DMSO): δ = 1.24 (d, J = 6 Hz, 6 H), 1.38 (s, 6 H), 4.40 (m, 1 H), 7.28 (d, J = 9 Hz, 1 H), 7.55 (dd, J = 9, 2.5 Hz, 1 H), 8.16 (d, J = 2.5 Hz, 1 H), 12.54 ppm (s, 1 H)
MS: (M + H)⁺ = 297

### Beispiel 8

### anti-6-Chlor-3,3-dimethyl-4-(n-propyloxyimino)-1,3-dihydroch inolin-2-on

Analog Beispiel 6 wurden aus der Verbindung des Beispiels 1A (954 mg, 4.0 mmol) und n-Propyljodid (2.0 ml, 20.0 mmol) 500 mg (44 %) der gewünschten Verbindung vom Schmp. 157 °C isoliert.
¹H-NMR (200 MHz, d₆-DMSO): δ = 0.91 (t, J = 7.5 Hz, 3 H), 1.25 (s, 6 H), 1.66 (m, 2 H), 4.09 (t, J = 7 Hz, 2 H), 7.04 (d, J = 9 Hz, 1 H), 7.46 (dd, J = 9, 2.5 Hz, 1 H), 8.13 (d, J = 2.5 Hz, 1 H), 10.58 ppm (s, 1 H)
MS: (M + H)⁺ = 281

### Beispiel 9

### anti-6-Chlor-3,3-dimethyl-4-(n-propyloxyimino)-1,3-dihydroch inolin-2-thion

Die Verbindung des Beispiels 8 (393 mg, 1.4 mmol) wurde wie in Beispiel 7 beschrieben geschwefelt. Man erhielt 370 mg (89 %) des gewünschten Produkts vom Schmp. 156 °C.
¹H-NMR (200 MHz, d₆-DMSO): δ = 0.91 (t, J = 7.5 Hz, 3 H), 1.38 (s, 6 H), 1.66 (m, 2 H), 4.11 (t, J = 6 Hz, 2 H), 7.28 (d, J = 9 Hz, 1 H), 7.55 (dd, J = 9, 2.5 Hz, 1 H), 8.14 (d, J = 2.5 Hz, 1 H), 12.55 ppm (s, 1 H)
MS: (M + H)⁺ = 297

### Beispiel 10

### anti-4-(Benzyloxyimino)-6-chlor-3,3-dimethyl-1,3-dihydrochin olin-2-on (A) und anti-(1-Benzyl)-4-(benzyloxyimino)-6-chlor-3,3-dimethyl-1,3- dihydrochinolin-2-on (B)

Die Verbindung des Beispiels 1A (954 mg, 4.0 mmol) in 30 ml wasserfreiem Tetrahydrofuran wurde mit 212 mg (4.4 mmol) einer 50 %-igen Suspension von Natriumhydrid in Mineralöl versetzt und für 30 min unter Rückfluß erhitzt. Dann wurde abgekühlt, 1.37 g (8.0 mmol) Benzylbromid zugegeben und weitere 20 h unter Rückfluß erhitzt. Die Reaktionslösung wurde eingeengt und durch Chromatographie an Kieselgel (Essigsäureethylester/Heptan = 1 : 3) getrennt. Nach Anrühren der Eluate mit Pentan erhielt man 580 mg (35 %) weiß kristalline Verbindung 10B vom Schmp. 143 °C und 410 mg (31 %) weiß kristalline Verbindung 10A vom Schmp. 183 °C.

### anti-4-(Benzyloxyimino)-6-chlor-3,3-dimethyl-1,3-dihydrochinolin-2-on (A)

¹H-NMR (200 MHz, d₆-DMSO): δ = 1.26 (s, 6 H), 5.20 (s, 2 H), 7.03 (d, J = 9.5 Hz, 1 H), 7.2 - 7.5 (m, 6 H), 8.13 (d, J = 2.5 Hz, 1 H), 10.60 ppm (s, 1 H)
MS: (M + H)⁺ = 329

### anti-(1-Benzyl)-4-(benzyloxyimino)-6-chlor-3,3-dimethyl-1,3-dihydrochinolin-2-on (B)

¹H-NMR (200 MHz, d₆-DMSO): δ = 1.35 (s, 6 H), 5.14 (s, 2 H), 5.21 (s, 2 H), 7.1 - 7.5 (m, 12 H), 8.10 ppm (d, J = 2.5 Hz, 1 H)
MS: (M + H)⁺ = 419

### Beispiel 11

### anti-6-Chlor-3,3-dimethyl-4-hydrazono-1,3-dihydrochinolin-2- on

Die Verbindung des Beispiels 1A (6.7 g, 0.03 mol) wurde in 200 ml wasserfreiem Ethanol suspendiert und nach Zugabe von 14.5 ml (0.3 mol) Hydrazinhydrat (100 %) und 2 g Pyridiniumhydrochlorid 14 h unter Rückfluß erhitzt. Die gelbe Reaktionslösung wurde eingeengt und der feste Rückstand aus Isopropanol umkristallisiert. Die Ausbeute betrug 3.3 g (46 %) vom Schmp. 228 - 230 °C.
¹H-NMR (200 MHz, d₆-DMSO): δ = 1.17 (s, 6 H), 6.66 (s, 2 H), 7.02 (d, J = 9Hz, 1 H), 7.36 (dd, J = 9, 2.5 Hz, 1 H), 7.86 (d, J = 2.5 Hz, 1 H), 10.38 ppm (s, 1 H).
MS: (M + H)⁺ = 238

### Beispiel 12

### anti-6-Chlor-3,3-dimethyl-4-(isopropylazino)-1,3-dihydrochin olin-2-on

Die Verbindung des Beispiels 11 (950 mg, 4 mmol) wurde mit je 30 ml Aceton und Methanol 2 h bei Raumtemperatur gerührt. Anschließend wurde eingeengt und aus Heptan/ Essigsäureethylester umkristallisiert. Ausbeute : 440 mg (40 %) vom Schmp. 184 - 185 °C.
¹H-NMR (200 MHz, d₆-DMSO): δ = 1.31 (s, 6 H), 1.86 (s, 3 H), 2.02 (s, 3 H), 7.02 (d, J = 9 Hz, 1 H), 7.43 (dd, J = 9, 2.5 Hz, 1 H), 7.83 (d, J = 2.5 Hz, 1 H), 10.58 ppm (s, 1 H).
MS: (M + H)⁺ = 306

### Beispiel 13

### anti-3,3-Dimethyl-4-(isopropenyloxycarbonyloxyimino)-6-methoxy-1,3-dihydrochinolin-2-on

Die Verbindung des Beispiels 36 (936 mg, 4 mmol) wurde in 50 ml wasserfreiem Dichlormethan suspendiert und 0.65 ml (8 mmol) Pyridin zugegeben. Unter Eiskühlung wurden 0.66 ml (6 mmol) Isopropenylchloroformat zugetropft und anschließend bei Raumtemperatur gerührt. Nach 18 h wurde mit Wasser gewaschen, getrocknet (Natriumsulfat) und das Rohprodukt aus Diisopropylether umkristallisiert. Ausbeute 750 mg (59 %) vom Schmp. 145 - 146 °C.
¹H-NMR (200 MHz, d₆-DMSO): δ = 1.34 (s, 6 H), 1.95 (s, 3 H), 3.77 (s, 3 H), 4.88 (s, 2 H), 7.03 (d, J = 9 Hz, 1 H), 7.16 (dd, J = 9, 3 Hz, 1 H), 7.53 (d, J = 3 Hz, 1 H), 10.51 ppm (s, 1 H).
MS: (M + H)⁺ = 319

### Beispiel 13a

### anti-6-Chlor-3,3-dimethyl-4-(ethyloxyimino)-2-hydroxyimino-1,3-dihydrochinolin (siehe auch Beispiel 70 in Tabelle 3)

Eine Suspension von 848 mg (3 mmol) der Verbindung des Beispiels 3 (anti-6-Chlor-3,3-dimethyl-4-(ethyloxyimino)-1,3-dihydrochinolin-2-thion) in 30 ml wasserfreiem Ethanol wurde bei 25°C mit 417 mg (6 mmol, 2 Äquivalente) Hydroxylaminhydrochlorid sowie 0.83 ml (6 mmol, 2 Äquivalente) Triethylamin versetzt.

Das Reaktionsgemisch wurde 18 h bei 25°C gerührt. Nach beendeter Reaktion wurde das Lösungsmittel im Vakuum abgezogen und der Rückstand in 150 ml Essigsäureethylester aufgenommen. Die organische Phase wurde zweimal mit jeweils 75 ml Wasser extrahiert, mittels Natriumsulfat getrocknet und das Lösungsmittel im Vakuum entfernt. Das auf diese Weise erhaltene Rohprodukt wurde mit n-Pentan verrieben. Man erhielt 810 mg (2.88 mmol) der gewünschten Verbindung (Ausbeute 96 %) vom Schmelzpunkt 192 - 193°C (RF-Wert: 0.25; Laufmittel: n-Heptan/Essigsäure-ethylester 3:1)
¹H-NMR (270 MHz, d₆-DMSO: δ = 1.23 (t, J = 7.6 Hz, 3 H), 1.25 (s, 6 H), 4.15 (d, J = 7.6 Hz, 2 H), 7.28 (d, J = 8 Hz, 1 H), 7.34 (dd, J = 8, 2.5 Hz, 1 H), 8.02 (d, J = 2.5 Hz, 1 H), 9.33 (br. s, 1 H), 10.13 (br. s, 1 H)
MS: (M + H)⁺ = 282

### Beispiel 13b

### anti-6-Chlor-3,3-dimethyl-2-(methylamino)-chinolin-4(3H)-on-4-O-ethyloxim (siehe auch Beispiel 127 in Tabelle 3)

Eine Suspension aus 1.41 g (5 mmol) der Verbindung des Beispiels 3 (anti-6-Chlor-3,3-dimethyl-4-(ethyloxyimino)-1,3-dihydrochinolin-2-thion) in 50 ml wasserfreiem Ethanol wurde bei 25°C mit 5 ml 40-prozentiger wässriger Methylaminlösung (71.4 mmol) versetzt. Das Reaktionsgemisch wurde 8 h unter Rückflußbedingungen gerührt. Nach beendeter Reaktion wurde das Lösungsmittel im Vakuum abgezogen und der Rückstand an Kieselgel chromatographiert. Mit Essigsäureethylester/n-Heptan 1:1 als Elutionsmittel wurden 550 mg (1.97 mmol; 39 %) des gewünschten Produkts als farbloser Feststoff vom Schmelzpunkt 112°C isoliert (RF-Wert: 0.51; Laufmittel: Essigsäure-ethylester/n-Heptan 1:1).
¹H-NMR (200 MHz, d₆-DMSO: δ = 1.26 (t, J = 7.5 Hz, 3 H), 1.25 (s, 6 H), 2.79 (d, J = 4.5 Hz, 3 H), 4.14 (q, J = 7.5 Hz, 2 H), 6.98 (d, J = 9 Hz, 1 H), 7.16 (br. q, J = 4.5 Hz, 1 H), 7.28 (dd, J = 3,9 Hz, 1 H), 8.05 (d, J = 3 Hz, 1 H)
MS: (M + H)⁺ = 280

In Analogie zu den Vorschriften der Beispiele 1 - 13b wurden die in Tabelle 3 aufgeführten Beispiele hergestellt:

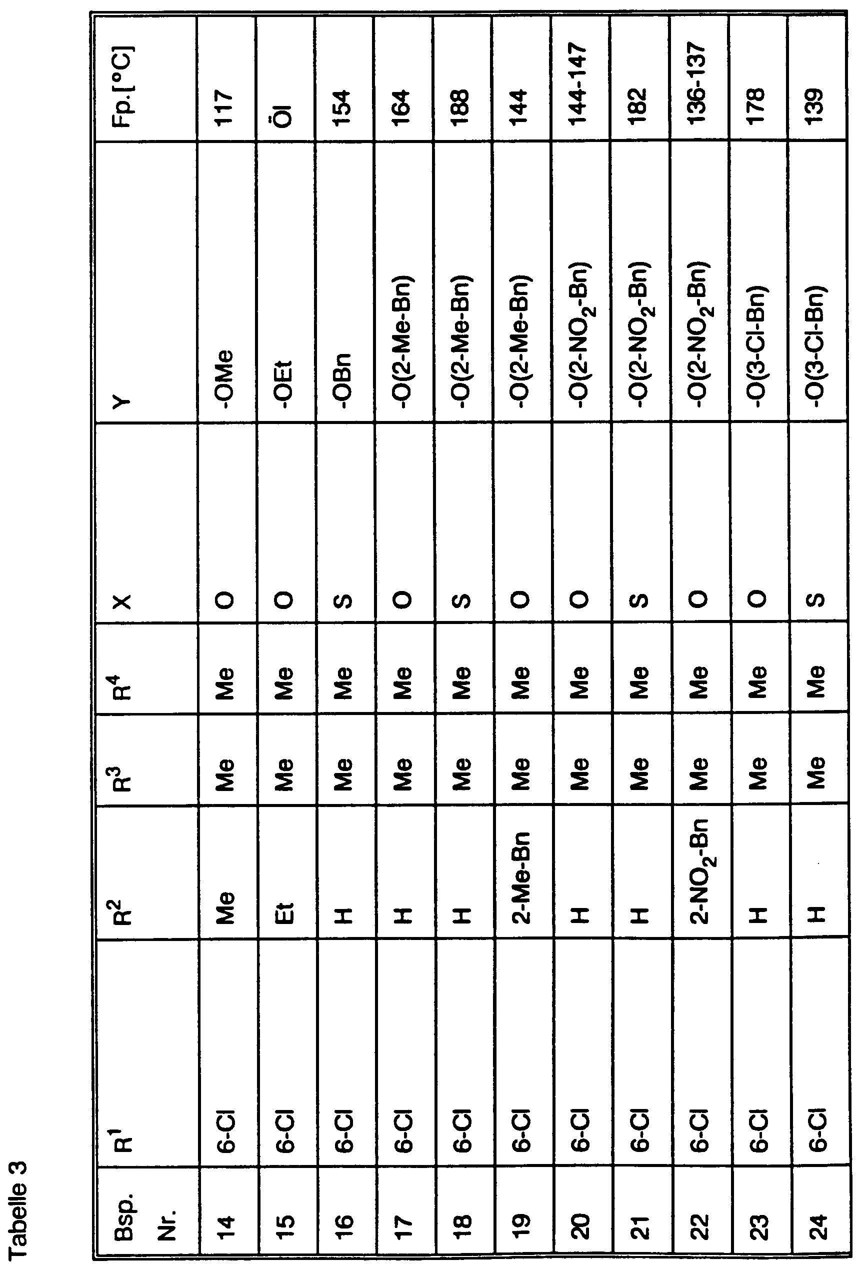

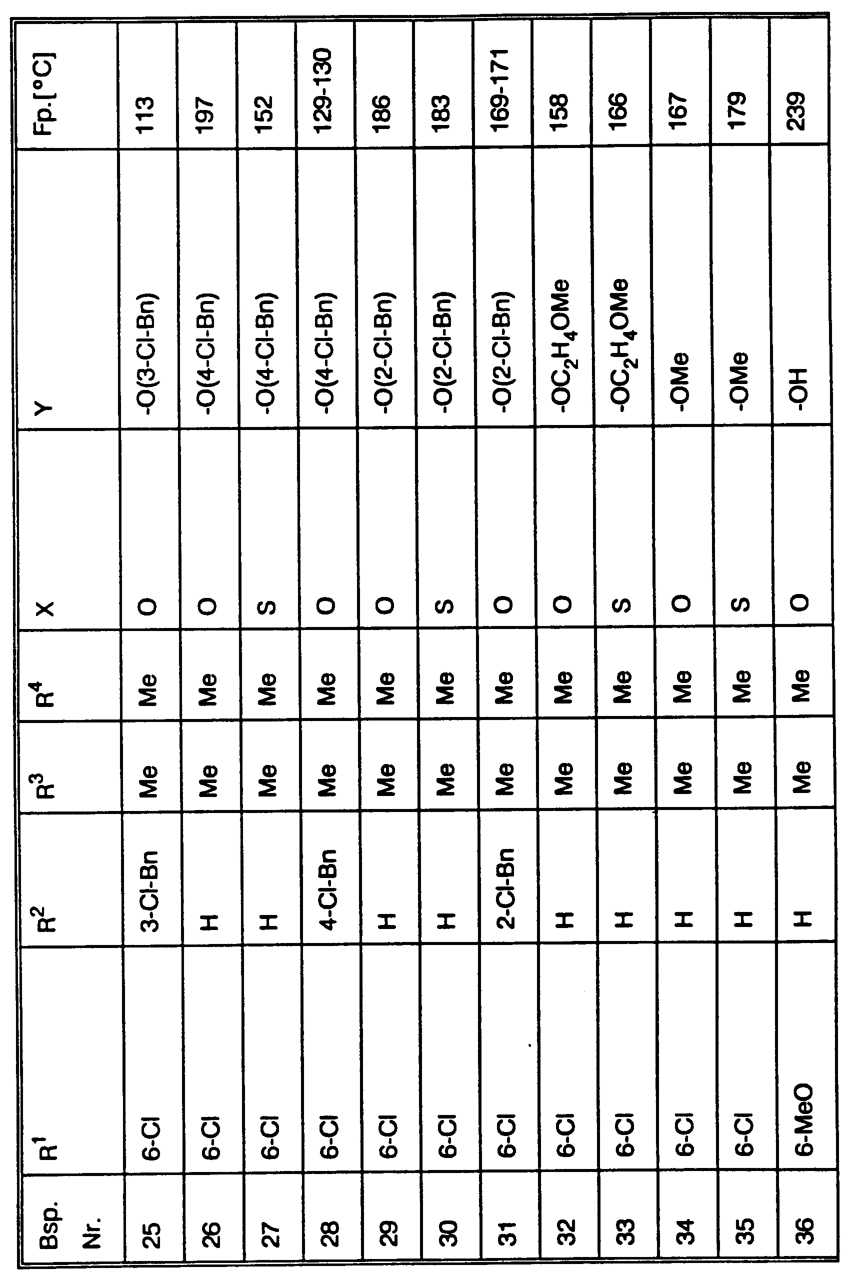

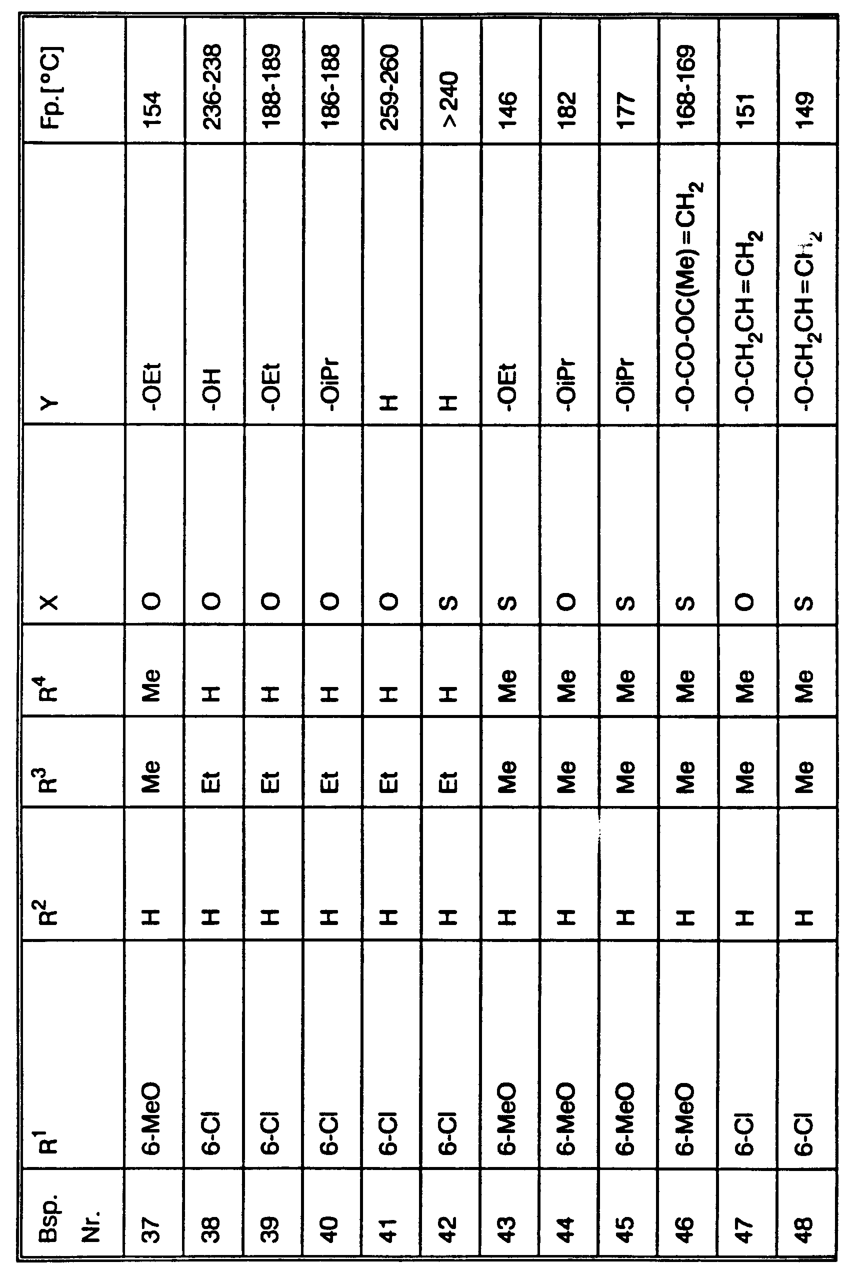

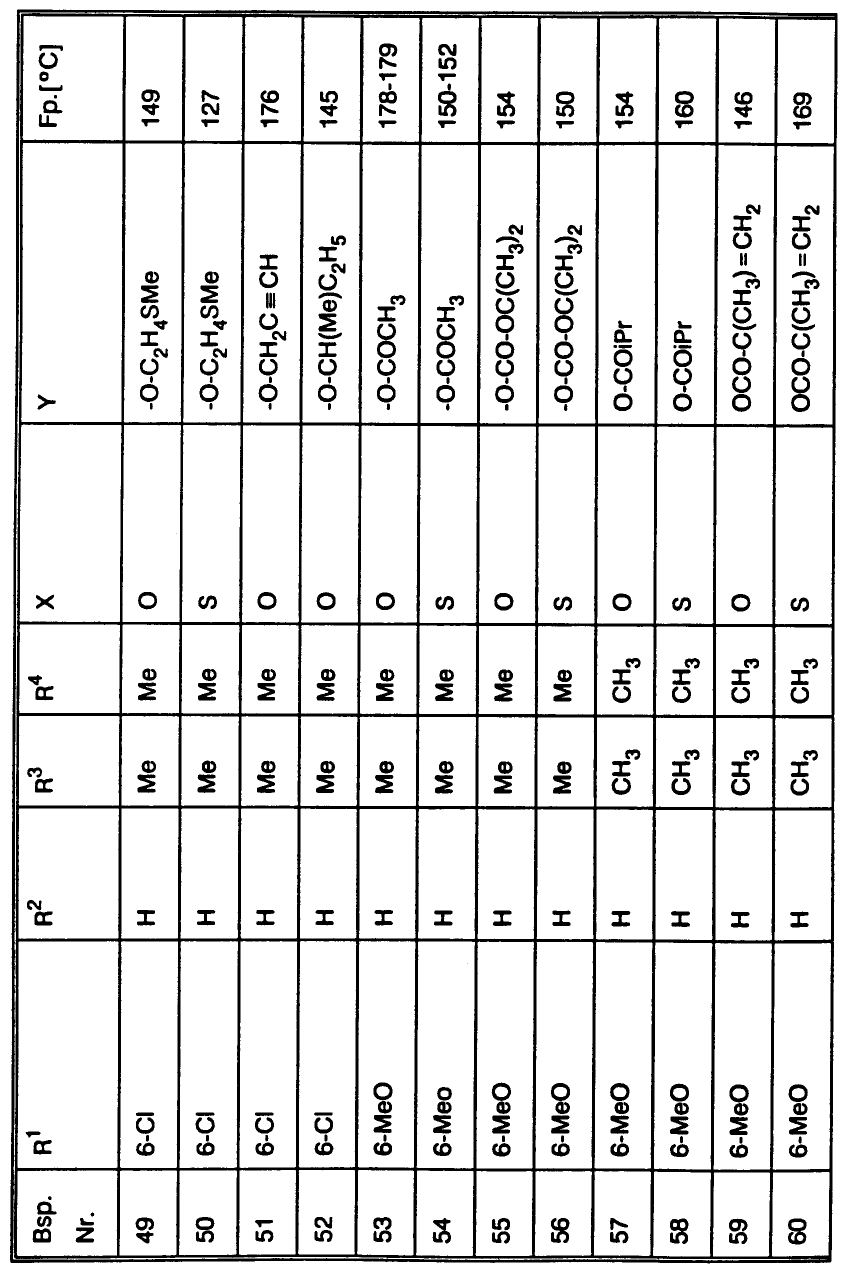

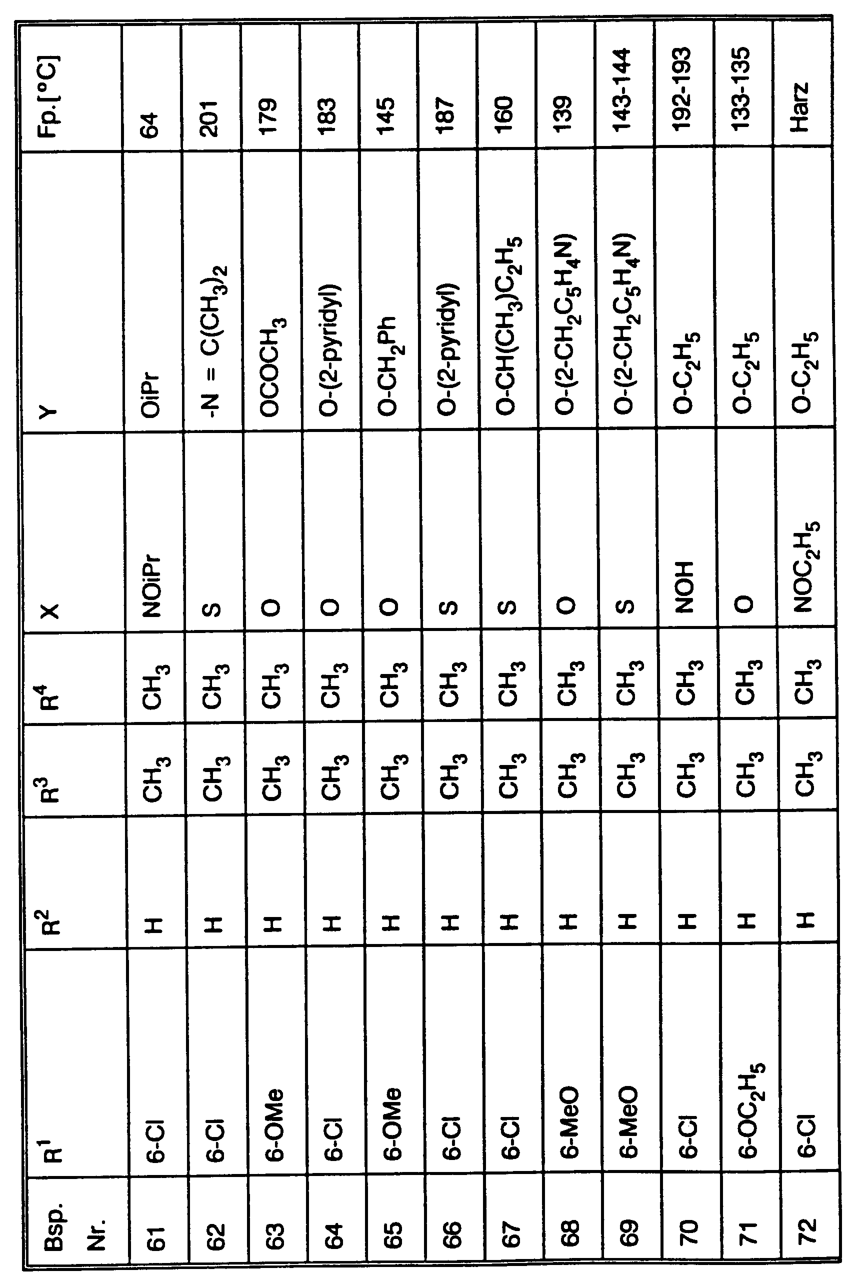

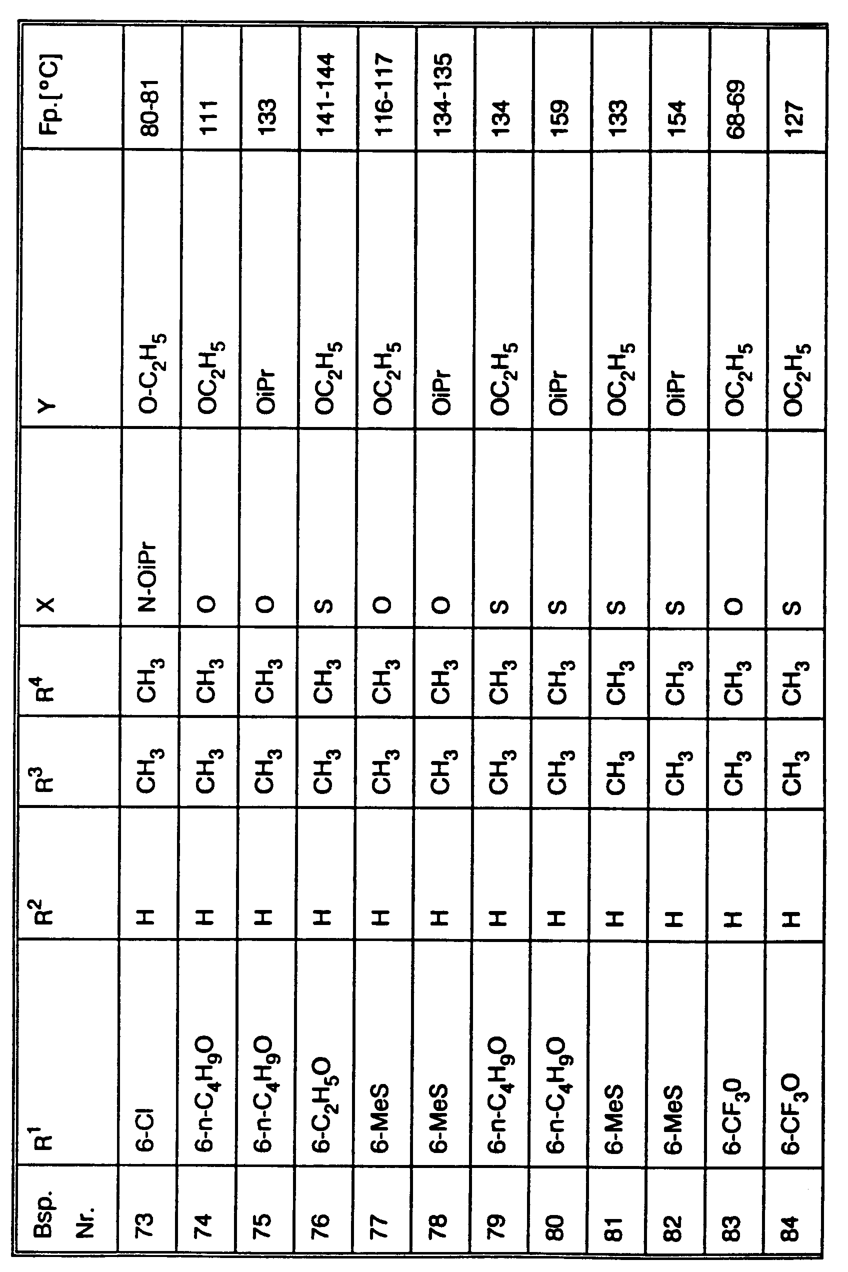

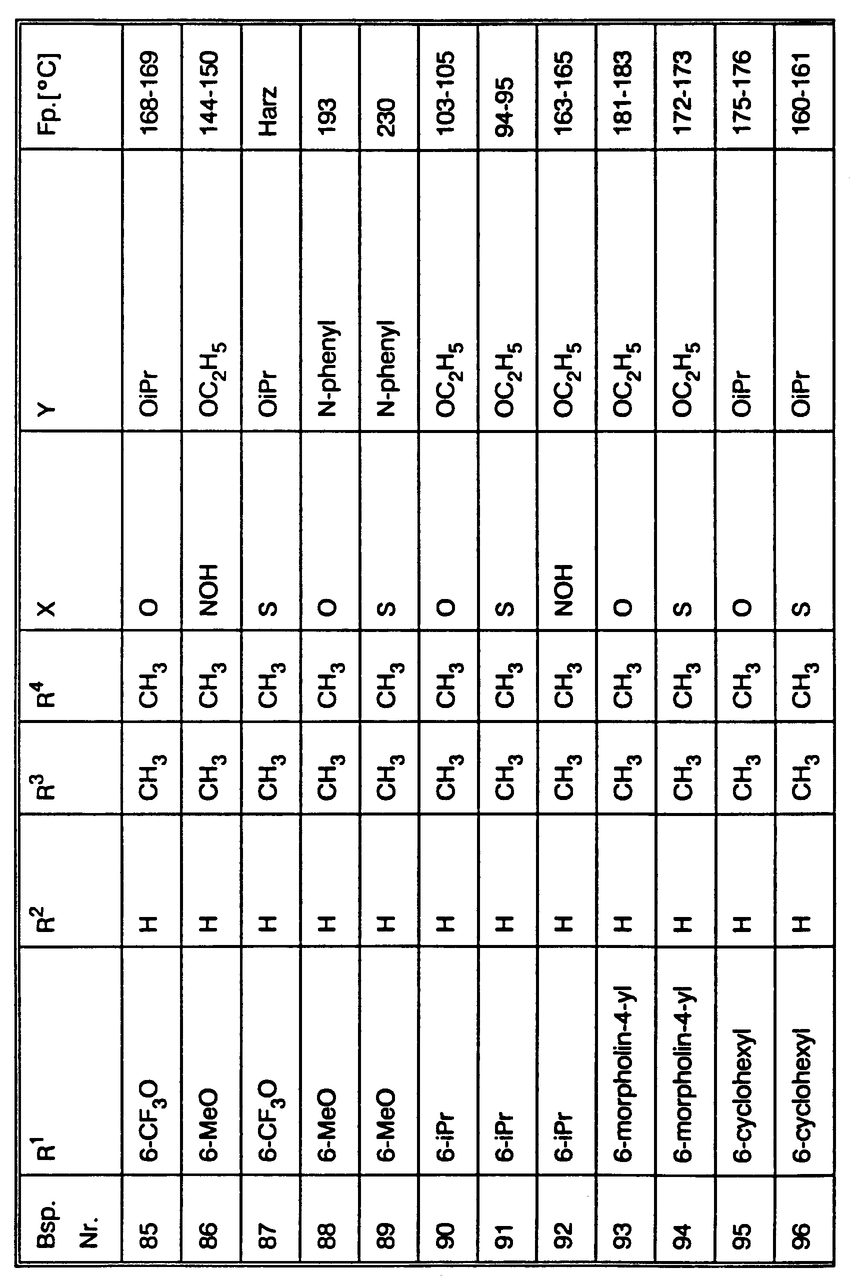

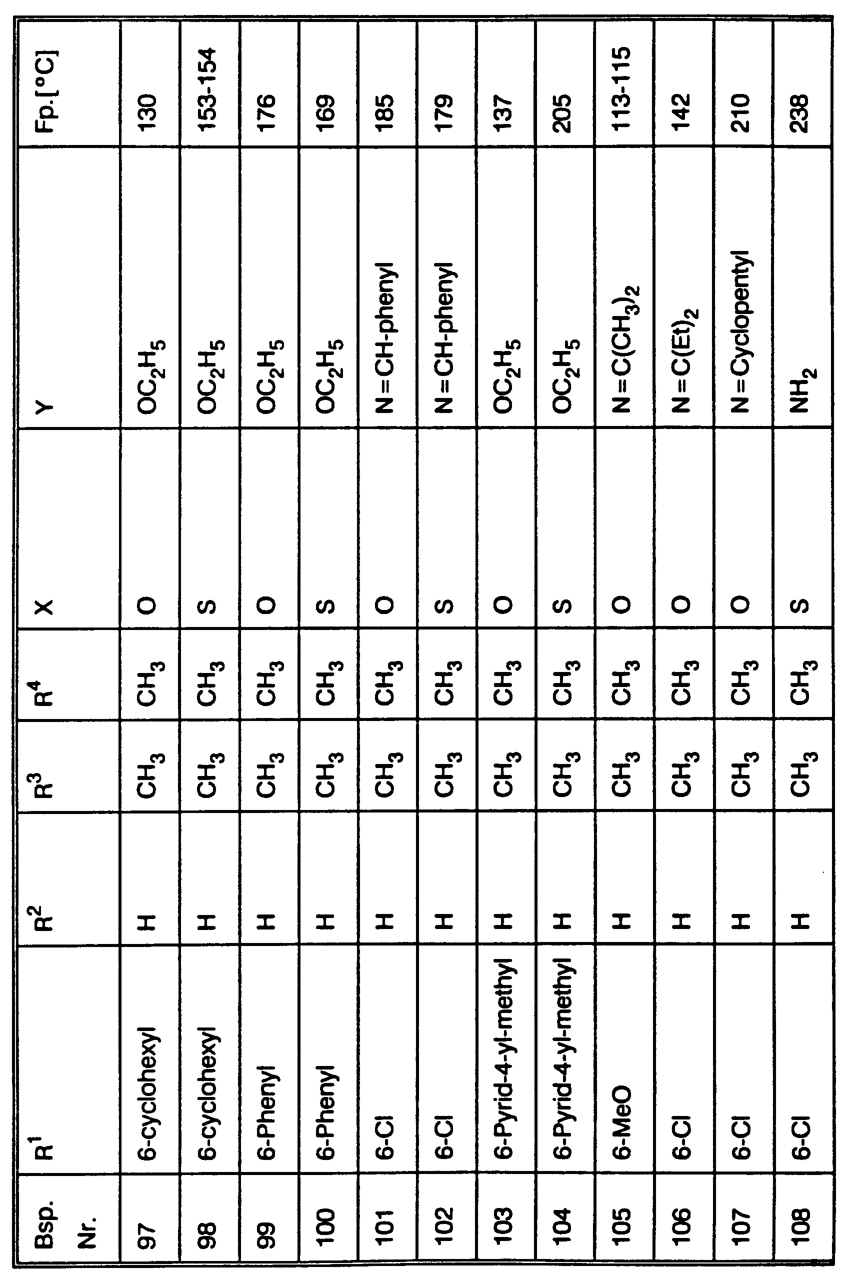

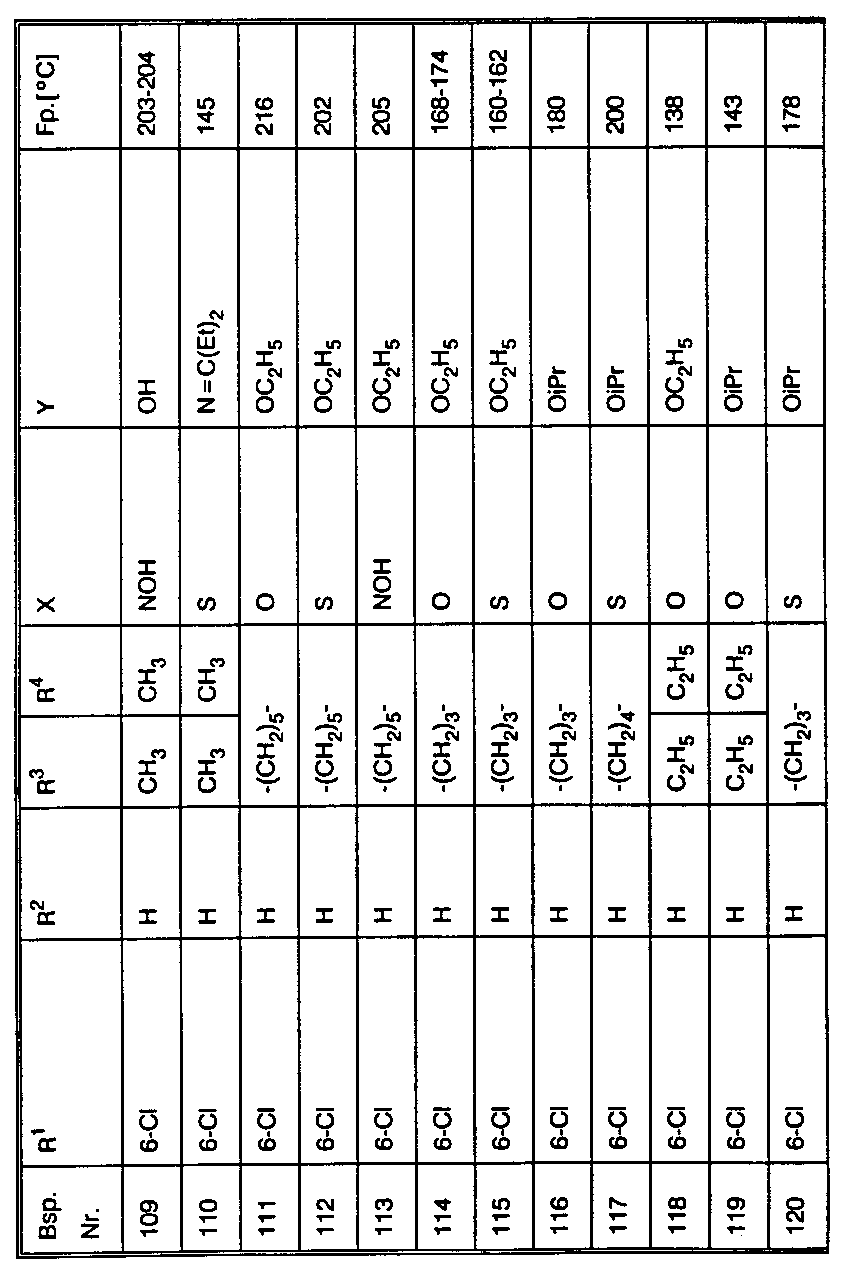

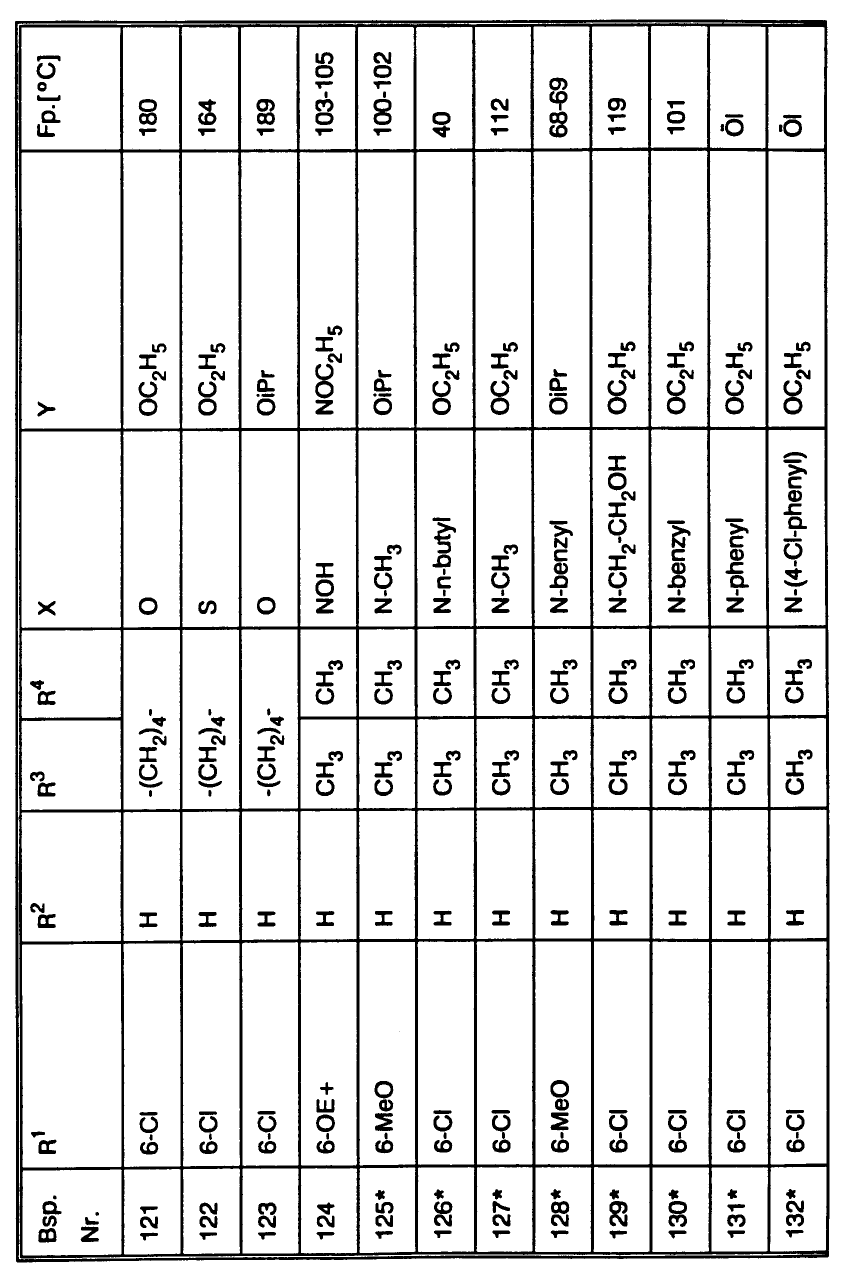

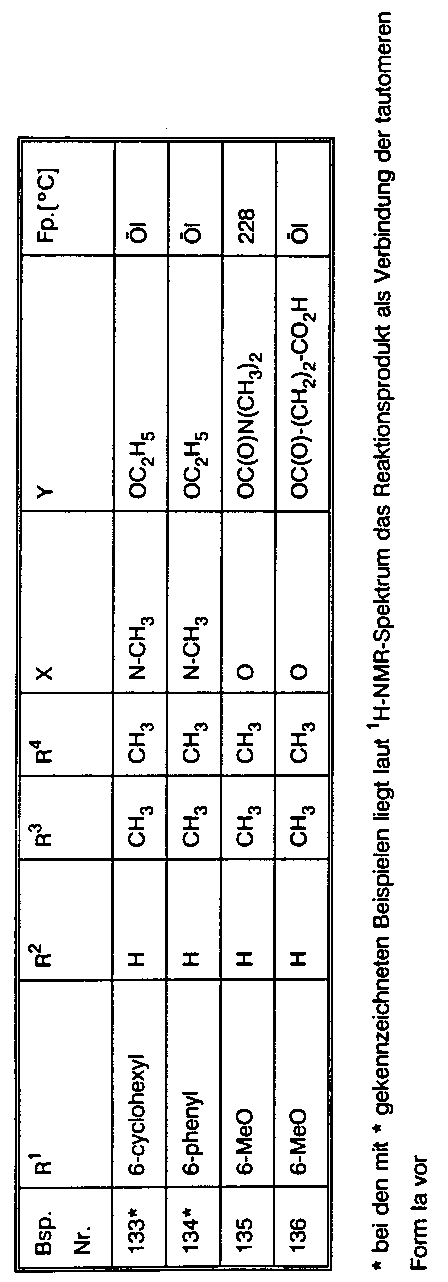

## Patentansprüche

1. Verbindungen der Formel I, 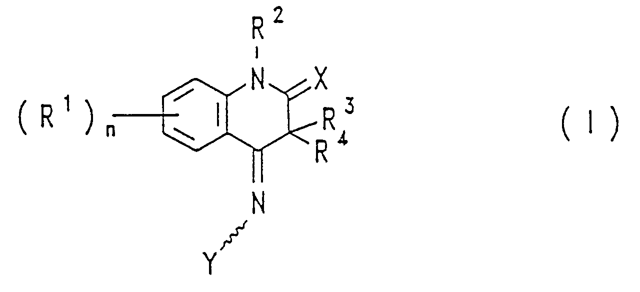 sowie deren tautomere Formen der allgemeinen Formeln Ia, Ib und Ic 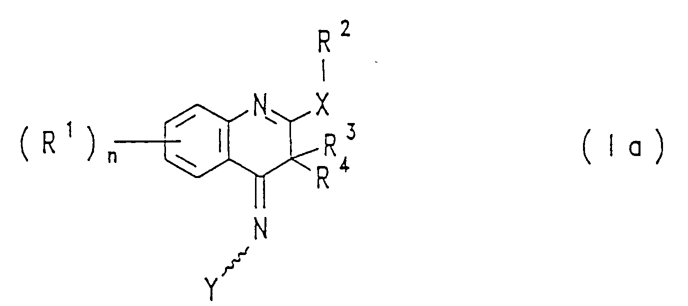 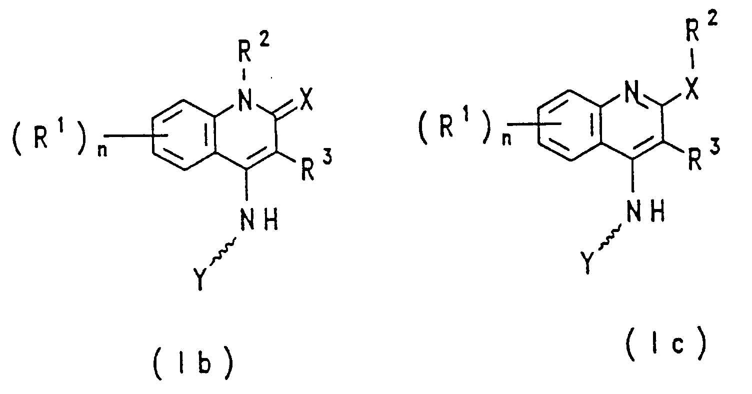 worin bedeuten:
n
null,
eins,
zwei,
drei,
oder vier,
die einzelnen Substituenten R1 unabhängig voneinander
Fluor, Chlor, Brom, Jod, Trifluormethyl, Trifluormethoxy, Hydroxy, C1-C8-Alkyl, C5-C8- Cycloalkyl, C1-C6-Alkoxy, (C1-C6-Alkoxy)-(C1-C4-alkoxy), C1-C6-Alkylthio, C1- C6-Alkylsulfinyl, C1-C6-Alkylsulfonyl, Nitro, Amino, Azido, C1-C6-Alkylamino, Di(C1 - C6-alkyl)amino, Piperidino, Morpholino, 1-Pyrrolidinyl, C1-C6-Acyl, C1-C6-Acyloxy, C1- C6-Acylamino, Cyano, Carbamoyl, Carboxy, (C1-C6-Alkyl)-oxycarbonyl, Hydroxysulfonyl, Sulfamoyl
oder
einen gegebenenfalls mit bis zu fünf voneinander unabhängigen Resten R5 substituierten Phenyl-, Phenoxy-, Phenoxycarbonyl-, Phenylthio-, Phenylsulfinyl-, Phenylsulfonyl-, Phenoxysulfonyl-, Phenylsulfonyloxy-, Phenylsulfonylamino-, Benzoyl-, Heteroaroyl-, Heteroaryl- oder Heteroarylmethylrest,
wobei R5
Fluor, Chlor, Brom, Jod, Cyano, Trifluormethyl, Trifluormethoxy, Nitro, Amino, Azido, C1-C6-Alkyl, C3-C8-Cycloalkyl, C1-C6-Alkoxy, C1-C6-Alkylthio, C1-C6-Alkylsulfinyl, C1- C6-Alkylsulfonyl, C1-C6-Alkylamino, Di(C1-C6-alkyl)amino, (C1-C6-Alkyl)-oxycarbonyl, Phenyl, Phenoxy oder Heteroaryl
sein kann,
X bedeutet Sauerstoff, Schwefel, Selen oder substituierten Stickstoff N-R2, N-O-R2, worin R2 die unten gegebenen Bedeutungen haben kann,
Y bedeutet R6, O-R6, S-R6, N-R6R7, N = CR6R7 oder C-R6R7R8, wobei R6, R7 und R8 die unten gegebenen Bedeutungen haben können,
R2, R6, R7 und R8 können gleich oder verschieden, unabhängig voneinander Wasserstoff,
C1-C8-Alkyl, gegebenenfalls substituiert mit Fluor, Chlor, Brom, Jod, Cyano, Amino, Mercapto, Hydroxy, C1-C6-Acyloxy, Benzoyloxy, Benzyloxy, Phenoxy, C1-C6-Alkoxy, C1-C6-Alkylamino, Di(C1-C6-alkyl)amino, C1-C6-Alkylthio, C1-C6-Alkylsulfonyl, Phenylsulfonyl, Oxo, Thioxo, Carboxy, Carbamoyl;
C2-C8-Alkenyl, gegebenenfalls substituiert mit Fluor, Chlor, Brom, Jod, Cyano, Amino, Mercapto, Hydroxy, C1-C6-Acyloxy, Benzoyloxy, Benzyloxy, Phenoxy, C1-C6-Alkoxy, C1-C6-Alkylamino, Di(C1-C6-alkyl)amino, C1-C6-Alkylthio, C1-C6-Alkylsulfonyl, Phenylsulfonyl, Oxo, Thioxo, Carboxy, Carbamoyl;
C3-C8-Alkinyl, gegebenenfalls substituiert mit Fluor, Chlor, Brom, Jod, Cyano, Amino, Mercapto, Hydroxy, C1-C6-Acyloxy, Benzoyloxy, Benzyloxy, Phenoxy, C1-C6-Alkoxy, C1-C6-Alkylamino, Di(C1-C6-alkyl)amino, C1-C6-Alkylthio, C1-C6-Alkylsulfonyl, Phenylsulfonyl, Oxo, Thioxo, Carboxy, Carbamoyl;
C3-C8-Cycloalkyl, gegebenenfalls substituiert mit Fluor, Chlor, Brom, Jod, Cyano, Amino, Mercapto, Hydroxy, C1-C6-Acyloxy, Benzoyloxy, Benzyloxy, Phenoxy, C1-C6-Alkoxy, C1-C6-Alkylamino, Di(C1-C6-alkyl)amino, C1-C6-Alkylthio, C1-C6-Alkylsulfonyl, Phenylsulfonyl, Oxo, Thioxo, Carboxy, Carbamoyl;
C5-C8-Cycloalkenyl, gegebenenfalls substituiert mit Fluor, Chlor, Brom, Jod, Cyano, Amino, Mercapto, Hydroxy, C1-C6-Acyloxy, Benzoyloxy, Benzyloxy, Phenoxy, C1-C6-Alkoxy, C1-C6-Alkylamino, Di(C1-C6- alkyl)amino, C1-C6-Alkylthio, C1-C6-Alkylsulfonyl, Phenylsulfonyl, Oxo, Thioxo, Carboxy, Carbamoyl;
(C3-C8-Cycloalkyl)-(C1-C4-alkyl), gegebenenfalls substituiert mit Fluor, Chlor, Brom, Jod, Cyano, Amino, Mercapto, Hydroxy, C1-C6-Acyloxy, Benzoyloxy, Benzyloxy, Phenoxy, C1-C6-Alkoxy, C1-C6-Alkylamino, Di(C1-C6-alkyl)amino, C1-C6-Alkylthio, C1-C6-Alkylsulfonyl, Phenylsulfonyl, Oxo, Thioxo, Carboxy, Carbamoyl;
(C5-C8-Cycloalkenyl)-(C1-C4-alkyl), gegebenenfalls substituiert mit Fluor, Chlor, Brom, Jod, Cyano, Amino, Mercapto, Hydroxy, C1-C6-Acyloxy, Benzoyloxy, Benzyloxy, Phenoxy, C1-C6-Alkoxy, C1-C6-Alkylamino, Di(C1-C6-alkyl)amino, C1-C6-Alkylthio,
C1-C6-Alkylsulfonyl, Phenylsulfonyl, Oxo, Thioxo, Carboxy, Carbamoyl;
C1-C6-Alkylcarbonyl, gegebenenfalls substituiert mit Fluor, Chlor, Brom, Jod, Cyano, Amino, Mercapto, Hydroxy, C1-C6-Acyloxy, Benzoyloxy, Benzyloxy, Phenoxy, C1- C6-Alkoxy, C1-C6-Alkylamino, Di(C1-C6-alkyl)amino, C1-C6-Alkylthio, C1- C6-Alkylsulfonyl, Phenylsulfonyl, Oxo, Thioxo, Carboxy, Carbamoyl;
C2-C8-Alkenylcarbonyl, gegebenenfalls substituiert durch Fluor, Chlor oder Hydroxy, C1-C4-Alkoxy, Oxo, Phenyl;
(C3-C8-Cycloalkyl)carbonyl, gegebenenfalls substituiert durch Fluor, Chlor oder Hydroxy, C1-C4-Alkoxy, Oxo, Phenyl;
(C5-C8-Cycloalkenyl)carbonyl, gegebenenfalls substituiert durch Fluor, Chlor oder Hydroxy, C1-C4-Alkoxy, Oxo, Phenyl;
(C3-C8-Cycloalkyl)-(C1-C3-alkyl)carbonyl, gegebenenfalls substituiert durch Fluor, Chlor oder Hydroxy, C1-C4-Alkoxy, Oxo, Phenyl;
(C5-C6-Cycloalkenyl)-(C1-C3-alkyl)carbonyl, gegebenenfalls substituiert durch Fluor, Chlor oder Hydroxy, C1-C4-Alkoxy, Oxo, Phenyl;
C1-C8-Alkyloxycarbonyl, gegebenenfalls substituiert durch Fluor, Chlor, Brom, Hydroxy, C1-C4-Alkoxy, C1-C4-Alkylamino, Di(C1-C4-alkyl)amino, C1-C4-Alkylthio, C2-C8-Alkenyloxycarbonyl, gegebenenfalls substituiert durch Fluor, Chlor, Hydroxy, C1-C4-Alkoxy, Oxo, Phenyl;
C2-C8-Alkinyloxycarbonyl, gegebenenfalls substituiert durch Fluor, Chlor, Hydroxy, C1-C4-Alkoxy, Oxo, Phenyl;
C1-C8-Alkylthiocarbonyl,gegebenenfalls substituiert durch Fluor, Chlor, Hydroxy, C1-C4-Alkoxy, Oxo, Phenyl;
C2-C8-Alkenylthiocarbonyl, gegebenenfalls substituiert durch Fluor, Chlor, Hydroxy, C1-C4-Alkoxy, Oxo, Phenyl;
C1-C8-Alkylamino- und Di(C1-C8-alkyl)aminocarbonyl, gegebenenfalls substituiert durch Fluor, Chlor, Hydroxy, C1-C4-Alkoxy, Oxo, Phenyl;
C2-C8-Alkenylamino- und Di(C2-C6-alkenyl)aminocarbonyl, gegebenenfalls substituiert durch Fluor, Chlor, Hydroxy, C1-C4-Alkoxy, Oxo, Phenyl;
C1-C6-Alkylsulfonyl, gegebenenfalls substituiert durch Fluor, Chlor, Hydroxy, C1-C4-Alkoxy, C1-C4-Alkylthio, Oxo, Phenyl;
C2-C6-Alkenylsulfonyl, gegebenenfalls substituiert durch Fluor, Chlor, Hydroxy, C1-C4-Alkoxy, Oxo, Phenyl;
oder mit bis zu fünf voneinander unabhängigen Resten R5 substituiertes Aryl, Arylcarbonyl, Aryl(thiocarbonyl), (Arylthio)carbonyl, (Arylthio)thiocarbonyl, Aryloxycarbonyl, (Arylamino)thiocarbonyl, Arylsulfonyl, Arylalkyl, Arylalkenyl, Arylalkinyl, Arylalkylcarbonyl, Arylalkenylcarbonyl, Arylalkoxycarbonyl, wobei der Alkylrest jeweils 1 bis 5 C-Atome enthalten kann und R5 wie oben definiert ist
oder mit bis zu drei voneinander unabhängigen Resten R5 substituiertes Heteroaryl, Heteroarylalkyl, Heteroarylalkenyl, Heteroarylalkylcarbonyl oder Heteroarylalkenylcarbonyl, wobei der Alkylrest jeweils 1 bis 3 C-Atome enthalten kann,
und
R3 und R4 gleich oder verschieden, unabhängig voneinander
Wasserstoff,
C1-C8-Alkyl, gegebenenfalls substituiert mit Fluor, Chlor, Hydroxy, Amino, Mercapto, C1-C4-Acyloxy, Benzoyloxy, Benzyloxy, Phenoxy, C1-C4-Alkoxy, C1-C4-Alkylamino, Di(C1-C4-alkyl)amino, C1-C4-Alkylthio, C1-C4-Alkylsulfonyl, C1-C4-Alkylsulfinyl, Carboxy, Carbamoyl;
C2-C8-Alkenyl, gegebenenfalls substituiert mit Fluor oder Chlor, Hydroxy, Amino, Mercapto, C1-C4-Acyloxy, Benzoyloxy, Benzyloxy, Phenoxy, C1-C4-Alkoxy, C1-C4-Alkylamino, Di(C1-C4-alkyl)amino, C1-C4-Alkylthio, C1-C4-Alkylsulfonyl, C1-C4-Alkylsulfinyl, Carboxy, Carbamoyl;
C3-C8-Cycloalkyl, gegebenenfalls substituiert mit Fluor, Chlor, Hydroxy, Amino, Mercapto, C1-C4-Acyloxy, Benzoyloxy, Benzyloxy, Phenoxy, C1-C4-Alkoxy, C1-C4-Alkylamino, Di(C1-C4-alkyl)amino, C1-C4-Alkylthio, C1-C4-Alkylsulfonyl, C1-C4-Alkylsulfinyl, Carboxy, Carbamoyl;
C3-C8-Cycloalkenyl, gegebenenfalls substituiert mit Fluor oder Chlor, Hydroxy, Amino, Mercapto, C1-C4-Acyloxy, Benzoyloxy, Benzyloxy, Phenoxy, C1 - C4-Alkoxy, C1 - C4-Alkylamino, Di(C1 - C4-alkyl)amino, C1-C4-Alkylthio, C1-C4-Alkylsulfonyl, C1- C4-Alkylsulfinyl; Carboxy, Carbamoyl;
mit bis zu fünf voneinander unabhängigen Resten R5 substituiertes Aryl, Arylalkyl, Heteroaryl oder Heteroarylalkyl bedeuten, wobei der Alkylrest jeweils 1 bis 3 C-Atome enthalten kann und R5 wie oben definiert ist;
R3 und R4 können zusammen auch
einen über eine Doppelbindung verknüpften Rest = C-Z1Z2 bedeuten, wobei Z1 und Z2 die oben für R3 und R4 gegebene Bedeutung haben,
R3 und R4 können ferner auch
Teil eines gesättigten oder ungesättigten carbo- oder heterocyclischen Ringes sein, der gegebenenfalls mit Fluor, Chlor, Hydroxy, Amino, C1 - C8-Alkyl, C2 - C8-Alkenyl, C2 - C8-Alkinyl, C1 - C8-Acyloxy, Benzoyloxy, C1 - C8-Alkoxy, C1 - C8-Alkylthio, Oxo, Thioxo, Carboxy, Carbamoyl oder Phenyl substituiert sein kann,
deren optische Isomere, Diastereomere in reiner Form oder in Form ihrer Mischungen und deren Additionsalze,
wobei in den vorstehenden Definitionen die heterocyclischen Ringe und Heteroarylgruppen 1-15 C-Atome und 1-6 Heteroatome ausgewählt aus der Gruppe O, S und N enthalten und wobei im Falle eines an dieser Stelle gesättigten N-haltigen Ringes N-Z vorliegt, worin Z H oder R2 mit den jeweiligen oben beschriebenen Definitionen bedeutet, mit Ausnahme der Verbindungen, in denen gleichzeitig R3 und/oder R4 Wasserstoff und Y R6 oder CR6R7R8 bedeuten
und mit Ausnahme der Verbindungen in denen gleichzeitig n = 0 oder 1, R1 = CH₃ oder OCH₃, R2 = CH₃, R3 = CH₂CH₂OCH₃, X = O und Y = H bedeuten und mit Ausnahme der Verbindungen in denen gleichzeitig n = 1, R1 = Cl, R2 = H oder CH₃, R3 = OH, R4 = Phenyl, X = O und Y = H oder OH bedeuten.

2. Verbindungen der Formel I, Ia, Ib bzw. Ic gemäß Anspruch 1, worin bedeuten:
n
null,
eins,
oder zwei,
die einzelnen Substituenten R1 unabhängig voneinander
Fluor, Chlor, Brom, Jod, Trifluormethyl, Trifluormethoxy, Hydroxy, C1-C6-Alkyl, C5-C6-Cycloalkyl, C1-C4-Alkoxy, (C1-C4-Alkoxy)-(C1-C2-alkoxy), C1-C4-Alkylthio, C1- C4-Alkylsulfinyl, C1-C4-Alkylsulfonyl, Nitro, Amino, C1-C4-Alkylamino, Di(C1-C4-alkyl)amino, C1-C6-Acyl, C1-C4-Acyloxy, C1-C4-Acylamino, Cyano, Carbamoyl, Carboxy, (C1-C4-Alkyl)-oxycarbonyl
oder
einen gegebenenfalls mit bis zu drei
voneinander unabhängigen Resten R5 substituierten Phenyl-, Phenoxy-, Phenoxycarbonyl-, Phenylthio-, Phenylsulfinyl-, Phenylsulfonyl-, Phenoxysulfonyl-, Phenylsulfonyloxy-, Phenylsulfonylamino-, Benzoyl-, Heteroaroyl-, Heteroaryl- oder Heteroarylmethylrest,
wobei R5
Fluor, Chlor, Trifluormethyl, Trifluormethoxy, Nitro, Amino, C1-C4-Alkyl, C3-C6-Cycloalkyl, C1-C4-Alkoxy, C1-C4-Alkylthio, C1-C4-Alkylsulfinyl, C1-C4-Alkylsulfonyl, C1-C4-Alkylamino, Di(C1-C4-alkyl)amino, Phenyl, Phenoxy oder Heteroaryl
sein kann,
X bedeutet Sauerstoff, Schwefel oder substituierten Stickstoff N-R2, N-O-R2, worin R2 die unten gegebenen Bedeutungen haben kann,
Y bedeutet R6, O-R6, S-R6, N-R6R7, N=CR6R7 oder C-R6R7R8, wobei R6, R7 und R8 die unten gegebenen Bedeutungen haben können,
R2, R6, R7 und R8 können gleich oder verschieden, unabhängig voneinander Wasserstoff,
C1-C6-Alkyl, gegebenenfalls substituiert mit Fluor, Chlor, Brom, Jod, Cyano, Amino, Mercapto, Hydroxy, C1-C4-Acyloxy, Benzoyloxy, Benzyloxy, Phenoxy, C1-C4-Alkoxy, C1-C4-Alkylamino, Di(C1-C4-alkyl)amino, C1-C4-Alkylthio, C1-C4-Alkylsulfonyl, Phenylsulfonyl, Oxo, Thioxo, Carboxy, Carbamoyl;
C2-C6-Alkenyl, gegebenenfalls substituiert mit Fluor, Chlor, Cyano, Amino, Mercapto, Hydroxy, C1-C4-Acyloxy, Benzoyloxy, Benzyloxy, Phenoxy, C1-C4-Alkoxy, C1- C4-Alkylamino, Di(C1-C4-alkyl)amino, C1-C4-Alkylthio, C1-C4-Alkylsulfonyl, Phenylsulfonyl, Carboxy, Carbamoyl;
C3-C6-Alkinyl, gegebenenfalls substituiert mit Fluor, Chlor, Cyano, Amino, Mercapto, Hydroxy, C1-C4-Acyloxy, Benzoyloxy, Benzyloxy, Phenoxy, C1-C4-Alkoxy, C1- C4-Alkylamino, Di(C1-C4-alkyl)amino, C1-C4-Alkylthio, C1-C4-Alkylsulfonyl, Phenylsulfonyl, Carboxy, Carbamoyl;
C3-C6-Cycloalkyl, gegebenenfalls substituiert mit Fluor, Chlor, Cyano, Amino, Mercapto, Hydroxy, C1-C4-Acyloxy, Benzoyloxy, Benzyloxy, Phenoxy, C1-C4-Alkoxy, C1-C4-Alkylamino, Di(C1-C4-alkyl)amino, C1-C4-Alkylthio, C1-C4-Alkylsulfonyl, Phenylsulfonyl, Carboxy, Carbamoyl;
C5-C6-Cycloalkenyl, gegebenenfalls substituiert mit Fluor, Chlor, Cyano, Amino, Mercapto, Hydroxy, C1-C4-Acyloxy, Benzoyloxy, Benzyloxy, Phenoxy, C1-C4-Alkoxy, C1-C4-Alkylamino, Di(C1-C4-alkyl)amino, C1-C4-Alkylthio, C1-C4-Alkylsulfonyl, Phenylsulfonyl, Carboxy, Carbamoyl;
(C3-C6-Cycloalkyl)-(C1-C2-alkyl), gegebenenfalls substituiert mit Fluor, Chlor, Cyano, Amino, Mercapto, Hydroxy, C1-C4-Acyloxy, Benzoyloxy, Benzyloxy, Phenoxy, C1-C4-Alkoxy, C1-C4-Alkylamino, Di(C1-C4-alkyl)amino, C1-C4-Alkylthio, C1-C4-Alkylsulfonyl, Phenylsulfonyl, Carboxy, Carbamoyl;
(C5-C6-Cycloalkenyl)-(C1-C2-alkyl), gegebenenfalls substituiert mit Fluor, Chlor, Cyano, Amino, Mercapto, Hydroxy, C1-C4-Acyloxy, Benzoyloxy, Benzyloxy, Phenoxy, C1-C4-Alkoxy, C1-C4-Alkylamino, Di(C1-C4-alkyl)amino, C1-C4-Alkylthio, C1- C4-Alkylsulfonyl, Phenylsulfonyl, Carboxy, Carbamoyl;
C1-C6-Alkylcarbonyl, gegebenenfalls substituiert mit Fluor, Chlor, Cyano, Amino, Mercapto, Hydroxy, C1-C4-Acyloxy, Benzoyloxy, Benzyloxy, Phenoxy, C1-C4-Alkoxy, C1-C4-Alkylamino, Di(C1-C4-alkyl)amino, C1-C4-Alkylthio, C1-C4-Alkylsulfonyl, Phenylsulfonyl, Carboxy, Carbamoyl;
C2-C6-Alkenylcarbonyl, gegebenenfalls substituiert durch Fluor, Chlor, Hydroxy, C1- C4-Alkoxy, Oxo, Phenyl;
(C3-C6-Cycloalkyl)carbonyl, gegebenenfalls substituiert durch Fluor, Chlor, Hydroxy, C1-C4-Alkoxy, Oxo, Phenyl;
(C5-C6-Cycloalkenyl)carbonyl, gegebenenfalls substituiert durch Fluor, Chlor, Hydroxy, C1-C4-Alkoxy, Oxo, Phenyl;
(C3-C6-Cycloalkyl)-(C1-C2-alkyl)carbonyl, gegebenenfalls substituiert durch Fluor, Chlor, Hydroxy, C1-C4-Alkoxy, Oxo, Phenyl;
(C5-C6-Cycloalkenyl)-(C1-C2-alkyl)carbonyl, gegebenenfalls substituiert durch Fluor, Chlor, Hydroxy, C1-C4-Alkoxy, Oxo, Phenyl;
C1-C6-Alkyloxycarbonyl, gegebenenfalls substituiert durch Fluor, Chlor, C1-C4-Alkoxy, C1-C4-Alkylamino, Di(C1-C4-alkyl)amino, C1-C4-Alkylthio;
C2-C6-Alkenyloxycarbonyl, gegebenenfalls substituiert durch Fluor, Chlor, C1-C4-Alkoxy, Phenyl;
C2-C6-Alkinyloxycarbonyl, gegebenenfalls substituiert durch Fluor, Chlor, C1-C4-Alkoxy, Phenyl;
C1-C6-Alkylthiocarbonyl,gegebenenfalls substituiert durch Fluor, Chlor, C1-C4-Alkoxy, Phenyl;
C2-C6-Alkenylthiocarbonyl, gegebenenfalls substituiert durch Fluor, Chlor, C1-C4-Alkoxy, Phenyl;
C1-C6-Alkylamino- und Di(C1-C6-alkyl)aminocarbonyl, gegebenenfalls substituiert durch Fluor, Chlor, C1 - C4-Alkoxy, Phenyl,
C2 - C6-Alkenylamino- und Di(C2 - C6-alkenyl)aminocarbonyl, gegebenenfalls substituiert durch Fluor, Chlor, C1 - C4-Alkoxy, Phenyl,
C1 - C6-Alkylsulfonyl, gegebenenfalls substituiert durch Fluor, Chlor, C1 - C4-Alkoxy, Phenyl,
C2 - C6-Alkenylsulfonyl,
oder mit bis zu drei voneinander unabhängigen Resten R5 substituiertes Aryl, Arylcarbonyl, Aryl(thiocarbonyl), (Arylthio)carbonyl, (Arylthio)thiocarbonyl, Aryloxycarbonyl, (Arylamino)thiocarbonyl, Arylsulfonyl, Arylalkyl, Arylalkenyl, Arylalkinyl, Arylalkylcarbonyl, Arylalkenylcarbonyl, Arylalkoxycarbonyl, wobei der Alkylrest jeweils 1 bis 4 C-Atome enthalten kann und R5 wie oben definiert ist
oder mit bis zu drei voneinander unabhängigen Resten R5 substituiertes Heteroaryl, Heteroarylalkyl, Heteroarylalkenyl, Heteroarylalkylcarbonyl oder Heteroarylalkenylcarbonyl, wobei der Alkylrest jeweils 1 bis 3 C-Atome enthalten kann,
und
R3 und R4 gleich oder verschieden, unabhängig voneinander
C1-C6-Alkyl, gegebenenfalls substituiert mit Fluor, Chlor, Hydroxy, Amino, Mercapto, C1-C4-Acyloxy, Benzoyloxy, Benzyloxy, Phenoxy, C1-C4-Alkoxy, C1-C4-Alkylamino, Di(C1-C4-alkyl)amino, C1-C4-Alkylthio, C1-C4-Alkylsulfonyl, C1-C4-Alkylsulfinyl, Carboxy, Carbamoyl;
C2-C6-Alkenyl, gegebenenfalls substituiert mit Fluor oder Chlor, Phenoxy, C1-C4-Alkoxy, C1-C4-Alkylthio, C1-C4-Alkylsulfonyl, C1-C4-Alkylsulfinyl;
C3-C6-Cycloalkyl, gegebenenfalls substituiert mit Fluor, Chlor, Hydroxy, Amino, Mercapto, C1-C4-Acyloxy, Benzoyloxy, Benzyloxy, Phenoxy, C1-C4-Alkoxy, C1-C4-Alkylamino, Di(C1-C4-alkyl)amino, C1-C4-Alkylthio, C1-C4-Alkylsulfonyl, C1-C4-Alkylsulfinyl;
C3-C6-Cycloalkenyl, gegebenenfalls substituiert mit Fluor oder Chlor, Phenoxy, C1-C4-Alkoxy, C1-C4-Alkylthio, C1-C4-Alkylsulfonyl, C1-C4-Alkylsulfinyl;
mit bis zu drei voneinander unabhängigen Resten R5 substituiertes Aryl, Arylalkyl, Heteroaryl oder Heteroarylalkyl bedeuten, wobei der Alkylrest jeweils 1 bis 3 C-Atome enthalten kann und R5 wie oben definiert ist,
einer der Reste R3 oder R4 Wasserstoff sein kann,
R3 und R4 können zusammen auch
einen über eine Doppelbindung verknüpften Rest =C-Z1Z2 bedeuten, wobei Z1 und Z2 die oben für R3 und R4 gegebene Bedeutung haben.

3. Verbindungen der Formel I, Ia, Ib bzw. Ic gemäß den Ansprüchen 1-2, worin bedeuten:
n
null,
eins,
oder zwei,
die einzelnen Substituenten R1 unabhängig voneinander
Fluor, Chlor, Brom, Trifluormethyl, Trifluormethoxy, Hydroxy, C1-C4-Alkyl, C1-C4-Alkoxy, (C1-C4-Alkoxy)-(C1-C2-alkoxy), C1-C4-Alkylthio, C1-C4-Alkylsulfinyl, C1-C4-Alkylsulfonyl, Nitro, Amino, C1-C4-Alkylamino, Di(C1-C4-alkyl)amino, C1-C4-Acyl, C1-C4-Acyloxy, C1-C4-Acylamino, Cyano, Carbamoyl, Carboxy, (C1-C4- Alkyl)-oxycarbonyl
oder
einen gegebenenfalls mit bis zu drei
voneinander unabhängigen Resten R5 substituierten Phenyl-, Phenoxy-, Phenoxycarbonyl-, Phenylthio-, Phenylsulfinyl-, Phenylsulfonyl-, Benzoyl-, Heteroaroyl-, Heteroaryl- oder Heteroarylmethylrest,
wobei R5
Fluor, Chlor, Trifluormethyl, Trifluormethoxy, Nitro, Amino, C1- C4-Alkyl, C1-C4-Alkoxy, C1-C4-Alkylthio, C1-C4-Alkylsulfinyl,
C1-C4-Alkylsulfonyl, C1-C4-Alkylamino, Di(C1-C4-alkyl)amino, Phenyl oder Phenoxy sein kann,
X bedeutet Sauerstoff, Schwefel oder substituierten Stickstoff N-R2, N-O-R2, worin R2 die unten gegebenen Bedeutungen haben kann,
Y bedeutet O-R6, S-R6, N-R6R7 oder N =CR6R7, wobei R6, R7 und R8 die unten gegebenen Bedeutungen haben können,
R2, R6, R7 und R8 können gleich oder verschieden, unabhängig voneinander Wasserstoff,
C1-C6-Alkyl, gegebenenfalls substituiert mit Fluor, Chlor, Amino, Mercapto, Hydroxy, C1-C4-Acyloxy, Benzoyloxy, Benzyloxy, Phenoxy, C1-C4-Alkoxy, C1-C4-Alkylamino, Di(C1-C4-alkyl)amino, C1- C4-Alkylthio;
C2-C6-Alkenyl, gegebenenfalls substituiert mit Fluor, Chlor, Amino, Mercapto, Hydroxy, C1-C4-Acyloxy, Benzoyloxy, Benzyloxy, Phenoxy, C1-C4-Alkoxy, C1-C4-Alkylamino, Di(C1-C4-alkyl)amino, C1-C4-Alkylthio;
C3-C6-Alkinyl, gegebenenfalls substituiert mit Fluor, Chlor, Amino, Mercapto, Hydroxy, C1-C4-Acyloxy, Benzoyloxy, Benzyloxy, Phenoxy, C1-C4-Alkoxy, C1-C4-Alkylamino, Di(C1-C4-alkyl)amino, C1-C4-Alkylthio;
C3-C6-Cycloalkyl, gegebenenfalls substituiert mit Fluor, Chlor, C1-C4-Acyloxy, Benzoyloxy, Phenoxy, C1-C4-Alkoxy, C1-C4- Alkylamino, Di(C1-C4-alkyl)amino, C1-C4-Alkylthio,
C5-C6-Cycloalkenyl,
(C3-C6-Cycloalkyl)-(C1-C2-alkyl),
(C5-C6-Cycloalkenyl)-(C1-C2-alkyl),
C1-C6-Alkylcarbonyl, gegebenenfalls substituiert mit Fluor, Chlor, Amino, Mercapto, Hydroxy, C1-C4-Acyloxy, Benzoyloxy, Benzyloxy, Phenoxy, C1-C4-Alkoxy, C1-C4-Alkylamino, Di(C1- C4-alkyl)amino, C1-C4-Alkylthio;
C2-C4-Alkenylcarbonyl,
(C3-C6-Cycloalkyl)carbonyl,
(C5-C6-Cycloalkenyl)carbonyl,
(C3-C6-Cycloalkyl)-(C1-C2-alkyl)carbonyl,
(C5-C6-Cycloalkenyl)-(C1-C2-alkyl)carbonyl,
C1-C6-Alkyloxycarbonyl, gegebenenfalls substituiert durch Fluor, Chlor,
C1-C4-Alkoxy, C1-C4-Alkylamino, Di(C1-C4-alkyl)amino, C1- C4-Alkylthio;
C2 - C6-Alkenyloxycarbonyl,
C2-C6-Alkinyloxycarbonyl,
C1-C6-Alkylthiocarbonyl,
C2-C6-Alkenylthiocarbonyl,
C1-C6-Alkylamino- und Di(C1-C6-alkyl)aminocarbonyl,
C2-C6-Alkenylamino- und Di(C2-C4-alkenyl)aminocarbonyl,
C1-C6-Alkylsulfonyl,
C2-C6-Alkenylsulfonyl,
oder mit bis zu zwei voneinander unabhängigen Resten R5 substituiertes Aryl, Arylcarbonyl, Aryl(thiocarbonyl), (Arylthio)carbonyl, (Arylthio)thiocarbonyl, Aryloxycarbonyl, (Arylamino)thiocarbonyl, Arylsulfonyl, Arylalkyl, Arylalkenyl, Arylalkinyl, Arylalkylcarbonyl, Arylalkenylcarbonyl, Arylalkoxycarbonyl, wobei der Alkylrest jeweils 1 bis 3 C-Atome enthalten kann und R5 wie oben definiert ist
oder mit bis zu zwei voneinander unabhängigen Resten R5 substituiertes Heteroaryl, Heteroarylalkyl, Heteroarylalkenyl, Heteroarylalkylcarbonyl oder Heteroarylalkenylcarbonyl, wobei der Alkylrest jeweils 1 bis 2 C-Atome enthalten kann,
und
R3 und R4 gleich oder verschieden, unabhängig voneinander
C1-C6-Alkyl, gegebenenfalls substituiert mit Fluor, Chlor, Hydroxy, Amino, Mercapto, C1-C4-Acyloxy, Benzoyloxy, Benzyloxy, Phenoxy, C1-C4-Alkoxy, C1-C4-Alkylamino, Di(C1-C4-alkyl)amino, C1-C4-Alkylthio, C1-C4-Alkylsulfonyl, C1-C4-Alkylsulfinyl, Carboxy;
C2-C6-Alkenyl, gegebenenfalls substituiert mit Fluor oder Chlor;
C3-C6-Cycloalkyl,
C5-C6-Cycloalkenyl, gegebenenfalls substituiert mit Fluor oder Chlor;
mit bis zu drei voneinander unabhängigen Resten R5 substituiertes Aryl, Arylalkyl, Heteroaryl oder Heteroarylalkyl bedeuten, wobei der Alkylrest jeweils 1 bis 2 C-Atome enthalten kann und R5 wie oben definiert ist,
sein,
einer der Reste R3 oder R4 kann Wasserstoff sein
R3 und R4 können zusammen auch
einen über eine Doppelbindung verknüpften Rest =C-Z1Z2 bedeuten, wobei Z1 und Z2 die oben für R3 und R4 gegebene Bedeutung haben.

4. Verbindungen gemäß einem oder mehreren der Ansprüche 1-3 zur Anwendung als Arzneimittel.

5. Verwendung von Verbindungen gemäß den Ansprüchen 1 - 3 zur Herstellung von Arzneimitteln zur Prophylaxe und/oder Behandlung von Krankheiten, die durch Viren verursacht werden.

6. Verfahren zur Herstellung von Verbindungen der Formeln I, Ia, Ib und Ic wie oben unter 1) - 3) erläutert, dadurch gekennzeichnet, daß
A) zur Herstellung von Verbindungen der Formeln I, Ib mit X gleich Sauerstoff und Ia, Ic mit X wie unter 1) - 3) definiert - mit der Ausnahme von N-R2 gleich N-H - Y gleich R6, O-R6, S-R6, N-R6R7, N=C-R6R7 oder C-R6R7R8 und den Resten R1, R2, R3, R4, R5, R6, R7 und R8 wie unter 1) - 3) definiert
eine Verbindung der Formel II, IIa, IIb bzw. IIc, 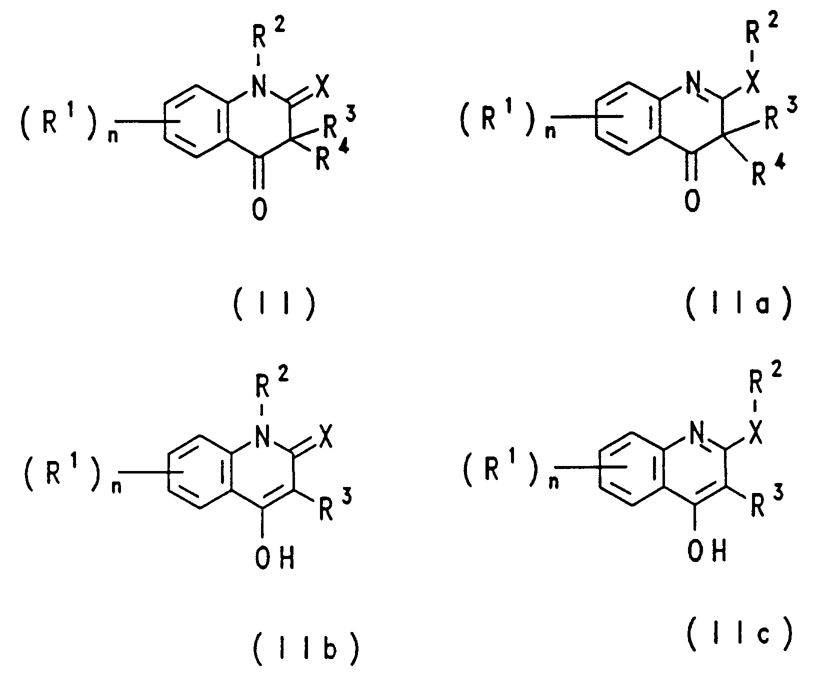 wobei für R1, R2, R3, R4 und R5 die unter 1) - 3) genannten Definitionen gelten,
mit einer Verbindung der Formel III,
Y-NH₂ (III)
wobei Y R6, O-R6, S-R6, N-R6R7, N =C-R6R7 oder C-R6R7R8 bedeuten kann und für R6, R7 und R8 die unter 1) - 3) genanten Definitionen gelten, umgesetzt wird
oder daß
B) Verbindungen der Formeln I, Ia, Ib und Ic mit X, Y und den Resten R1, R2, R3, R4, R5, R6, R7 und R8 wie unter 1) - 3) definiert, hergestellt werden durch Reaktion einer Verbindung der Formel I, Ia, Ib bzw. Ic, wobei für X und die Reste R1, R2, R3, R4, R5 und R6 die unter 1) - 3) genannten Definitionen gelten und Y gleich H, OH, SH, NH₂ oder NHR6 ist, mit einem Reagenz der Formel IV,
R9-Z (IV)
wobei R9 die oben unter 1) - 3) genannten Bedeutungen für R2, R6, R7 und R8 mit Ausnahme von Wasserstoff hat und Z eine Abgangsgruppe ist
oder daß
C) Verbindungen der Formeln I und Ib, mit X gleich Schwefel und R1, R2, R3, R4, R5, R6, R7 und R8 wie unter 1) - 3) definiert hergestellt werden durch Reaktion einer Verbindung der Formel I bzw Ib, wobei X gleich Sauerstoff ist und für R1, R2, R3, R4, R5, R6, R7 und R8 die unter 1) - 3) genannten Definitionen gelten, mit einem Schwefelungsreagenz
oder daß
D) zur Herstellung von Verbindungen der Formeln I, Ia, Ib und Ic, mit X und R1, R2, R3, R4 und R5 wie unter 1) - 3) definiert und Y gleich O-R6, S-R6 oder N-R6R7 Verbindungen der Formeln I, Ia, Ib bzw. Ic mit X und R1, R2, R3, R4 und R5 wie unter 1) - 3) definiert und Y gleich OH, SH, NH₂ oder NHR6
mit einer Verbindung der Formel V,
R9-OH (V)
wobei R9
Alkyl, gegebenenfalls substituiert mit Fluor, Chlor, Brom, Jod, Cyano, Amino, Mercapto, Hydroxy, Acyloxy, Benzoyloxy, Benzyloxy, Phenoxy, Alkoxy, Alkylamino, Dialkylamino, Alkylthio, Alkylsulfonyl, Phenylsulfonyl, Oxo, Thioxo, Carboxy, Carbamoyl;
Alkenyl, gegebenenfalls substituiert mit Fluor, Chlor, Brom, Jod, Cyano, Amino, Mercapto, Hydroxy, Acyloxy, Benzoyloxy, Benzyloxy, Phenoxy, Alkoxy, Alkylamino, Dialkylamino, Alkylthio, Alkylsulfonyl, Phenylsulfonyl, Oxo, Thioxo, Carboxy, Carbamoyl;
Alkinyl, gegebenenfalls substituiert mit Fluor, Chlor, Brom, Jod, Cyano, Amino, Mercapto, Hydroxy, Acyloxy, Benzoyloxy, Benzyloxy, Phenoxy, Alkoxy, Alkylamino, Dialkylamino, Alkylthio, Alkylsulfonyl, Phenylsulfonyl, Oxo, Thioxo, Carboxy, Carbamoyl;
Cycloalkyl, gegebenenfalls substituiert mit Fluor, Chlor, Brom, Jod, Cyano, Amino, Mercapto, Hydroxy, Acyloxy, Benzoyloxy, Benzyloxy, Phenoxy, Alkoxy, Alkylamino, Dialkylamino, Alkylthio, Alkylsulfonyl, Phenylsulfonyl, Oxo, Thioxo, Carboxy, Carbamoyl;
Cycloalkenyl, gegebenenfalls substituiert mit Fluor, Chlor, Brom, Jod, Cyano, Amino, Mercapto, Hydroxy, Acyloxy, Benzoyloxy, Benzyloxy, Phenoxy, Alkoxy, Alkylamino, Dialkylamino, Alkylthio, Alkylsulfonyl, Phenylsulfonyl, Oxo, Thioxo, Carboxy, Carbamoyl;
(Cycloalkyl)-(alkyl), gegebenenfalls substituiert mit Fluor,
Chlor, Brom, Jod, Cyano, Amino, Mercapto, Hydroxy, Acyloxy, Benzoyloxy, Benzyloxy, Phenoxy, Alkoxy, Alkylamino, Dialkylamino, Alkylthio, Alkylsulfonyl, Phenylsulfonyl, Oxo, Thioxo, Carboxy, Carbamoyl;
(Cycloalkenyl)-(alkyl), gegebenenfalls substituiert mit Fluor, Chlor, Brom, Jod, Cyano, Amino, Mercapto, Hydroxy, Acyloxy, Benzoyloxy, Benzyloxy, Phenoxy, Alkoxy, Alkylamino, Dialkylamino, Alkylthio, Alkylsulfonyl, Phenylsulfonyl, Oxo, Thioxo, Carboxy, Carbamoyl;
Alkylcarbonyl, gegebenenfalls substituiert mit Fluor, Chlor, Brom, Jod, Cyano, Amino, Mercapto, Hydroxy, Acyloxy, Benzoyloxy, Benzyloxy, Phenoxy, Alkoxy, Alkylamino, Dialkylamino, Alkylthio, Alkylsulfonyl, Phenylsulfonyl, Oxo, Thioxo, Carboxy, Carbamoyl;
Alkenylcarbonyl, gegebenenfalls substituiert durch Fluor, Chlor oder Hydroxy, Alkoxy, Oxo, Phenyl;
(Cycloalkyl)carbonyl, gegebenenfalls substituiert durch Fluor, Chlor oder Hydroxy, Alkoxy, Oxo, Phenyl;
(Cycloalkenyl)carbonyl, gegebenenfalls substituiert durch Fluor, Chlor oder Hydroxy, Alkoxy, Oxo, Phenyl;
(Cycloalkyl)-(alkyl)carbonyl, gegebenenfalls substituiert durch Fluor, Chlor oder Hydroxy, Alkoxy, Oxo, Phenyl;
(Cycloalkenyl)-(alkyl)carbonyl, gegebenenfalls substituiert durch Fluor, Chlor oder Hydroxy, Alkoxy, Oxo, Phenyl;
oder mit bis zu fünf voneinander unabhängigen Resten R5 substituiertes Aryl, Arylcarbonyl, Arylalkyl, Arylalkenyl, Arylalkinyl, Arylalkylcarbonyl, Arylalkenylcarbonyl, wobei R5 wie oben definiert ist
oder mit bis zu drei voneinander unabhängigen Resten R5 substituiertes Heteroaryl, Heteroarylalkyl, Heteroarylalkenyl, Heteroarylalkylcarbonyl oder Heteroarylalkenylcarbonyl,
sein kann
in Gegenwart eines wasserentziehenden Mittels umgesetzt werden
oder daß
E) Verbindungen der Formeln I und Ib, mit X gleich Sauerstoff und Y, R1, R2, R3, R4 und R5 wie unter 1) - 3), definiert sind, durch Cyclisierung einer Verbindung der Formel VI, 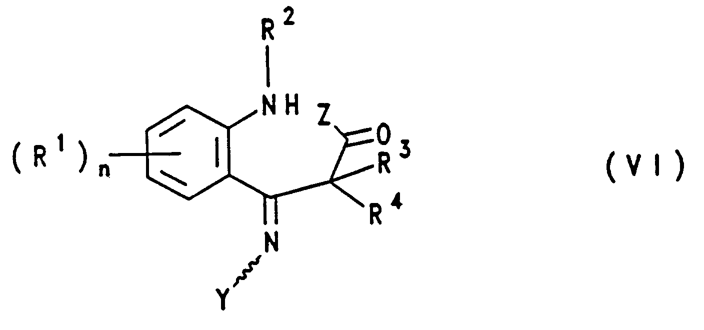 mit Y, R1, R2, R3, R4 und R5 wie unter 1) - 3) definiert und Z eine Abgangsgruppe ist, hergestellt werden.

7. Arzneimittel, enthaltend eine wirksame Menge einer Verbindung gemäß den Ansprüchen 1-3.

8. Verfahren zur Herstellung von Arzneimitteln, dadurch gekennzeichnet, daß man eine Verbindung gemäß den Ansprüchen 1-3 in eine geeignete Darreichungsform bringt.

## Claims

1. A compound of the formula I 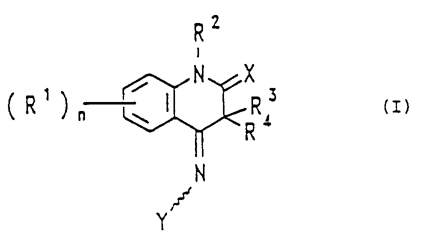 or a tautomeric form thereof of the formula Ia, Ib or Ic 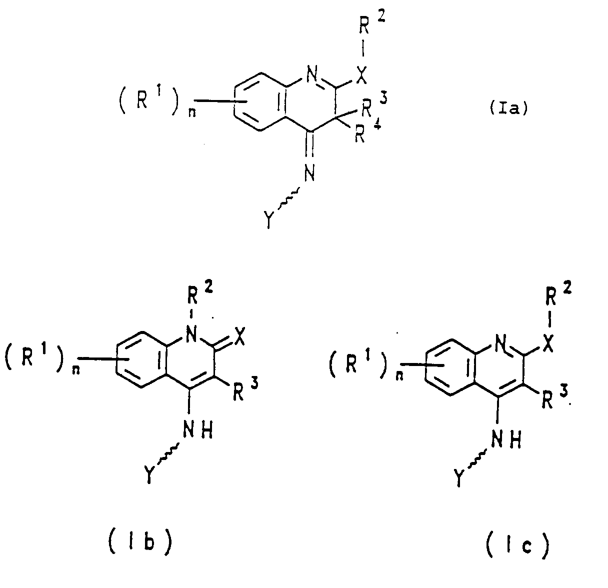 in which:
n is
zero,
one,
two,
three
or four,
the individual substituents R1 independently of one another are
fluorine, chlorine, bromine, iodine, trifluoromethyl, trifluoromethoxy, hydroxyl, C1-C8-alkyl, C5-C8-cycloalkyl, C1-C6-alkoxy, (C1-C6-alkoxy)-(C1-C4-alkoxy), C1-C6-alkylthio, C1-C6-alkylsulfinyl, C1-C6-alkylsulfonyl, nitro, amino, azido, C1-C6-alkylamino, di(C1-C6-alkyl)-amino, piperidino, morpholino, 1-pyrrolidinyl, C1-C6-acyl, C1-C6-acyloxy, C1-C6-acylamino, cyano, carbamoyl, carboxyl, (C1-C6-alkyl)-oxycarbonyl, hydroxysulfonyl or sulfamoyl
or
a phenyl, phenoxy, phenoxycarbonyl, phenylthio, phenylsulfinyl, phenylsulfonyl, phenoxysulfonyl, phenylsulfonyloxy, phenylsulfonylamino, benzoyl, heteroaroyl, heteroaryl or heteroarylmethyl radical, which is optionally substituted by up to five radicals R5 which are independent of one another,
in which R5 can be
fluorine, chlorine, bromine, iodine, cyano, trifluoromethyl, trifluoromethoxy, nitro, amino, azido, C1-C6-alkyl, C3-C8-cycloalkyl, C1-C6-alkoxy, C1-C6-alkylthio, C1-C6-alkylsulfinyl, C1-C6-alkylsulfonyl, C1-C6-alkylamino, di(C1-C6-alkyl)amino, (C1-C6-alkyl)-oxycarbonyl, phenyl, phenoxy or heteroaryl,
X is oxygen, sulfur, selenium or substituted nitrogen N-R2 or N-O-R2, in which R2 can have the meanings given below,
Y is R6, O-R6, S-R6, N-R6R7, N=CR6R7 or C-R6R7R8, in which R6, R7 and R8 can have the meanings given below, R2, R6, R7 and R8 can be identical or different and, independently of one another, can be
hydrogen,
C1-C8-alkyl, which is optionally substituted by fluorine, chlorine, bromine, iodine, cyano, amino, mercapto, hydroxyl, C1-C6-acyloxy, benzoyloxy, benzyloxy, phenoxy, C1-C6-alkoxy, C1-C6-alkylamino, di(C1-C6-alkyl)amino, C1-C6-alkylthio, C1-C6-alkylsulfonyl, phenylsulfonyl, oxo, thioxo, carboxyl or carbamoyl;
C2-C8-alkenyl, which is optionally substituted by fluorine, chlorine, bromine, iodine, cyano, amino, mercapto, hydroxyl, C1-C6-acyloxy, benzoyloxy, benzyloxy, phenoxy, C1-C6-alkoxy, C1-C6-alkylamino, di(C1-C6-alkyl)-amino, C1-C6-alkylthio, C1-C6-alkylsulfonyl, phenylsulfonyl, oxo, thioxo, carboxyl or carbamoyl;
C3-C8-alkynyl, which is optionally substituted by fluorine, chlorine, bromine, iodine, cyano, amino, mercapto, hydroxyl, C1-C6-acyloxy, benzoyloxy, benzyloxy, phenoxy, C1-C6-alkoxy, C1-C6-alkylamino, di(C1-C6-alkyl)-amino, C1-C6-alkylthio, C1-C6-alkylsulfonyl, phenylsulfonyl, oxo, thioxo, carboxyl or carbamoyl;
C3-C8-cycloalkyl, which is optionally substituted by fluorine, chlorine, bromine, iodine, cyano, amino, mercapto, hydroxyl, C1-C6-acyloxy, benzoyloxy, benzyloxy, phenoxy, C1-C6-alkoxy, C1-C6-alkylamino, di(C1-C6-alkyl)-amino, C1-C6-alkylthio, C1-C6-alkylsulfonyl, phenylsulfonyl, oxo, thioxo, carboxyl or carbamoyl;
C5-C8-cycloalkenyl, which is optionally substituted by fluorine, chlorine, bromine, iodine, cyano, amino, mercapto, hydroxyl, C1-C6-acyloxy, benzoyloxy, benzyloxy, phenoxy, C1-C6-alkoxy, C1-C6-alkylamino, di(C1-C6-alkyl)-amino, C1-C6-alkylthio, C1-C6-alkylsulfonyl, phenylsulfonyl, oxo, thioxo, carboxyl or carbamoyl;
(C3-C8-cycloalkyl)-(C1-C4-alkyl), which is optionally substituted by fluorine, chlorine, bromine, iodine, cyano, amino, mercapto, hydroxyl, C1-C6-acyloxy, benzoyloxy, benzyloxy, phenoxy, C1-C6-alkoxy, C1-C6-alkylamino, di(C1-C6-alkyl)amino, C1-C6-alkylthio, C1-C6-alkylsulfonyl, phenylsulfonyl, oxo, thioxo, carboxyl or carbamoyl;
(C5-C8-cycloalkenyl)-(C1-C4-alkyl), which is optionally substituted by fluorine, chlorine, bromine, iodine, cyano, amino, mercapto, hydroxyl, C1-C6-acyloxy, benzoyloxy, benzyloxy, phenoxy, C1-C6-alkoxy, C1-C6-alkylamino, di(C1-C6-alkyl)amino, C1-C6-alkylthio, C1-C6-alkylsulfonyl, phenylsulfonyl, oxo, thioxo, carboxyl or carbamoyl;
C1-C6-alkylcarbonyl, which is optionally substituted by fluorine, chlorine, bromine, iodine, cyano, amino, mercapto, hydroxyl, C1-C6-acyloxy, benzoyloxy, benzyloxy, phenoxy, C1-C6-alkoxy, C1-C6-alkylamino, di(C1-C6-alkyl)-amino, C1-C6-alkylthio, C1-C6-alkylsulfonyl, phenylsulfonyl, oxo, thioxo, carboxyl or carbamoyl;
C2-C8-alkenylcarbonyl, which is optionally substituted by fluorine, chlorine or hydroxyl, C1-C4-alkoxy, oxo or phenyl;
(C3-C8-cycloalkyl)carbonyl, which is optionally substituted by fluorine, chlorine or hydroxyl, C1-C4-alkoxy, oxo or phenyl;
(C5-C8-cycloalkenyl) carbonyl, which is optionally substituted by fluorine, chlorine or hydroxyl, C1-C4-alkoxy, oxo or phenyl;
(C3-C8-cycloalkyl)-(C1-C3-alkyl)carbonyl, which is optionally substituted by fluorine, chlorine or hydroxyl, C1-C4-alkoxy, oxo or phenyl;
(C5-C6-cycloalkenyl)-(C1-C3-alkyl)carbonyl, which is optionally substituted by fluorine, chlorine or hydroxyl, C1-C4-alkoxy, oxo or phenyl;
C1-C8-alkyloxycarbonyl, which is optionally substituted by fluorine, chlorine, bromine, hydroxyl, C1-C4-alkoxy, C1-C4-alkylamino, di(C1-C4-alkyl)amino, C1-C4-alkylthio;
C2-C8-alkenyloxycarbonyl, which is optionally substituted by fluorine, chlorine, hydroxyl, C1-C4-alkoxy, oxo or phenyl;
C2-C8-alkynyloxycarbonyl, which is optionally substituted by fluorine, chlorine, hydroxyl, C1-C4-alkoxy, oxo or phenyl;
C1-C8-alkylthiocarbonyl, which is optionally substituted by fluorine, chlorine, hydroxyl, C1-C4-alkoxy, oxo or phenyl;
C2-C8-alkenylthiocarbonyl, which is optionally substituted by fluorine, chlorine, hydroxyl, C1-C4-alkoxy, oxo or phenyl;
C1-C8-alkylamino- or di(C1-C8-alkyl)aminocarbonyl, which is optionally substituted by fluorine, chlorine, hydroxyl, C1-C4-alkoxy, oxo or phenyl;
C2-C8-alkenylamino- or di(C2-C6-alkenyl)aminocarbonyl, which is optionally substituted by fluorine, chlorine, hydroxyl, C1-C4-alkoxy, oxo or phenyl;
C1-C6-alkylsulfonyl, which is optionally substituted by fluorine, chlorine, hydroxyl, C1-C4-alkoxy, C1-C4-alkylthio, oxo or phenyl;
C2-C6-alkenylsulfonyl, which is optionally substituted by fluorine, chlorine, hydroxyl, C1-C4-alkoxy, oxo or phenyl;
or aryl, arylcarbonyl, aryl(thiocarbonyl), (arylthio)-carbonyl, (arylthio)thiocarbonyl, aryloxycarbonyl, (arylamino)thiocarbonyl, arylsulfonyl, arylalkyl, arylalkenyl, arylalkynyl, arylalkylcarbonyl, arylalkenylcarbonyl or arylalkoxycarbonyl, which are substituted by up to five radicals R5, which are independent of one another, and in which the alkyl radical in each case can contain 1 to 5 carbon atoms and R5 is as defined above,
or heteroaryl, heteroarylalkyl, heteroarylalkenyl, heteroarylalkylcarbonyl or heteroarylalkenylcarbonyl, which are substituted by up to three radicals R5, which are independent of one another, and in which the alkyl radical in each case can contain 1 to 3 carbon atoms,
and
R3 and R4 are identical or different and independently of one another are
hydrogen,
C1-C8-alkyl, which is optionally substituted by fluorine, chlorine, hydroxyl, amino, mercapto, C1-C4-acyloxy, benzoyloxy, benzyloxy, phenoxy, C1-C4-alkoxy, C1-C4-alkylamino, di(C1-C4-alkyl)amino, C1-C4-alkylthio, C1-C4-alkylsulfonyl, C1-C4-alkylsulfinyl, carboxyl or carbamoyl;
C2-C8-alkenyl, which is optionally substituted by fluorine or chlorine, hydroxyl, amino, mercapto, C1-C4-acyloxy, benzoyloxy, benzyloxy, phenoxy, C1-C4-alkoxy, C1-C4-alkylamino, di(C1-C4-alkyl)amino, C1-C4-alkylthio, C1-C4-alkylsulfonyl, C1-C4-alkylsulfinyl, carboxyl or carbamoyl;
C3-C8-cycloalkyl, which is optionally substituted by fluorine, chlorine, hydroxyl, amino, mercapto, C1-C4-acyloxy, benzoyloxy, benzyloxy, phenoxy, C1-C4-alkoxy, C1-C4-alkylamino, di(C1-C4-alkyl)amino, C1-C4-alkylthio, C1-C4-alkylsulfonyl, C1-C4-alkylsulfinyl, carboxyl or carbamoyl;
C3-C8-cycloalkenyl, which is optionally substituted by fluorine or chlorine, hydroxyl, amino, mercapto, C1-C4-acyloxy, benzoyloxy, benzyloxy, phenoxy, C1-C4-alkoxy, C1-C4-alkylamino, di(C1-C4-alkyl)amino, C1-C4-alkylthio, C1-C4-alkylsulfonyl, C1-C4-alkylsulfinyl, carboxyl or carbamoyl;
or aryl, arylalkyl, heteroaryl or heteroarylalkyl, which are substituted by up to five radicals R5, which are independent of one another, and in which the alkyl radical in each case can contain 1 to 3 carbon atoms and R5 is as defined above;
R3 and R4 together can also be
a radical =C-Z1Z2 linked via a double bond, in which Z1 and Z2 have the meaning given above for R3 and R4,
R3 and R4 furthermore can also be
part of a saturated or unsaturated carbo- or heterocyclic ring, which can optionally be substituted by fluorine, chlorine, hydroxyl, amino, C₁-C₈-alkyl, C₂-C₈-alkenyl, C₂-C₈-alkynyl, C₁-C₈-acyloxy, benzoyloxy, C₁-C₈-alkoxy, C₁-C₈-alkylthio, oxo, thioxo, carboxyl, carbamoyl or phenyl,
or an optical isomer thereof or a diastereomer in the pure form or in the form of a mixture or an addition salt thereof,
and in the above definitions the heterocyclic rings and heteroaryl groups contain 1-15 carbon atoms and 1-6 heteroatoms selected from the group O, S and N and if there is a nitrogen-containing ring which is saturated at this point then there is N-Z, in which Z is H or R2 as defined above,
except for compounds in which simultaneously R3 and/or R4 are hydrogen and Y is R6 or CR6R7R8 and for those in which simultaneously n is 0 or 1, R1 is CH₃ or OCH₃, R2 is CH₃, R3 is CH₂CH₂OCH₃, X is O and Y is H and for those in which simultaneously n is 1, R1 is Cl, R2 is H or CH₃, R3 is OH, R4 is phenyl, X is O and Y is H or OH.

2. A compound of the formula I, Ia, Ib or Ic as claimed in claim 1,
in which:
n is
zero,
one
or two,
the individual substituents R1 independently of one another are
fluorine, chlorine, bromine, iodine, trifluoromethyl, trifluoromethoxy, hydroxyl, C1-C6-alkyl, C5-C6-cycloalkyl, C1-C4-alkoxy, (C1-C4-alkoxy)-(C1-C2-alkoxy), C1-C4-alkylthio, C1-C4-alkylsulfinyl, C1-C4-alkylsulfonyl, nitro, amino, C1-C4-alkylamino, di(C1-C4-alkyl)amino, C1-C6-acyl, C1-C4-acyloxy, C1-C4-acylamino, cyano, carbamoyl, carboxyl, (C1-C4-alkyl)-oxycarbonyl
or
a phenyl, phenoxy, phenoxycarbonyl, phenylthio, phenylsulfinyl, phenylsulfonyl, phenoxysulfonyl, phenylsulfonyloxy, phenylsulfonylamino, benzoyl, heteroaroyl, heteroaryl or heteroarylmethyl radical, which is optionally substituted by up to three radicals R5 which are independent of one another,
in which R5 can be
fluorine, chlorine, trifluoromethyl, trifluoromethoxy, nitro, amino, C1-C4-alkyl, C3-C6-cycloalkyl, C1-C4-alkoxy, C1-C4-alkylthio, C1-C4-alkylsulfinyl, C1-C4-alkylsulfonyl, C1-C4-alkylamino, di(C1-C4-alkyl)amino, phenyl, phenoxy or heteroaryl,
X is oxygen, sulfur or substituted nitrogen N-R2 or N-O-R2, in which R2 can have the meanings given below,
Y is R6, O-R6, S-R6, N-R6R7, N=CR6R7 or C-R6R7R8, in which R6, R7 and R8 can have the meanings given below,
R2, R6, R7 and R8 can be identical or different and, independently of one another, can be
hydrogen,
C1-C6-alkyl, which is optionally substituted by fluorine, chlorine, bromine, iodine, cyano, amino, mercapto, hydroxyl, C1-C4-acyloxy, benzoyloxy, benzyloxy, phenoxy, C1-C4-alkoxy, C1-C4-alkylamino, di(C1-C4-alkyl)amino, C1-C4-alkylthio, C1-C4-alkylsulfonyl, phenylsulfonyl, oxo, thioxo, carboxyl or carbamoyl;
C2-C6-alkenyl, which is optionally substituted by fluorine, chlorine, cyano, amino, mercapto, hydroxyl, C1-C4-acyloxy, benzoyloxy, benzyloxy, phenoxy, C1-C4-alkoxy, C1-C4-alkylamino, di(C1-C4-alkyl)amino, C1-C4-alkylthio, C1-C4-alkylsulfonyl, phenylsulfonyl, carboxyl or carbamoyl;
C3-C6-alkynyl, which is optionally substituted by fluorine, chlorine, cyano, amino, mercapto, hydroxyl, C1-C4-acyloxy, benzoyloxy, benzyloxy, phenoxy, C1-C4-alkoxy, C1-C4-alkylamino, di(C1-C4-alkyl)amino, C1-C4-alkylthio, C1-C4-alkylsulfonyl, phenylsulfonyl, carboxyl or carbamoyl;
C3-C6-cycloalkyl, which is optionally substituted by fluorine, chlorine, cyano, amino, mercapto, hydroxyl, C1-C4-acyloxy, benzoyloxy, benzyloxy, phenoxy, C1-C4-alkoxy, C1-C4-alkylamino, di(C1-C4-alkyl)amino, C1-C4-alkylthio, C1-C4-alkylsulfonyl, phenylsulfonyl, carboxyl or carbamoyl;
C5-C6-cycloalkenyl, which is optionally substituted by fluorine, chlorine, cyano, amino, mercapto, hydroxyl, C1-C4-acyloxy, benzoyloxy, benzyloxy, phenoxy, C1-C4-alkoxy, C1-C4-alkylamino, di(C1-C4-alkyl)amino, C1-C4-alkylthio, C1-C4-alkylsulfonyl, phenylsulfonyl, carboxyl or carbamoyl;
(C3-C6-cycloalkyl)-(C1-C2-alkyl), which is optionally substituted by fluorine, chlorine, cyano, amino, mercapto, hydroxyl, C1-C4-acyloxy, benzoyloxy, benzyloxy, phenoxy, C1-C4-alkoxy, C1-C4-alkylamino, di(C1-C4-alkyl)-amino, C1-C4-alkylthio, C1-C4-alkylsulfonyl, phenylsulfonyl, carboxyl or carbamoyl;
(C5-C6-cycloalkenyl)-(C1-C2-alkyl), which is optionally substituted by fluorine, chlorine, cyano, amino, mercapto, hydroxyl, C1-C4-acyloxy, benzoyloxy, benzyloxy, phenoxy, C1-C4-alkoxy, C1-C4-alkylamino, di(C1-C4-alkyl)-amino, C1-C4-alkylthio, C1-C4-alkylsulfonyl, phenylsulfonyl, carboxyl or carbamoyl;
C1-C6-alkylcarbonyl, which is optionally substituted by fluorine, chlorine, cyano, amino, mercapto, hydroxyl, C1-C4-acyloxy, benzoyloxy, benzyloxy, phenoxy, C1-C4-alkoxy, C1-C4-alkylamino, di(C1-C4-alkyl)amino, C1-C4-alkylthio, C1-C4-alkylsulfonyl, phenylsulfonyl, carboxyl or carbamoyl;
C2-C6-alkenylcarbonyl, which is optionally substituted by fluorine, chlorine, hydroxyl, C1-C4-alkoxy, oxo or phenyl;
(C3-C6-cycloalkyl)carbonyl, which is optionally substituted by fluorine, chlorine, hydroxyl, C1-C4-alkoxy, oxo or phenyl;
(C5-C6-cycloalkenyl)carbonyl, which is optionally substituted by fluorine, chlorine, hydroxyl, C1-C4-alkoxy, oxo or phenyl;
(C3-C6-cycloalkyl)-(C1-C2-alkyl)carbonyl, which is optionally substituted by fluorine, chlorine, hydroxyl, C1-C4-alkoxy, oxo or phenyl;
(C5-C6-cycloalkenyl)-(C1-C2-alkyl)carbonyl, which is optionally substituted by fluorine, chlorine, hydroxyl, C1-C4-alkoxy, oxo or phenyl;
C1-C6-alkyloxycarbonyl, which is optionally substituted by fluorine, chlorine, C1-C4-alkoxy, C1-C4-alkylamino, di(C1-C4-alkyl)amino or C1-C4-alkylthio;
C2-C6-alkenyloxycarbonyl, which is optionally substituted by fluorine, chlorine, C1-C4-alkoxy or phenyl;
C2-C6-alkynyloxycarbonyl, which is optionally substituted by fluorine, chlorine, C1-C4-alkoxy or phenyl;
C1-C6-alkylthiocarbonyl, which is optionally substituted by fluorine, chlorine, C1-C4-alkoxy or phenyl;
C2-C6-alkenylthiocarbonyl, which is optionally substituted by fluorine, chlorine, C1-C4-alkoxy or phenyl;
C1-C6-alkylamino- or di(C1-C6-alkyl)aminocarbonyl, which is optionally substituted by fluorine, chlorine, C1-C4-alkoxy or phenyl;
C2-C6-alkenylamino- or di(C2-C6-alkenyl)aminocarbonyl, which is optionally substituted by fluorine, chlorine, C1-C4-alkoxy or phenyl;
C1-C6-alkylsulfonyl, which is optionally substituted by fluorine, chlorine, C1-C4-alkoxy or phenyl;
C2-C6-alkenylsulfonyl,
or aryl, arylcarbonyl, aryl(thiocarbonyl), (arylthio)carbonyl, (arylthio)thiocarbonyl, aryloxycarbonyl, (arylamino)thiocarbonyl, arylsulfonyl, arylalkyl, arylalkenyl, arylalkynyl, arylalkylcarbonyl, arylalkenylcarbonyl or arylalkoxycarbonyl, which are substituted by up to three radicals R5, which are independent of one another, and in which the alkyl radical in each case can contain 1 to 4 carbon atoms and R5 is as defined above,
or heteroaryl, heteroarylalkyl, heteroarylalkenyl, heteroarylalkylcarbonyl or heteroarylalkenylcarbonyl, which are substituted by up to three radicals R5, which are independent of one another, and in which the alkyl radical in each case can contain 1 to 3 carbon atoms,
and
R3 and R4 are identical or different and independently of one another are
C1-C6-alkyl, which is optionally substituted by fluorine, chlorine, hydroxyl, amino, mercapto, C1-C4-acyloxy, benzoyloxy, benzyloxy, phenoxy, C1-C4-alkoxy, C1-C4-alkylamino, di(C1-C4-alkyl)amino, C1-C4-alkylthio, C1-C4-alkylsulfonyl, C1-C4-alkylsulfinyl, carboxyl or carbamoyl;
C2-C6-alkenyl, which is optionally substituted by fluorine or chlorine, phenoxy, C1-C4-alkoxy, C1-C4-alkylthio, C1-C4-alkylsulfonyl or C1-C4-alkylsulfinyl;
C3-C6-cycloalkyl, which is optionally substituted by fluorine, chlorine, hydroxyl, amino, mercapto, C1-C4-acyloxy, benzoyloxy, benzyloxy, phenoxy, C1-C4-alkoxy, C1-C4-alkylamino, di(C1-C4-alkyl)amino, C1-C4-alkylthio, C1-C4-alkylsulfonyl or C1-C4-alkylsulfinyl;
C3-C6-cycloalkenyl, which is optionally substituted by fluorine or chlorine, phenoxy, C1-C4-alkoxy, C1-C4-alkylthio, C1-C4-alkylsulfonyl or C1-C4-alkylsulfinyl;
or aryl, arylalkyl, heteroaryl or heteroarylalkyl, which are substituted by up to three radicals R5, which are independent of one another, and in which the alkyl radical in each case can contain 1 to 3 carbon atoms and R5 is as defined above,
one of the radicals R3 or R4 can be hydrogen, and
R3 and R4 together can also be
a radical =C-Z1Z2 linked via a double bond, in which Z1 and Z2 have the meaning given above for R3 and R4.

3. A compound of the formula I, Ia, Ib or Ic as claimed in either of claims 1-2, in which:
n is
zero,
one
or two,
the individual substituents R1 independently of one another are
fluorine, chlorine, bromine, trifluoromethyl, trifluoromethoxy, hydroxyl, C1-C4-alkyl, C1-C4-alkoxy, (C1-C4-alkoxy)-(C1-C2-alkoxy), C1-C4-alkylthio, C1-C4-alkylsulfinyl, C1-C4-alkylsulfonyl, nitro, amino, C1-C4-alkylamino, di(C1-C4-alkyl)amino, C1-C4-acyl, C1-C4-acyloxy, C1-C4-acylamino, cyano, carbamoyl, carboxyl, (C1-C4-alkyl)-oxycarbonyl
or
a phenyl, phenoxy, phenoxycarbonyl, phenylthio, phenylsulfinyl, phenylsulfonyl, benzoyl, heteroaroyl, heteroaryl or heteroarylmethyl radical, which is optionally substituted by up to three radicals R5 which are independent of one another,
in which R5 can be
fluorine, chlorine, trifluoromethyl, trifluoromethoxy, nitro, amino, C1-C4-alkyl, C1-C4-alkoxy, C1-C4-alkylthio, C1-C4-alkylsulfinyl, C1-C4-alkylsulfonyl, C1-C4-alkylamino, di(C1-C4-alkyl)amino, phenyl or phenoxy,
X is oxygen, sulfur or substituted nitrogen N-R2 or N-O-R2, in which R2 can have the meanings given below,
Y is O-R6, S-R6, N-R6R7 or N=CR6R7, in which R6, R7 and R8 can have the meanings given below,
R2, R6, R7 and R8 can be identical or different and, independently of one another, can be
hydrogen,
C1-C6-alkyl, which is optionally substituted by fluorine, chlorine, amino, mercapto, hydroxyl, C1-C4-acyloxy, benzoyloxy, benzyloxy, phenoxy, C1-C4-alkoxy, C1-C4-alkylamino, di(C1-C4-alkyl)amino or C1-C4-alkylthio;
C2-C6-alkenyl, which is optionally substituted by fluorine, chlorine, amino, mercapto, hydroxyl, C1-C4-acyloxy, benzoyloxy, benzyloxy, phenoxy, C1-C4-alkoxy, C1-C4-alkylamino, di(C1-C4-alkyl)amino or C1-C4-alkylthio;
C3-C6-alkynyl, which is optionally substituted by fluorine, chlorine, amino, mercapto, hydroxyl, C1-C4-acyloxy, benzoyloxy, benzyloxy, phenoxy, C1-C4-alkoxy, C1-C4-alkylamino, di(C1-C4-alkyl)amino or C1-C4-alkylthio;
C3-C6-cycloalkyl, which is optionally substituted by fluorine, chlorine, C1-C4-acyloxy, benzoyloxy, phenoxy, C1-C4-alkoxy, C1-C4-alkylamino, di(C1-C4-alkyl)amino or C1-C4-alkylthio;
C5-C6-cycloalkenyl,
(C3-C6-cycloalkyl)-(C1-C2-alkyl),
(C5-C6-cycloalkenyl)-(C1-C2-alkyl),
C1-C6-alkylcarbonyl, which is optionally substituted by fluorine, chlorine, amino, mercapto, hydroxyl, C1-C4-acyloxy, benzoyloxy, benzyloxy, phenoxy, C1-C4-alkoxy, C1-C4-alkylamino, di(C1-C4-alkyl)amino or C1-C4-alkylthio;
C2-C4-alkenylcarbonyl,
(C3-C6-cycloalkyl)carbonyl,
(C5-C6-cycloalkenyl) carbonyl,
(C3-C6-cycloalkyl)-(C1-C2-alkyl)carbonyl,
(C5-C6-cycloalkenyl)-(C1-C2-alkyl)carbonyl,
C1-C6-alkyloxycarbonyl, which is optionally substituted by fluorine, chlorine, C1-C4-alkoxy, C1-C4-alkylamino, di(C1-C4-alkyl)amino or C1-C4-alkylthio;
C2-C6-alkenyloxycarbonyl,
C2-C6-alkynyloxycarbonyl,
C1-C6-alkylthiocarbonyl,
C2-C6-alkenylthiocarbonyl,
C1-C6-alkylamino- or di(C1-C6-alkyl)aminocarbonyl,
C2-C6-alkenylamino- or di(C2-C4-alkenyl)aminocarbonyl,
C1-C6-alkylsulfonyl,
C2-C6-alkenylsulfonyl,
or aryl, arylcarbonyl, aryl(thiocarbonyl), (arylthio)carbonyl, (arylthio)thiocarbonyl, aryloxycarbonyl, (arylamino)thiocarbonyl, arylsulfonyl, arylalkyl, arylalkenyl, arylalkynyl, arylalkylcarbonyl, arylalkenylcarbonyl or arylalkoxycarbonyl, which are substituted by up to two radicals R5, which are independent of one another, and in which the alkyl radical in each case can contain 1 to 3 carbon atoms and R5 is as defined above,
or heteroaryl, heteroarylalkyl, heteroarylalkenyl, heteroarylalkylcarbonyl or heteroarylalkenylcarbonyl, which are substituted by up to two radicals R5, which are independent of one another, and in which the alkyl radical in each case can contain 1 to 2 carbon atoms,
and
R3 and R4 are identical or different and independently of one another are
C1-C6-alkyl, which is optionally substituted by fluorine, chlorine, hydroxyl, amino, mercapto, C1-C4-acyloxy, benzoyloxy, benzyloxy, phenoxy, C1-C4-alkoxy, C1-C4-alkylamino, di(C1-C4-alkyl)amino, C1-C4-alkylthio, C1-C4-alkylsulfonyl, C1-C4-alkylsulfinyl or carboxyl;
C2-C6-alkenyl, which is optionally substituted by fluorine or chlorine;
C3-C6-cycloalkyl,
C5-C6-cycloalkenyl, which is optionally substituted by fluorine or chlorine;
or aryl, arylalkyl, heteroaryl or heteroarylalkyl which are substituted by up to three radicals R5, which are independent of one another, and in which the alkyl radical in each case can contain 1 to 2 carbon atoms and R5 is as defined above,
one of the radicals R3 or R4 can be hydrogen, and
R3 and R4 together can also be
a radical =C-Z1Z2 linked via a double bond, in which Z1 and Z2 have the meaning given above for R3 and R4.

4. A compound as claimed in one or more of claims 1-3 for use as a medicament.

5. The use of a compound as claimed in any of claims 1-3 for the preparation of a medicament for the prophylaxis and/or treatment of diseases caused by viruses.

6. A process for the preparation of a compound of the formula I, Ia, Ib or Ic as described above under 1) - 3), which comprises
A) for the preparation of a compound of the formula I or Ib, where X is oxygen, or Ia or Ic, where X is as defined under 1) - 3) - with the exception of N-R2 being N-H - Y is R6, O-R6, S-R6, N-R6R7, N=C-R6R7 or C-R6R7R8 and the radicals R1, R2, R3, R4, R5, R6, R7 and R8 are as defined under 1) - 3),
reacting a compound of the formula II, IIa, IIb or IIc 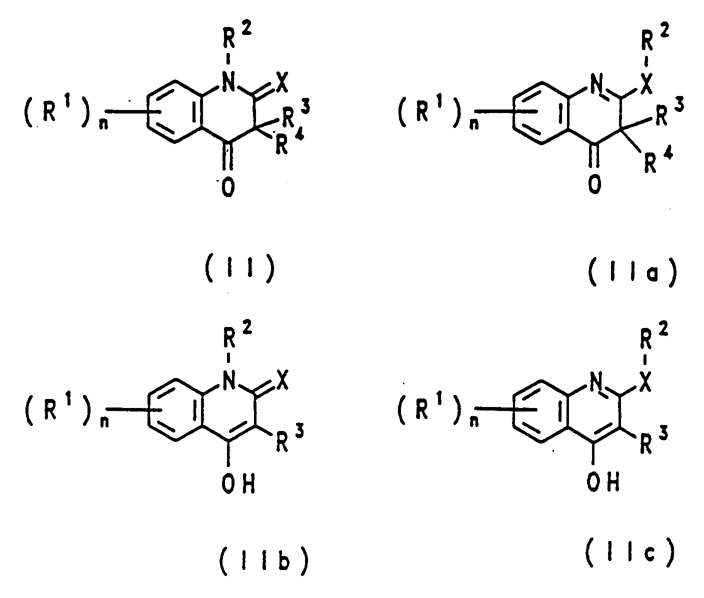 in which the definitions mentioned under 1) - 3) apply to R1, R2, R3, R4 and R5,
with a compound of the formula III
Y-NH₂ (III)
in which Y can be R6, O-R6, S-R6, N-R6R7, N=C-R6R7 or C-R6R7R8 and the definitions mentioned under 1) - 3) apply to R6, R7 and R8,
or
B) preparing a compound of the formula I, Ia, Ib or Ic, where X, Y and the radicals R1, R2, R3, R4, R5, R6, R7 and R8 are as defined under 1) - 3), by reaction of a compound of the formula I, Ia, Ib or Ic, in which the definitions mentioned under 1) - 3) apply to X and the radicals R1, R2, R3, R4, R5 and R6 and Y is H, OH, SH, NH₂ or NHR6, with a reagent of the formula IV
R9-Z (IV)
in which R9 has the meanings mentioned above under 1) - 3) for R2, R6, R7 and R8, with the exception of hydrogen, and Z is a leaving group,
or
C) preparing a compound of the formula I or Ib, where X is sulfur and R1, R2, R3, R4, R5, R6, R7 and R8 are as defined under 1) - 3), by reaction of a compound of the formula I or Ib, in which X is oxygen and the definitions mentioned under 1) - 3) apply to R1, R2, R3, R4, R5, R6, R7 and R8, with a sulfurizing reagent,
or
D) for the preparation of a compound of the formula I, Ia, Ib or Ic, where X and R1, R2, R3, R4 and R5 are as defined under 1) - 3) and Y is O-R6, S-R6 or N-R6R7, reacting a compound of the formula I, Ia, Ib or Ic, where X and R1, R2, R3, R4 and R5 are as defined under 1) - 3) and Y is OH, SH, NH₂ or NHR6,
with a compound of the formula V
R9-OH (V)
in which R9 can be
alkyl, which is optionally substituted by fluorine, chlorine, bromine, iodine, cyano, amino, mercapto, hydroxyl, acyloxy, benzoyloxy, benzyloxy, phenoxy, alkoxy, alkylamino, dialkylamino, alkylthio, alkylsulfonyl, phenylsulfonyl, oxo, thioxo, carboxyl or carbamoyl;
alkenyl, which is optionally substituted by fluorine, chlorine, bromine, iodine, cyano, amino, mercapto, hydroxyl, acyloxy, benzoyloxy, benzyloxy, phenoxy, alkoxy, alkylamino, dialkylamino, alkylthio, alkylsulfonyl, phenylsulfonyl, oxo, thioxo, carboxyl or carbamoyl; alkynyl, which is optionally substituted by fluorine, chlorine, bromine, iodine, cyano, amino, mercapto, hydroxyl, acyloxy, benzoyloxy, benzyloxy, phenoxy, alkoxy, alkylamino, dialkylamino, alkylthio, alkylsulfonyl, phenylsulfonyl, oxo, thioxo, carboxyl or carbamoyl;
cycloalkyl, which is optionally substituted by fluorine, chlorine, bromine, iodine, cyano, amino, mercapto, hydroxyl, acyloxy, benzoyloxy, benzyloxy, phenoxy, alkoxy, alkylamino, dialkylamino, alkylthio, alkylsulfonyl, phenylsulfonyl, oxo, thioxo, carboxyl or carbamoyl;
cycloalkenyl, which is optionally substituted by fluorine, chlorine, bromine, iodine, cyano, amino, mercapto, hydroxyl, acyloxy, benzoyloxy, benzyloxy, phenoxy, alkoxy, alkylamino, dialkylamino, alkylthio, alkylsulfonyl, phenylsulfonyl, oxo, thioxo, carboxyl or carbamoyl;
(cycloalkyl)-(alkyl), which is optionally substituted by fluorine, chlorine, bromine, iodine, cyano, amino, mercapto, hydroxyl, acyloxy, benzoyloxy, benzyloxy, phenoxy, alkoxy, alkylamino, dialkylamino, alkylthio, alkylsulfonyl, phenylsulfonyl, oxo, thioxo, carboxyl or carbamoyl;
(cycloalkenyl)-(alkyl), which is optionally substituted by fluorine, chlorine, bromine, iodine, cyano, amino, mercapto, hydroxyl, acyloxy, benzoyloxy, benzyloxy, phenoxy, alkoxy, alkylamino, dialkylamino, alkylthio, alkylsulfonyl, phenylsulfonyl, oxo, thioxo, carboxyl or carbamoyl;
alkylcarbonyl, which is optionally substituted by fluorine, chlorine, bromine, iodine, cyano, amino, mercapto, hydroxyl, acyloxy, benzoyloxy, benzyloxy, phenoxy, alkoxy, alkylamino, dialkylamino, alkylthio, alkylsulfonyl, phenylsulfonyl, oxo, thioxo, carboxyl or carbamoyl;
alkenylcarbonyl, which is optionally substituted by fluorine, chlorine or hydroxyl, alkoxy, oxo or phenyl;
(cycloalkyl)carbonyl, which is optionally substituted by fluorine, chlorine or hydroxyl, alkoxy, oxo or phenyl;
fluorine, chlorine or hydroxyl, alkoxy, oxo or phenyl;
(cycloalkenyl)carbonyl, which is optionally substituted by fluorine, chlorine or hydroxyl, alkoxy, oxo or phenyl;
(cycloalkyl)-(alkyl)carbonyl, which is optionally substituted by fluorine, chlorine or hydroxyl, alkoxy, oxo or phenyl;
(cycloalkenyl)-(alkyl)carbonyl, which is optionally substituted by fluorine, chlorine or hydroxyl, alkoxy, oxo or phenyl;
or aryl, arylcarbonyl, arylalkyl, arylalkenyl, arylalkynyl, arylalkylcarbonyl or arylalkenylcarbonyl, which are substituted by up to five radicals R5 which are independent of one another, in which R5 is as defined above,
or heteroaryl, heteroarylalkyl, heteroarylalkenyl, heteroarylalkylcarbonyl or heteroarylalkenylcarbonyl, which are substituted by up to three radicals R5 which are independent of one another,
in the presence of a dehydrating agent,
or
E) preparing a compound of the formula I or Ib, where X is oxygen and Y, R1, R2, R3, R4 and R5 are as defined under 1) - 3), by cyclization of a compound of the formula VI 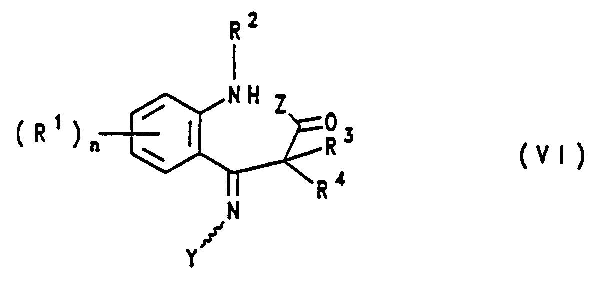 where Y, R1, R2, R3, R4 and R5 are as defined under 1) - 3) and Z is a leaving group.

7. A medicament comprising an active amount of a

8. A process for the preparation of a medicament, which comprises bringing a compound as claimed in any of claims 1-3 into a suitable presentation form.

## Revendications

1. Composés de formule I 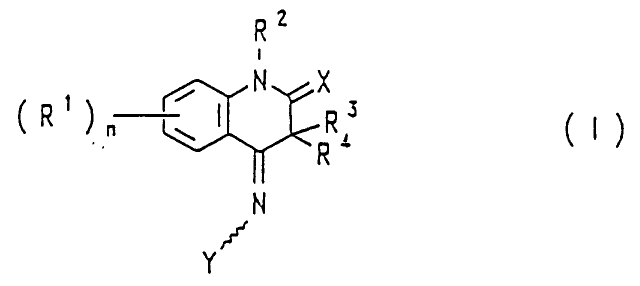 ainsi que leurs formes tautomères de formules générales Ia, Ib et Ic 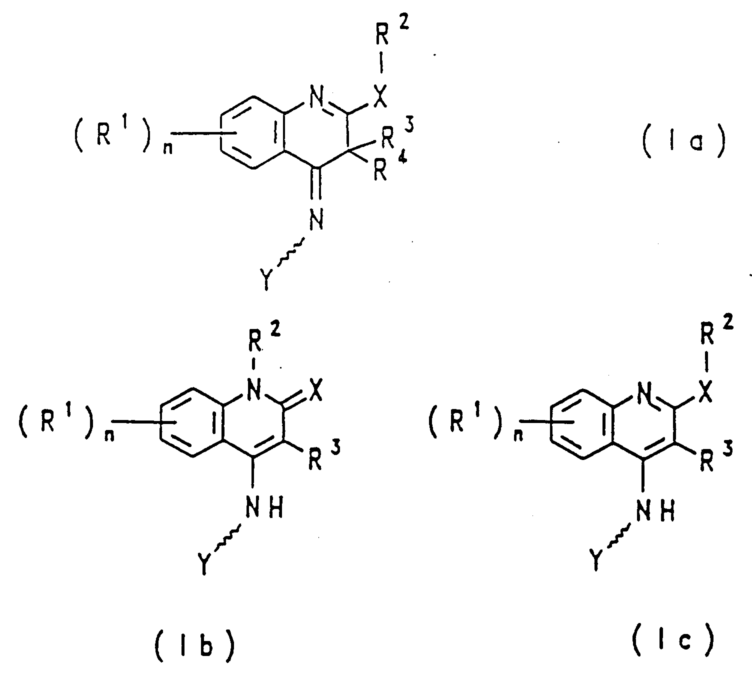 formules dans lesquelles
n représente zéro, un, deux, trois ou quatre,
les substituants individuels R¹ représentent, indépendamment les uns des autres, un atome de fluor, chlore, brome ou iode ou un groupe trifluorométhyle, trifluorométhoxy, hydroxyle, alkyle en C₁-C₈, cycloalkyle en C₅-C₈, alcoxy en C₁-C₆, alcoxy(C₁-C₆)-alcoxy(C₁-C₄), alkyl(C₁-C₆)thio, alkyl(C₁-C₆)sulfinyle, alkyl(C₁-C₆)sulfonyle, nitro, amino, azido, alkyl(C₁-C₆)amino, dialkyl(C₁-C₆)-amino, pipéridino, morpholino, 1-pyrrolidinyle, acyle en C₁-C₆, acyloxy en C₁-C₆, acyl(C₁-C₆)amino, cyano, carbamoyle, carboxyle, alkyl(C₁-C₆)oxycarbonyle, hydroxysulfonyle, sulfamoyle,
ou
un groupe phényle, phénoxy, phénoxycarbonyle, phénylthio, phénylsulfinyle, phénylsulfonyle, phénoxysulfonyle, phénylsulfonyloxy, phénylsulfonylamino, benzoyle, hétéroaroyle, hétéroaryle ou hétéroarylméthyle, éventuellement substitué par jusqu'à cinq radicaux R⁵ indépendants les uns des autres,
R⁵ pouvant être
un atome de fluor, chlore, brome ou iode ou un groupe cyano, trifluorométhyle, trifluorométhoxy, nitro, amino, azido, alkyle en C₁-C₆, cycloalkyle en C₃-C₈, alcoxy en C₁-C₆, alkyl(C₁-C₆)thio, alkyl(C₁-C₆)sulfinyle, alkyl(C₁-C₆)sulfonyle, alkyl(C₁-C₆)amino, dialkyl(C₁-C₆)amino, alkyl(C₁-C₆)oxycarbonyle, phényle, phénoxy ou hétéroaryle,
X représente un atome d'oxygène, de soufre ou de sélénium ou un atome d'azote substitué N-R², N-O-R², où R² peut avoir les significations indiquées plus loin,
Y représente R⁶, O-R⁶, S-R⁶, N-R⁶R⁷, N=CR⁶R⁷ ou C-R⁶R⁷R⁸, R⁶, R⁷ et R⁸ pouvant avoir les significations indiquées plus loin,
R², R⁶, R⁷ et R⁸ peuvent être identiques ou différents et représenter, indépendamment les uns des autres,
un atome d'hydrogène,
un groupe alkyle en C₁-C₈, éventuellement substitué par un atome de fluor, chlore, brome ou iode ou par un groupe cyano, amino, mercapto, hydroxyle, acyloxy en C₁-C₆, benzoyloxy, benzyloxy, phénoxy, alcoxy en C₁-C₆, alkyl(C₁-C₆)amino, dialkyl(C₁-C₆)amino, alkyl(C₁-C₆)thio, alkyl(C₁-C₆)sulfonyle, phénylsulfonyle, oxo, thioxo, carboxyle, carbamoyle,
un groupe alcényle en C₂ -C₈, éventuellement substitué par un atome de fluor, chlore, brome ou iode ou par un groupe cyano, amino, mercapto, hydroxyle, acyloxy en C₁-C₆, benzoyloxy, benzyloxy, phénoxy, alcoxy en C₁-C₆, alkyl(C₁-C₆)amino, dialkyl(C₁-C₆)amino, alkyl(C₁-C₆)thio, alkyl(C₁-C₆)sulfonyle, phénylsulfonyle, oxo, thioxo, carboxyle, carbamoyle,
un groupe alcynyle en C₃ -C₈, éventuellement substitué par un atome de fluor, chlore, brome ou iode ou par un groupe cyano, amino, mercapto, hydroxyle, acyloxy en C₁-C₆, benzoyloxy, benzyloxy, phénoxy, alcoxy en C₁-C₆, alkyl(C₁-C₆)amino, dialkyl(C₁-C₆)amino, alkyl(C₁-C₆)thio, alkyl(C₁-C₆)sulfonyle, phénylsulfonyle, oxo, thioxo, carboxyle, carbamoyle,
un groupe cycloalkyle en C₃-C₈, éventuellement substitué par un atome de fluor, chlore, brome ou iode ou par un groupe cyano, amino, mercapto, hydroxyle, acyloxy en C₁-C₆, benzoyloxy, benzyloxy, phénoxy, alcoxy en C₁-C₆, alkyl(C₁-C₆)amino, dialkyl(C₁-C₆)amino, alkyl(C₁-C₆)thio, alkyl(C₁-C₆)sulfonyle, phénylsulfonyle, oxo, thioxo, carboxyle, carbamoyle,
un groupe cycloalcényle en C₅-C₈, éventuellement substitué par un atome de fluor, chlore, brome ou iode ou par un groupe cyano, amino, mercapto, hydroxyle, acyloxy en C₁-C₆, benzoyloxy, benzyloxy, phénoxy, alcoxy en C₁-C₆, alkyl(C₁-C₆)amino, dialkyl(C₁-C₆)amino, alkyl(C₁-C₆)thio, alkyl(C₁-C₆)sulfonyle, phénylsulfonyle, oxo, thioxo, carboxyle, carbamoyle,
un groupe cycloalkyl(C₃-C₈)-alkyle(C₁-C₄), éventuellement substitué par un atome de fluor, chlore, brome ou iode ou par un groupe cyano, amino, mercapto, hydroxyle, acyloxy en C₁-C₆, benzoyloxy, benzyloxy, phénoxy, alcoxy en C₁-C₆, alkyl(C₁-C₆)amino, dialkyl(C₁-C₆)amino, alkyl(C₁-C₆)thio, alkyl(C₁-C₆)sulfonyle, phénylsulfonyle, oxo, thioxo, carboxyle, carbamoyle,
un groupe cycloalcényl(C₅-C₈)-alkyle(C₁-C₄), éventuellement substitué par un atome de fluor, chlore, brome ou iode ou par un groupe cyano, amino, mercapto, hydroxyle, acyloxy en C₁-C₆, benzoyloxy, benzyloxy, phénoxy, alcoxy en C₁-C₆, alkyl(C₁-C₆)amino, dialkyl(C₁-C₆)amino, alkyl(C₁-C₆)thio, alkyl(C₁-C₆)sulfonyle, phénylsulfonyle, oxo, thioxo, carboxyle, carbamoyle,
un groupe alkyl(C₁-C₆)carbonyle, éventuellement substitué par un atome de fluor, chlore, brome ou iode ou par un groupe cyano, amino, mercapto, hydroxyle, acyloxy en C₁-C₆, benzoyloxy, benzyloxy, phénoxy, alcoxy en C₁-C₆, alkyl(C₁-C₆)amino, dialkyl(C₁-C₆)amino, alkyl(C₁-C₆)thio, alkyl(C₁-C₆)sulfonyle, phénylsulfonyle, oxo, thioxo, carboxyle, carbamoyle,
un groupe alcényl(C₂-C₈)carbonyle, éventuellement substitué par un atome de fluor ou de chlore ou par un groupe hydroxyle, alcoxy en C₁-C₄, oxo ou phényle,
un groupe cycloalkyl(C₃-C₈)carbonyle, éventuellement substitué par un atome de fluor ou de chlore ou par un groupe hydroxyle, alcoxy en C₁-C₄, oxo ou phényle,
un groupe cycloalcényl(C₅-C₈)carbonyle, éventuellement substitué par un atome de fluor ou de chlore ou par un groupe hydroxyle, alcoxy en C₁-C₄, oxo ou phényle;
un groupe cycloalkyl(C₃-C₈)-alkyl(C₁-C₃)carbonyle, éventuellement substitué par un atome de fluor ou de chlore ou par un groupe hydroxyle, alcoxy en C₁-C₄, oxo ou phényle,
un groupe cycloalcényl(C₅-C₆)-alkyl(C₁-C₃)-carbonyle, éventuellement substitué par un atome de fluor ou de chlore ou par un groupe hydroxyle, alcoxy en C₁-C₄, oxo ou phényle,
un groupe alkyloxy(C₁-C₈)carbonyle, éventuellement substitué par un atome de fluor, chlore ou brome ou par un groupe hydroxyle, alcoxy en C₁-C₄, alkyl(C₁-C₄)amino, dialkyl(C₁-C₄)amino, alkyl(C₁-C₄)thio,
un groupe alcényloxy(C₂-C₈)carbonyle, éventuellement substitué par un atome de fluor ou de chlore ou par un groupe hydroxyle, alcoxy en C₁-C₄, oxo ou phényle,
un groupe alcynyloxy(C₂-C₈)carbonyle, éventuellement substitué par un atome de fluor ou de chlore ou par un groupe hydroxyle, alcoxy en C₁-C₄, oxo ou phényle,
un groupe alkyl(C₁-C₈)thiocarbonyle, éventuellement substitué par un atome de fluor ou de chlore ou par un groupe hydroxyle, alcoxy en C₁-C₄, oxo ou phényle,
un groupe alcényl(C₂-C₈)thiocarbonyle, éventuellement substitué par un atome de fluor ou de chlore ou par un groupe hydroxyle, alcoxy en C₁-C₄, oxo ou phényle,
un groupe alkyl(C₁-C₈)aminocarbonyle et un groupe dialkyl(C₁-C₈)aminocarbonyle, éventuellement substitués par un atome de fluor ou de chlore ou par un groupe hydroxyle, alcoxy en C₁-C₄, oxo ou phényle,
un groupe alcényl(C₂-C₈)aminocarbonyle et un groupe dialcényl(C₂-C₆)aminocarbonyle, éventuellement substitués par un atome de fluor ou de chlore ou par un groupe hydroxyle, alcoxy en C₁-C₄, oxo ou phényle,
un groupe alkyl(C₁-C₆)sulfonyle, éventuellement substitué par un atome de fluor ou de chlore ou par un groupe hydroxyle, alcoxy en C₁-C₄, alkyl(C₁-C₄)thio, oxo ou phényle,
un groupe alcényl(C₂-C₆)sulfonyle, éventuellement substitué par un atome de fluor ou de chlore ou par un groupe hydroxyle, alcoxy en C₁-C₄, oxo ou phényle,
ou un groupe aryle, arylcarbonyle, aryl(thiocarbonyle), (arylthio)carbonyle, (arylthio)thiocarbonyle, aryloxycarbonyle, (arylamino) thiocarbonyle, arylsulfonyle, arylalkyle, arylalcényle, arylalcynyle, arylalkylcarbonyle, arylalcénylcarbonyle, arylalcoxycarbonyle, substitué par jusqu'à cinq radicaux R⁵ indépendants les uns des autres, le fragment alkyle pouvant comporter dans chaque cas de 1 à 5 atomes de carbone, et R⁵ étant tel que défini plus haut,
ou un groupe hétéroaryle, hétéroarylalkyle, hétéroarylalcényle, hétéroarylalkylcarbonyle ou hétéroarylalcénylcarbonyle substitué par jusqu'à trois radicaux R⁵ indépendants les uns des autres, le fragment alkyle pouvant comporter dans chaque cas de 1 à 3 atomes de carbone, et
R³ et R⁴ sont identiques ou différents et représentent, indépendamment l'un de l'autre,
un atome d'hydrogène,
un groupe alkyle en C₁-C₈, éventuellement substitué par un atome de fluor ou de chlore ou par un groupe hydroxyle, amino, mercapto, acyloxy en C₁-C₄, benzoyloxy, benzyloxy, phénoxy, alcoxy en C₁-C₄, alkyl(C₁-C₄)amino, dialkyl(C₁-C₄)amino, alkyl(C₁-C₄)thio, alkyl(C₁-C₄)-sulfonyle, alkyl(C₁-C₄)sulfinyle, carboxyle, carbamoyle,
un groupe alcényle en C₂-C₈, éventuellement substitué par un atome de fluor ou de chlore ou par un groupe hydroxyle, amino, mercapto, acyloxy en C₁-C₄, benzoyloxy, benzyloxy, phénoxy, alcoxy en C₁-C₄, alkyl(C₁-C₄)amino, dialkyl(C₁-C₄)amino, alkyl(C₁-C₄)thio, alkyl(C₁-C₄)-sulfonyle, alkyl(C₁-C₄)sulfinyle, carboxyle, carbamoyle,
un groupe cycloalkyle en C₃-C₈, éventuellement substitué par un atome de fluor ou de chlore ou par un groupe hydroxyle, amino, mercapto, acyloxy en C₁-C₄, benzoyloxy, benzyloxy, phénoxy, alcoxy en C₁-C₄, alkyl(C₁-C₄)amino, dialkyl(C₁-C₄)amino, alkyl(C₁-C₄)thio, alkyl(C₁-C₄)sulfonyle, alkyl(C₁-C₄)sulfinyle, carboxyle, carbamoyle,
un groupe cycloalcényle en C₃-C₈, éventuellement substitué par un atome de fluor ou de chlore ou par un groupe hydroxyle, amino, mercapto, acyloxy en C₁-C₄, benzoyloxy, benzyloxy, phénoxy, alcoxy en C₁-C₄, alkyl(C₁-C₄)amino, dialkyl(C₁-C₄)amino, alkyl(C₁-C₄)thio, alkyl(C₁-C₄)- sulfonyle, alkyl(C₁-C₄)sulfinyle, carboxyle, carbamoyle,
un groupe aryle, arylalkyle, hétéroaryle ou hétéroarylalkyle substitué par jusqu'à cinq radicaux R⁵ indépendants les uns des autres, le fragment alkyle pouvant comporter dans chaque cas de 1 à 3 atomes de carbone et R⁵ étant tel que défini plus haut,
R³ et R⁴ peuvent également représenter un radical =C-Z¹Z² relié par une double liaison, Z¹ et Z² ayant les significations indiquées plus haut pour R³ et R⁴,
R³ et R⁴ peuvent en outre faire également partie d'un cycle carbo- ou hétérocyclique saturé ou insaturé, qui peut éventuellement être substitué par un atome de fluor ou de chlore ou par un groupe hydroxyle, amino, alkyle en C₁-C₈, alcényle en C₂-C₈, alcynyle en C₂-C₈, acyloxy en C₁-C₈, benzoyloxy, alcoxy en C₁-C₈, alkyl(C₁-C₈)thio, oxo, thioxo, carboxyle, carbamoyle, ou phényle,
leurs isomères optiques, diastéréoisomères sous forme pure ou sous forme de leurs mélanges, et sels d'addition de tels composés,
dans les définitions précédentes, les cycles hétérocycliques et les groupes hétéroaryle contenant de 1 à 15 atomes de carbone et de 1 à 6 hétéroatomes choisis parmi les atomes d'oxygène, de soufre et d'azote, et, dans le cas de la présence en cette position d'un cycle azoté saturé N-Z, dans lequel Z représente H ou R² avec les définitions respectives données plus haut,
à l'exclusion des composés dans lesquels R³ et/ou R⁴ représentent simultanément l'atome d'hydrogène et Y représente R⁶ ou CR⁶R⁷R⁸,
et à l'exclusion des composés dans lesquels simultanément n = 0 ou 1, R¹ = CH₃ ou OCH₃, R² = CH₃, R³ = CH₂CH₂OCH₃, X = O et Y = H,
et à l'exclusion des composés dans lesquels simultanément n = 1, R¹ = Cl, R² = H ou CH₃, R³ = OH, R⁴ = phényle, X = O et Y = H ou OH.

2. Composés de formule I, Ia, Ib ou Ic selon la revendication 1, dans lesquels:
n représente zéro, un ou deux,
les substituants individuels R¹ représentent, indépendamment les uns des autres,
un atome de fluor, chlore, brome ou iode ou un groupe trifluorométhyle, trifluorométhoxy, hydroxyle, alkyle en C₁-C₆, cycloalkyle en C₅-C₆, alcoxy en C₁-C₄, alcoxy(C₁-C₄)-alcoxy(C₁-C₂), alkyl(C₁-C₄)thio, alkyl(C₁-C₄)sulfinyle, alkyl(C₁-C₄)sulfonyle, nitro, amino, alkyl(C₁-C₄)amino, dialkyl(C₁-C₄)amino, acyle en C₁-C₆, acyloxy en C₁-C₄, acyl(C₁-C₄)amino, cyano, carbamoyle, carboxyle, alkyl(C₁-C₄)oxycarbonyle, ou
un groupe phényle, phénoxy, phénoxycarbonyle, phénylthio, phénylsulfinyle, phénylsulfonyle, phénoxysulfonyle, phénylsulfonyloxy, phénylsulfonylamino, benzoyle, hétéroaroyle, hétéroaryle ou hétéroarylméthyle, éventuellement substitué par jusqu'à trois radicaux R⁵ indépendants les uns des autres,
R⁵ pouvant être un atome de fluor ou de chlore ou un groupe trifluorométhyle, trifluorométhoxy, nitro, amino, alkyle en C₁-C₄, cycloalkyle en C₃-C₆, alcoxy en C₁-C₄, alkyl(C₁-C₄)thio, alkyl(C₁-C₄)sulfinyle, alkyl(C₁-C₄)sulfonyle, alkyl(C₁-C₄)amino, dialkyl(C₁-C₄)amino, phényle, phénoxy ou hétéroaryle,
X représente un atome d'oxygène ou de soufre ou un atome d'azote substitué N-R², N-O-R², où R² peut avoir les significations indiquées plus loin,
Y représente R⁶, O-R⁶, S-R⁶, N-R⁶R⁷, N=CR⁶R⁷ ou C-R⁶R⁷R⁸, R⁶, R⁷ et R⁸ pouvant avoir les significations indiquées plus loin,
R², R⁶, R⁷ et R⁸ peuvent être identiques ou différents et représenter, indépendamment les uns des autres,
un atome d'hydrogène,
un groupe alkyle en C₁-C₆, éventuellement substitué par un atome de fluor, chlore, brome ou iode ou par un groupe cyano, amino, mercapto, hydroxyle, acyloxy en C₁-C₄, benzoyloxy, benzyloxy, phénoxy, alcoxy en C₁-C₄, alkyl(C₁-C₄)amino, dialkyl(C₁-C₄)amino, alkyl(C₁-C₄)thio, alkyl(C₁-C₄)sulfonyle, phénylsulfonyle, oxo, thioxo, carboxyle, carbamoyle,
un groupe alcényleen C₂-C₆, éventuellement substitué par un atome de fluor ou de chlore ou par un groupe cyano, amino, mercapto, hydroxyle, acyloxy en C₁-C₄, benzoyloxy, benzyloxy, phénoxy, alcoxy en C₁-C₄, alkyl(C₁-C₄)amino, dialkyl(C₁-C₄)amino, alkyl(C₁-C₄)thio, alkyl(C₁-C₄)sulfonyle, phénylsulfonyle, carboxyle, carbamoyle,
un groupe alcynyle en C₃-C₆, éventuellement substitué par un atome de fluor ou de chlore ou par un groupe cyano, amino, mercapto, hydroxyle, acyloxy en C₁-C₄, benzoyloxy, benzyloxy, phénoxy, alcoxy en C₁-C₄, alkyl(C₁-C₄)amino, dialkyl(C₁-C₄)amino, alkyl(C₁-C₄)thio, alkyl(C₁-C₄)sulfonyle, phénylsulfonyle, carboxyle, carbamoyle,
un groupe cycloalkyle en C₃-C₆, éventuellement substitué par un atome de fluor ou de chlore ou par un groupe cyano, amino, mercapto, hydroxyle, acyloxy en C₁-C₄, benzoyloxy, benzyloxy, phénoxy, alcoxy en C₁-C₄, alkyl(C₁-C₄)amino, dialkyl(C₁-C₄)amino, alkyl(C₁-C₄)thio, alkyl(C₁-C₄)sulfonyle, phénylsulfonyle, carboxyle, carbamoyle,
un groupe cycloalcényle en C₅-C₆, éventuellement substitué par un atome de fluor ou de chlore ou par un groupe cyano, amino, mercapto, hydroxyle, acyloxy en C₁-C₄, benzoyloxy, benzyloxy, phénoxy, alcoxy en C₁-C₄, alkyl(C₁-C₄)amino, dialkyl(C₁-C₄)amino, alkyl(C₁-C₄)thio, alkyl(C₁-C₄)sulfonyle, phénylsulfonyle, carboxyle, carbamoyle,
un groupe cycloalkyl(C₃-C₆)-alkyle(C₁-C₂), éventuellement substitué par un atome de fluor ou de chlore ou par un groupe cyano, amino, mercapto, hydroxyle, acyloxy en C₁-C₄, benzoyloxy, benzyloxy, phénoxy, alcoxy en C₁-C₄, alkyl(C₁-C₄)amino, dialkyl(C₁-C₄)amino, alkyl(C₁-C₄)thio, alkyl(C₁-C₄)sulfonyle, phénylsulfonyle, carboxyle, carbamoyle,
un groupe cycloalcényl(C₅-C₆)-alkyle(C₁-C₂), éventuellement substitué par un atome de fluor ou de chlore ou par un groupe cyano, amino, mercapto, hydroxyle, acyloxy en C₁-C₄, benzoyloxy, benzyloxy, phénoxy, alcoxy en C₁-C₄, alkyl(C₁-C₄)amino, dialkyl(C₁-C₄)amino, alkyl(C₁-C₄)thio, alkyl(C₁-C₄)sulfonyle, phénylsulfonyle, carboxyle, carbamoyle,
un groupe alkyl(C₁-C₆)carbonyle, éventuellement substitué par un atome de fluor ou de chlore ou par un groupe cyano, amino, mercapto, hydroxyle, acyloxy en C₁-C₄, benzoyloxy, benzyloxy, phénoxy, alcoxy en C₁-C₄, alkyl(C₁-C₄)amino, dialkyl(C₁-C₄)amino, alkyl(C₁-C₄)thio, alkyl(C₁-C₄)sulfonyle, phénylsulfonyle, carboxyle, carbamoyle,
un groupe alcényl(C₂-C₆)carbonyle, éventuellement substitué par un atome de fluor ou de chlore ou par un groupe hydroxyle, alcoxy en C₁-C₄, oxo, phényle,
un groupe cycloalkyl(C₃-C₆)carbonyle, éventuellement substitué par un atome de fluor ou de chlore ou par un groupe hydroxyle, alcoxy en C₁-C₄, oxo, phényle,
un groupe cycloalcényl(C₅-C₆)carbonyle, éventuellement substitué par un atome de fluor ou de chlore ou par un groupe hydroxyle, alcoxy en C₁-C₄, oxo, phényle,
un groupe cycloalkyl(C₃-C₆)-alkyl(C₁-C₂)carbonyle, éventuellement substitué par un atome de fluor ou de chlore ou par un groupe hydroxyle, alcoxy en C₁-C₄, oxo, phényle,
un groupe cycloalcényl(C₅-C₆)-alkyl(C₁-C₂)-carbonyle, éventuellement substitué par un atome de fluor ou de chlore ou par un groupe hydroxyle, alcoxy en C₁-C₄, oxo, phényle,
un groupe alkyloxy(C₁-C₆)carbonyle, éventuellement substitué par un atome de fluor ou de chlore ou par un groupe alcoxy en C₁-C₄, alkyl(C₁-C₄)amino, dialkyl(C₁-C₄)amino ou alkyl(C₁-C₄)thio,
un groupe alcényloxy(C₂-C₆)carbonyle, éventuellement substitué par un atome de fluor ou de chlore ou par un groupe alcoxy en C₁-C₄ ou phényle,
un groupe alcynyloxy(C₂-C₆)carbonyle, éventuellement substitué par un atome de fluor ou de chlore ou par un groupe alcoxy en C₁-C₄ ou phényle,
un groupe alkyl(C₁-C₆)thiocarbonyle, éventuellement substitué par un atome de fluor ou de chlore ou par un groupe alcoxy en C₁-C₄ ou phényle,
un groupe alcényl(C₂-C₆)thiocarbonyle, éventuellement substitué par un atome de fluor ou de chlore ou par un groupe alcoxy en C₁-C₄ ou phényle,
un groupe alkyl(C₁-C₆)aminocarbonyle et un groupe dialkyl(C₁-C₆)aminocarbonyle, éventuellement substitués par un atome de fluor ou de chlore ou par un groupe alcoxy en C₁-C₄ ou phényle,
un groupe alcényl(C₂-C₆)aminocarbonyle et un groupe dialcényl(C₂-C₆)aminocarbonyle, éventuellement substitués par un atome de fluor ou de chlore ou par un groupe alcoxy en C₁-C₄ ou phényle,
un groupe alkyl(C₁-C₆)sulfonyle, éventuellement substitué par un atome de fluor ou de chlore ou par un groupe alcoxy en C₁-C₄ ou phényle,
un groupe alcényl(C₂-C₆)sulfonyle, ou
un groupe aryle, arylcarbonyle, aryl(thiocarbonyle), (arylthio)carbonyle, (arylthio)thiocarbonyle, aryloxycarbonyle, (arylamino)thiocarbonyle, arylsulfonyle, arylalkyle, arylalcényle, arylalcynyle, arylalkylcarbonyle, arylalcénylcarbonyle, arylalcoxycarbonyle, substitué par jusqu'à trois radicaux R⁵ indépendants les uns des autres, le fragment alkyle pouvant comporter dans chaque cas de 1 à 4 atomes de carbone, et R⁵ étant tel que défini plus haut,
ou un groupe hétéroaryle, hétéroarylalkyle, hétéroarylalcényle, hétéroarylalkylcarbonyle ou hétéroarylalcénylcarbonyle, substitué par jusqu'à trois radicaux R⁵ indépendants les uns des autres, le fragment alkyle pouvant comporter dans chaque cas de 1 à 3 atomes de carbone, et
R³ et R⁴ sont identiques ou différents et réprésentent, indépendamment l'un de l'autre,
un groupe alkyle en C₁-C₆, éventuellement substitué par un atome de fluor ou de chlore ou par un groupe hydroxyle, amino, mercapto, acyloxy en C₁-C₄, benzoyloxy, benzyloxy, phénoxy, alcoxy en C₁-C₄, alkyl(C₁-C₄)amino, dialkyl(C₁-C₄)amino, alkyl(C₁-C₄)thio, alkyl(C₁-C₄)sulfonyle, alkyl(C₁-C₄)sulfinyle, carboxyle, carbamoyle,
un groupe alcényle en C₂-C₆, éventuellement substitué par un atome de fluor ou de chlore ou par un groupe phénoxy, alcoxy en C₁-C₄, alkyl(C₁-C₄)thio, alkyl(C₁-C₄)sulfonyle, alkyl(C₁-C₄)sulfinyle,
un groupe cycloalkyle en C₃-C₆, éventuellement substitué par un atome de fluor ou de chlore ou par un groupe hydroxyle, amino, mercapto, acyloxy en C₁-C₄, benzoyloxy, benzyloxy, phénoxy, alcoxy en C₁-C₄, alkyl(C₁-C₄)amino, dialkyl(C₁-C₄)amino, alkyl(C₁-C₄)thio, alkyl(C₁-C₄)sulfonyle, alkyl(C₁-C₄)sulfinyle,
un groupe cycloalcényle en C₃-C₆, éventuellement substitué par un atome de fluor ou de chlore ou par un groupe phénoxy, alcoxy en C₁-C₄, alkyl(C₁-C₄)thio, alkyl(C₁-C₄)sulfonyle, alkyl(C₁-C₄)sulfinyle,
un groupe aryle, arylalkyle, hétéroaryle ou hétéroarylalkyle, substitué par jusqu'à trois radicaux R⁵ indépendants les uns des autres, le fragment aryle pouvant comporter dans chaque cas de 1 à 3 atomes de carbone, et R⁵ étant tel que défini plus haut,
l'un des radicaux R³ et R⁴ peut être un atome d'hydrogène,
R³ et R⁴ peuvent également représenter ensemble un radical =C-Z¹-Z² relié par une double liaison, Z¹ et Z² ayant les significations données plus haut pour R³ et R⁴.

3. Composés de formule I, Ia, Ib ou Ic selon les revendications 1-2, dans lesquels
n représente zéro, un ou deux,
les substituants individuels R¹ représentent, indépendamment les uns des autres, un atome de fluor, chlore ou brome ou un groupe trifluorométhyle, trifluorométhoxy, hydroxyle, alkyle en C₁-C₄, alcoxy en C₁-C₄, alcoxy(C₁-C₄)-alcoxy(C₁-C₂), alkyl(C₁-C₄)thio, alkyl(C₁-C₄)-sulfinyle, alkyl(C₁-C₄)sulfonyle, nitro, amino, alkyl(C₁-C₄)amino, dialkyl(C₁-C₄)amino, acyle en C₁-C₄, acyloxy en C₁-C₄, acyl(C₁-C₄)amino, cyano, carbamoyle, carboxyle, alkyl(C₁-C₄)oxycarbonyle, ou
un groupe phényle, phénoxy, phénoxycarbonyle, phénylthio, phénylsulfinyle, phénylsulfonyle, benzoyle, hétéroaroyle, hétéroaryle ou hétéroarylméthyle, éventuellement substitué par jusqu'à trois radicaux R⁵ indépendants les uns des autres,
R⁵ représentant un atome de fluor ou de chlore ou un groupe trifluorométhyle, trifluorométhoxy, nitro, amino, alkyle en C₁-C₄, alcoxy en C₁-C₄, alkyl(C₁-C₄)thio, alkyl(C₁-C₄)sulfinyle, alkyl(C₁-C₄)sulfonyle, alkyl(C₁-C₄)amino, dialkyl(C₁-C₄)amino, phényle ou phénoxy,
X représente un atome d'oxygène ou de soufre ou un atome d'azote substitué N-R², N-O-R², où R² peut avoir les significations données plus loin,
Y représente O-R⁶, S-R⁶, N-R⁶R⁷ ou N=CR⁶R⁷, R⁶, R⁷ et R⁸ pouvant avoir les significations données plus loin,
R², R⁶, R⁷ et R⁸ peuvent être identiques ou différents et représenter, indépendamment les uns des autres,
un atome d'hydrogène,
un groupe alkyle en C₁-C₆ éventuellement substitué par un atome de fluor ou de chlore ou par un groupe amino, mercapto, hydroxyle, acyloxy en C₁-C₄, benzoyloxy, benzyloxy, phénoxy, alcoxy en C₁-C₄, alkyl(C₁-C₄)amino, dialkyl(C₁-C₄)amino, alkyl(C₁-C₄)thio,
un groupe alcényle en C₂-C₆, éventuellement substitué par un atome de fluor ou de chlore ou par un groupe amino, mercapto, hydroxyle, acyloxy en C₁-C₄, benzoyloxy, benzyloxy, phénoxy, alcoxy en C₁-C₄, alkyl(C₁-C₄)amino, dialkyl(C₁-C₄)amino, alkyl(C₁-C₄)thio,
un groupe alcynyle en C₃-C₆, éventuellement substitué par un atome de fluor ou de chlore ou par un groupe amino, mercapto, hydroxyle, acyloxy en C₁-C₄, benzoyloxy, benzyloxy, phénoxy, alcoxy en C₁-C₄, alkyl(C₁-C₄)amino, dialkyl(C₁-C₄)amino, alkyl(C₁-C₄)thio,
un groupe cycloalkyle en C₃-C₆, éventuellement substitué par un atome de fluor ou de chlore ou par un groupe acyloxy en C₁-C₄, benzoyloxy, phénoxy, alcoxy en C₁-C₄, alkyl(C₁-C₄)amino, dialkyl(C₁-C₄)amino, alkyl(C₁-C₄)thio,
un groupe cycloalcényle en C₅-C₆,
un groupe cycloalkyl(C₃-C₆)-alkyle(C₁-C₂),
un groupe cycloalcényl(C₅-C₆)-alkyle(C₁-C₂),
un groupe cycloalkyl(C₁-C₆)carbonyle, éventuellement substitué par un atome de fluor ou de chlore ou par un groupe amino, mercapto, hydroxyle, acyloxy en C₁-C₄, benzoyloxy, benzyloxy, phénoxy, alcoxy en C₁-C₄, alkyl(C₁-C₄)amino, dialkyl(C₁-C₄)amino, alkyl(C₁-C₄)thio,
un groupe alcényl(C₂-C₄)carbonyle,
un groupe cycloalkyl(C₃-C₆)carbonyle,
un groupe cycloalcényl(C₅-C₆)carbonyle,
un groupe cycloalkyl(C₃-C₆)-alkyl(C₁-C₂)carbonyle,
un groupe cycloalcényl(C₅-C₆)-alkyl(C₁-C₂)-carbonyle,
un groupe alkyloxy(C₁-C₆)carbonyle, éventuellement substitué par un atome de fluor ou de chlore ou par un groupe alcoxy en C₁-C₄, alkyl(C₁-C₄)amino, dialkyl(C₁-C₄)amino, alkyl- (C₁-C₄)thio,
un groupe alcényloxy(C₂-C₆)carbonyle,
un groupe alcynyl(C₂-C₆)oxycarbonyle,
un groupe alkyl(C₁-C₆)thiocarbonyle,
un groupe alcényl(C₂-C₆)thiocarbonyle,
un groupe alkyl(C₁-C₆)aminocarbonyle et un groupe dialkyl (C₁-C₆) aminocarbonyle,
un groupe alcényl(C₂-C₆)aminocarbonyle et un groupe dialcényl (C₂-C₆) aminocarbonyle,
un groupe alkyl(C₁-C₆)sulfonyle,
un groupe alcényl(C₂-C₆)sulfonyle,
ou un groupe aryle, arylcarbonyle, aryl(thiocarbonyle), (arylthio)carbonyle, (arylthio)thiocarbonyle, aryloxycarbonyle, (arylamino)thiocarbonyle, arylsulfonyle, arylalkyle, arylalcényle, arylalcynyle, arylalkylcarbonyle, arylalcénylcarbonyle, arylalcoxycarbonyle, substitué par jusqu'à deux radicaux R⁵ indépendants l'un de l'autre,
le fragment alkyle pouvant comporter dans chaque cas de 1 à 3 atomes de carbone et R⁵ étant tel que défini plus haut,
ou un groupe hétéroaryle, hétéroarylalkyle, hétéroarylalcényle, hétéroarylalkylcarbonyle ou hétéroarylalcénylcarbonyle, substitué par jusqu'à deux radicaux R⁵ indépendants l'un de l'autre, le fragment alkyle pouvant comporter dans chaque cas 1 ou 2 atomes de carbone, et
R³ et R⁴ sont identiques ou différents et réprésentent, indépendamment l'un de l'autre,
un groupe alkyle en C₁-C₆, éventuellement substitué par un atome de fluor ou de chlore ou par un groupe hydroxyle, amino, mercapto, acyloxy en C₁-C₄, benzoyloxy, benzyloxy, phénoxy, alcoxy en C₁-C₄, alkyl(C₁-C₄)amino, dialkyl(C₁-C₄)amino, alkyl(C₁-C₄)thio, alkyl(C₁-C₄)sulfonyle, alkyl(C₁-C₄)sulfinyle, carboxyle,
un groupe alcényle en C₂-C₆, éventuellement substitué par un atome de fluor ou de chlore,
un groupe cycloalkyle en C₃-C₆,
un groupe cycloalcényle en C₅-C₆, éventuellement substitué par un atome de fluor ou de chlore,
un groupe aryle, arylalkyle, hétéroaryle ou hétéroarylalkyle, substitué par jusqu'à trois radicaux R⁵ indépendants les uns des autres, le fragment alkyle pouvant comporter dans chaque cas 1 ou 2 atomes de carbone, et R⁵ étant tel que défini plus haut,
l'un des radicaux R³ et R⁴ peut être un atome d'hydrogène,
R³ et R⁴ peuvent également représenter ensemble un radical =C-Z¹Z² relié par une double liaison, Z¹ et Z² ayant les significations données plus haut pour R³ et R⁴.

4. Composés selon une ou plusieurs des revendications 1 à 3, pour utilisation en tant que médicament.

5. Utilisation des composés selon les revendications 1 à 3, pour la fabrication de médicaments destinés à la prophylaxie et/ou au traitement de maladies qui sont provoquées par des virus.

6. Procédé pour la préparation des composés de formules I, Ia, Ib et Ic telles que définies plus haut en 1)-3), caractérisé en ce que
A) pour la préparation des composés de formules I, Ib dans lesquelles X représente un atome d'oxygène et Ia, Ic dans lesquelles X est tel que défini en 1)-3), à l'exclusion de N-R² représentant N-H, Y représentant R⁶, O-R⁶, S-R⁶, N-R⁶R⁷, N=C-R⁶R⁷ ou C-R⁶R⁷R⁸ et les radicaux R¹, R², R³, R⁴, R⁵, R⁶, R⁷ et R⁸ étant tels que définis en 1)-3),
on fait réagir un composé de formule II, IIa, IIb ou IIc, 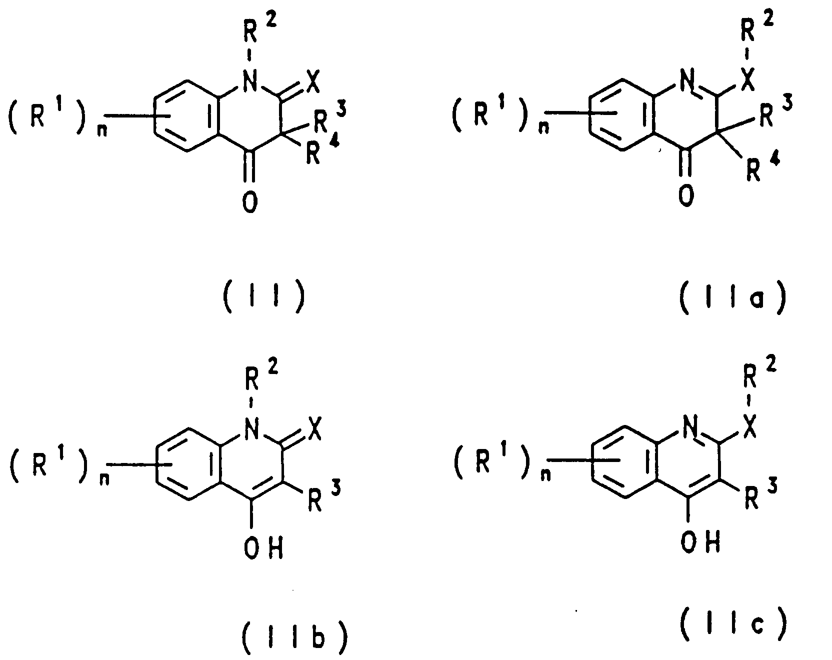 les définitions données en 1)-3) pour R¹, R², R³, R⁴ et R⁵ étant valables,
avec un composé de formule III
Y-NH₂ (III)
dans laquelle Y peut représenter R⁶ O-R⁶, S-R⁶, N-R⁶R⁷, N=C-R⁶R⁷ ou C-R⁶R⁷R⁸ et les définitions données en 1)-3) pour R⁶, R⁷ et R⁸ sont valables,
ou en ce que
B) on prépare des composés de formules I, Ia, Ib et Ic, dans lesquels X, Y et les radicaux R¹, R², R³, R⁴, R⁵, R⁶, R⁷ et R⁸ sont tels que définis en 1)-3), en faisant réagir un composé de formule I, Ia, Ib ou Ic, les définitions données en 1)-3) pour X et les radicaux R¹, R², R³, R⁴, R⁵ et R⁶ étant valables et Y représentant H, OH, SH, NH₂ ou NHR⁶,
avec un réactif de formule IV
R⁹-Z (IV)
dans laquelle R⁹ a les significations données plus haut en 1)-3) pour R², R⁶, R⁷ et R⁸, à l'exclusion de celles d'un atome d'hydrogène, et Z est un groupe partant,
ou en ce que
C) on prépare des composés de formules I et Ib, dans lesquels X représente un atome de soufre et R¹, R², R³, R⁴, R⁵, R⁶, R⁷ et R⁸ sont tels que définis en 1)-3), en faisant réagir un composé de formule I ou Ib, dans lequel X représente un atome d'oxygène, et les définitions données en 1)-3) pour R¹, R², R³, R⁴, R⁵, R⁶, R⁷ et R⁸ sont valables, avec un réactif de sulfuration,
ou en ce que
D) pour la préparation des composés de formules I, Ia, Ib et Ic, dans lesquels X et R¹, R², R³, R⁴ et R⁵ sont tels que définis en 1)-3) et Y représente O-R⁶, S-R⁶ ou N-R⁶R⁷, on fait réagir en présence d'un agent de déshydratation un composé de formule V
R⁹-OH (V)
dans laquelle R⁹ peut être
un groupe alkyle, éventuellement substitué par un atome de fluor, chlore, brome ou iode ou par un groupe cyano, amino, mercapto, hydroxyle, acyloxy, benzoyloxy, benzyloxy, phénoxy, alcoxy, alkylamino, dialkylamino, alkylthio, alkylsulfonyle, phénylsulfonyle, oxo, thioxo, carboxyle, carbamoyle,
un groupe alcényle, éventuellement substitué par un atome de fluor, chlore, brome ou iode ou par un groupe cyano, amino, mercapto, hydroxyle, acyloxy, benzoyloxy, benzyloxy, phénoxy, alcoxy, alkylamino, dialkylamino, alkylthio, alkylsulfonyle, phénylsulfonyle, oxo, thioxo, carboxyle, carbamoyle,
un groupe alcynyle, éventuellement substitué par un atome de fluor, chlore, brome ou iode ou par un groupe cyano, amino, mercapto, hydroxyle, acyloxy, benzoyloxy, benzyloxy, phénoxy, alcoxy, alkylamino, dialkylamino, alkylthio, alkylsulfonyle, phénylsulfonyle, oxo, thioxo, carboxyle, carbamoyle,
un groupe cycloalkyle, éventuellement substitué par un atome de fluor, chlore, brome ou iode ou par un groupe cyano, amino, mercapto, hydroxyle, acyloxy, benzoyloxy, benzyloxy, phénoxy, alcoxy, alkylamino, dialkylamino, alkylthio, alkylsulfonyle, phénylsulfonyle, oxo, thioxo, carboxyle, carbamoyle,
un groupe cycloalcényle, éventuellement substitué par un atome de fluor, chlore, brome ou iode ou par un groupe cyano, amino, mercapto, hydroxyle, acyloxy, benzoyloxy, benzyloxy, phénoxy, alcoxy, alkylamino, dialkylamino, alkylthio, alkylsulfonyle, phénylsulfonyle, oxo, thioxo, carboxyle, carbamoyle,
un groupe cycloalkyl-alkyle, éventuellement substitué par un atome de fluor, chlore, brome ou iode ou par un groupe cyano, amino, mercapto, hydroxyle, acyloxy, benzoyloxy, benzyloxy, phénoxy, alcoxy, alkylamino, dialkylamino, alkylthio, alkylsulfonyle, phénylsulfonyle, oxo, thioxo, carboxyle, carbamoyle,
un groupe cycloalcényl-alkyle, éventuellement substitué par un atome de fluor, chlore, brome ou iode ou par un groupe cyano, amino, mercapto, hydroxyle, acyloxy, benzoyloxy, benzyloxy, phénoxy, alcoxy, alkylamino, dialkylamino, alkylthio, alkylsulfonyle, phénylsulfonyle, oxo, thioxo, carboxyle, carbamoyle,
un groupe alkylcarbonyle, éventuellement substitué par un atome de fluor, chlore, brome ou iode ou par un groupe cyano, amino, mercapto, hydroxyle, acyloxy, benzoyloxy, benzyloxy, phénoxy, alcoxy, alkylamino, dialkylamino, alkylthio, alkylsulfonyle, phénylsulfonyle, oxo, thioxo, carboxyle, carbamoyle,
un groupe alcénylcarbonyle, éventuellement substitué par un atome de fluor ou de chlore ou par un groupe hydroxyle, alcoxy, oxo, phényle,
un groupe (cycloalkyl)carbonyle, éventuellement substitué par un atome de fluor ou de chlore ou par un groupe hydroxyle, alcoxy, oxo, phényle,
un groupe (cycloalcényl)carbonyle, éventuellement substitué par un atome de fluor ou de chlore ou par un groupe hydroxyle, alcoxy, oxo, phényle,
un groupe cycloalkyl-(alkyl)carbonyle, éventuellement substitué par un atome de fluor ou de chlore ou par un groupe hydroxyle, alcoxy, oxo, phényle,
un groupe cycloalcényl-(alkyl)carbonyle, éventuellement substitué par un atome de fluor ou de chlore ou par un groupe hydroxyle, alcoxy, oxo, phényle,
ou un groupe aryle, arylcarbonyle, arylalkyle, arylalcényle, arylalcynyle, arylalkylcarbonyle, arylalcénylcarbonyle, substitué par jusqu'à cinq radicaux R⁵ indépendants les uns des autres, R⁵ étant tel que défini plus haut,
ou un groupe hétéroaryle, hétéroarylalkyle, hétéroarylalcényle, hétéroarylalkylcarbonyle ou hétéroarylalcénylcarbonyle, substitué par jusqu'à trois radicaux R⁵ indépendants les uns des autres,
ou en ce que
E) on prépare des composés de formules I et Ib, dans lesquels X représente un atome d'oxygène et Y, R¹, R², R³, R⁴ et R⁵ sont tels que définis en 1)-3), par cyclisation d'un composé de formule VI 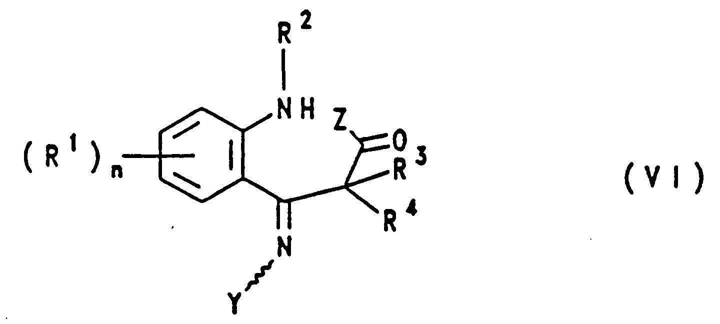 dans laquelle Y, R¹, R², R³, R⁴, R⁵ sont tels que définis en 1)-3) et Z est un groupe partant.

7. Médicament, contenant une quantité efficace d'un composé selon les revendications 1 à 3.

8. Procédé pour la fabrication de médicaments, caractérisé en ce que l'on met sous une forme d'administration appropriée un composé selon les revendications 1 à 3.
